(19) **Europäisches Patentamt / European Patent Office / Office européen des brevets**

(11) **EP 4 703 385 A1**

(12) **EUROPEAN PATENT APPLICATION**
published in accordance with Art. 153(4) EPC

(43) Date of publication:
04.03.2026 Bulletin 2026/10

(21) Application number: 24796266.5

(22) Date of filing: 26.04.2024

(51) International Patent Classification (IPC):
C07K 16/28 (2006.01)    C07K 16/46 (2006.01)
A61K 39/395 (2006.01)

(52) Cooperative Patent Classification (CPC):
A61K 39/395; C07K 16/28; C07K 16/46

(86) International application number:
PCT/CN2024/090122

(87) International publication number:
WO 2024/222895 (31.10.2024 Gazette 2024/44)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 28.04.2023  CN 202310484003

(71) Applicant: Fortvita Biologics Inc.
1205 Grand Cayman (KY)

(72) Inventors:
• RAN, Hao
Suzhou, Jiangsu 215123 (CN)

• WANG, Shiyi
Suzhou, Jiangsu 215123 (CN)
• HE, Chenglin
Suzhou, Jiangsu 215123 (CN)
• CAO, Lei
Suzhou, Jiangsu 215123 (CN)

(74) Representative: Sagittarius IP
Marlow International
Parkway
Marlow SL7 1YL (GB)

Remarks:
The complete document including Reference
Table(s) and the Sequence Listing(s) can be
downloaded from the EPO website

(54) **ANTI-CD40L ANTIBODY AND USE THEREOF IN TREATMENT OF HUMAN AUTOIMMUNE DISEASES**

(57) The present invention relates to a novel antibody and an antibody fragment that specifically binds to CD40L, as well as a composition comprising the antibody or the antibody fragment. The present invention further relates to a nucleic acid encoding the antibody or the antibody fragment thereof, and a host cell comprising the same, as well as related uses. Furthermore, the present invention relates to therapeutic and diagnostic uses of the antibodies and the antibody fragments.

EP 4 703 385 A1

**Description**

The present invention relates to a novel antibody and an antibody fragment that specifically bind to CD40L, and a composition containing the antibody or antibody fragment. The present invention further relates to a nucleic acid encoding the antibody or the antibody fragment thereof, a host cell comprising the same, and related uses. In addition, the present invention relates to therapeutic and diagnostic uses of the antibody and antibody fragment.

**Background of the Invention**

CD40 ligand (CD40L), also referred to as CD154, gp39, TNF receptor-associated protein (TRAP), 5c8 antigen, or T-BAM, is a type II transmembrane protein with a full length of 39 kDa. CD40L belongs to the tumor necrosis factor (TNF) superfamily and exists on the cell membrane in the form of a homotrimer. It is mainly expressed on activated T cells, and is also present on other types of immune and non-immune cells, such as epithelial cells, monocytes, dendritic cells, fibroblasts, smooth muscle cells, endothelial cells, and platelets. The CD40L/CD40 signaling pathway mediates various immune and inflammatory responses and plays an important role in normal immune responses. Existing studies have shown that the CD40L/CD40 signaling pathway not only mediates signaling between various lymphocytes but also participates in interactions between non-immune cells. Therefore, the CD40L/CD40 signaling pathway plays an important pathogenic role in various chronic inflammatory diseases, such as autoimmune diseases, neurodegenerative diseases, graft-versus-host disease, tumors, and atherosclerosis.

Currently, multiple antibodies or protein drugs targeting CD40/CD40L have demonstrated positive efficacy in clinical settings. An early anti-CD40L antibody, Ruplizumab (CN101072587B), demonstrated good treatment effects in diseases such as systemic lupus erythematosus and lupus nephritis, but its development was halted due to serious safety events, including thrombosis (Nat. Med. 6 (2) (2000) 114). Subsequent studies have shown that thrombotic events are primarily caused by free CD40L forming immune complexes with the antibody, which then interact with C1q and FcgR, particularly FcγRIIa expressed on platelets, triggering platelet activation and aggregation and resulting in thrombotic events. Therefore, complete removal of Fc region effector function is critical for the safety of anti-CD40L antibodies.

Anti-CD40L antibodies that subsequently entered clinical trials have all been modified in their Fc region. The main technical approaches are twofold: first, reducing Fc region effector function through mutations; and second, completely removing the Fc region while using other techniques to extend the molecule's half-life. However, both approaches have certain issues. Among these, in the first technical approach, taking Frexalimab (AU2016294417B2) as an example, the Fc region effector function was reduced using C220S/C226S/C229S/P238S and E269R/K322A mutations, respectively. However, subsequent studies have shown that all these mutations cannot completely eliminate Fc effector function, and therefore certain safety risks still exist. The second technical approach is exemplified by Dapirolizumab pegol (US8293237B2) and Dazodalibep (CN101679521B), wherein Dapirolizumab pegol retains only the monovalent anti-CD40L Fab and extends its half-life by conjugation with PEG. However, this monovalent molecular format reduces the molecule's biological activity, and PEG conjugation also increases the difficulty of reaching the molecule's critical micelle concentration (CMC). Dazodalibep uses a Tn3 scaffold protein in a non-conventional antibody format as a molecule for targeted inhibition of CD40L and extends the molecule's half-life by fusion with albumin. However, clinical data shows that this molecular format has high immunogenicity and therefore also carries certain safety risks.

Therefore, drugs targeting CD40/CD40L, particularly antibody drugs, that offer improved safety while retaining the pharmacokinetic characteristics of antibody drugs, and at the same time possess stronger biological activity, are desirable.

**Summary of the Invention**

The present invention provides an antibody or fragment thereof (e.g., an antigen-binding fragment) targeting CD40L. Specifically, the present invention employs mouse hybridoma technology to screen for and obtain a novel antibody targeting CD40L with high affinity. In addition, the present invention provides mutation techniques and mutants produced using such techniques, which completely eliminate Fc region-mediated effector functions (ADCC, ADCP, CDC) while retaining the conventional IgG molecular format, thereby reducing safety risks. Specifically, the present invention provides an antibody targeting CD40L through different Fc mutation techniques (mutation sites: L234A/L235A/ΔP329).

The antibody in the embodiments of the present invention exhibits improved safety. Specifically, the antibody adopts the molecular format of an IgG and, via Fc mutation techniques, completely eliminates binding capability of Fc region to all FcγRs while retaining affinity for FcRn. Therefore, the antibody and a fragment thereof retain the favorable pharmacokinetic properties of antibodies while reducing immunogenicity and safety risks.

On the other hand, the antibody in the embodiments of the present invention also exhibits enhanced biological activity. Specifically, the high-affinity antibody targeting CD40L obtained through screening, and the fragment thereof, can more effectively block the CD40L/CD40 signaling pathway and/or the biological functions mediated thereby. Therefore, the antibody and the fragment thereof exhibit superior biological activity.

In some embodiments, the present invention relates to an anti-CD40L antibody or an antigen-binding fragment thereof, wherein the antibody comprises:

(i) the three complementarity-determining regions HCDR1, HCDR2, and HCDR3 comprised in the VH set forth in SEQ ID NO: 4, and the three complementarity-determining regions LCDR1, LCDR2, and LCDR3 comprised in the VL set forth in SEQ ID NO: 10; or

(ii) the three complementarity-determining regions HCDR1, HCDR2, and HCDR3 comprised in the VH set forth in SEQ ID NO: 16, and the three complementarity-determining regions LCDR1, LCDR2, and LCDR3 comprised in the VL set forth in SEQ ID NO: 21.

For example, HCDR1 is defined according to the Kabat and Chothia combination, and HCDR2, HCDR3, LCDR1, LCDR2, and LCDR3 are defined according to Kabat.

In some embodiments, the present invention relates to an anti-CD40L antibody or an antigen-binding fragment thereof, which comprises a first heavy chain complementarity-determining region (HCDR1), a second heavy chain complementarity-determining region (HCDR2), a third heavy chain complementarity-determining region (HCDR3), and a first light chain complementarity-determining region (LCDR1), a second light chain complementarity-determining region (LCDR2), and a third light chain complementarity-determining region (LCDR3), wherein:

(i) the HCDR1, HCDR2, HCDR3, and LCDR1, LCDR2, and LCDR3 comprise the amino acid sequences set forth in SEQ ID NO: 1, SEQ ID NO: 2, SEQ ID NO: 3, SEQ ID NO: 7, SEQ ID NO: 8, and SEQ ID NO: 9, respectively, or consist of the amino acid sequences set forth in SEQ ID NO: 1, SEQ ID NO: 2, SEQ ID NO: 3, SEQ ID NO: 7, SEQ ID NO: 8, and SEQ ID NO: 9, respectively; or

(ii) the HCDR1, HCDR2, HCDR3, and LCDR1, LCDR2, and LCDR3 comprise the amino acid sequences set forth in SEQ ID NO: 13, SEQ ID NO: 14, SEQ ID NO: 15, SEQ ID NO: 18, SEQ ID NO: 19, and SEQ ID NO: 20, respectively, or consist of the amino acid sequences set forth in SEQ ID NO: 13, SEQ ID NO: 14, SEQ ID NO: 15, SEQ ID NO: 18, SEQ ID NO: 19, and SEQ ID NO: 20, respectively.

In some embodiments, the present invention relates to an anti-CD40L antibody or an antigen-binding fragment thereof, which comprises a heavy chain variable region (VH), wherein the heavy chain variable region comprises or consists of an amino acid sequence having at least 90% identity to an amino acid sequence selected from SEQ ID NO: 4 or 16, or comprises or consists of an amino acid sequence selected from SEQ ID NO: 4 or 16.

In some embodiments, the present invention relates to an anti-CD40L antibody or an antigen-binding fragment thereof, which comprises a light chain variable region (VL), wherein the light chain variable region comprises or consists of an amino acid sequence having at least 90% identity to an amino acid sequence selected from SEQ ID NO: 10 or 21, or comprises or consists of an amino acid sequence selected from SEQ ID NO: 10 or 21.

In some embodiments, the present invention relates to an anti-CD40L antibody or an antigen-binding fragment thereof, which comprises a heavy chain variable region and a light chain variable region, wherein

(i) the heavy chain variable region comprises or consists of the amino acid sequence set forth in SEQ ID NO: 4, or an amino acid sequence having at least 90%, 91%, 92%, 93%, 94%, 95%, 96%, 97%, 98%, or 99% identity thereto, and the light chain variable region comprises or consists of the amino acid sequence set forth in SEQ ID NO: 10, or an amino acid sequence having at least 90%, 91%, 92%, 93%, 94%, 95%, 96%, 97%, 98%, or 99% identity thereto;

(ii) the heavy chain variable region comprises or consists of the amino acid sequence set forth in SEQ ID NO: 16, or an amino acid sequence having at least 90%, 91%, 92%, 93%, 94%, 95%, 96%, 97%, 98%, or 99% identity thereto, and the light chain variable region comprises or consists of the amino acid sequence set forth in SEQ ID NO: 21, or an amino acid sequence having at least 90%, 91%, 92%, 93%, 94%, 95%, 96%, 97%, 98%, or 99% identity thereto.

In some embodiments, the present invention relates to an anti-CD40L antibody or an antigen-binding fragment thereof, which comprises a heavy chain variable region and a light chain variable region, wherein

(i) the heavy chain variable region comprises or consists of the amino acid sequence set forth in SEQ ID NO: 4, and the light chain variable region comprises or consists of the amino acid sequence set forth in SEQ ID NO: 10; or

(ii) the heavy chain variable region comprises or consists of the amino acid sequence set forth in SEQ ID NO: 16, and the light chain variable region comprises or consists of the amino acid sequence set forth in SEQ ID NO: 21.

In some embodiments, the present invention relates to an anti-CD40L antibody or an antigen-binding fragment thereof, which comprises an Fc region.

In some embodiments, the present invention relates to an anti-CD40L antibody or an antigen-binding fragment thereof, wherein the Fc region is derived from a human IgG Fc, for example, from a human IgG1 Fc, a human IgG2 Fc, a human IgG3 Fc, or a human IgG4 Fc.

In some embodiments, the present invention relates to an anti-CD40L antibody or an antigen-binding fragment thereof, wherein the Fc region comprises mutation(s) that reduce or eliminate effector function and/or mutation(s) that eliminate a binding to FcγR while retaining an affinity for FcRn. In some embodiments, the mutations are L234A/L235A and P329 deletion mutations. In some embodiments, the Fc region

(i) comprises or consists of the amino acid sequence set forth in SEQ ID NO: 23 or SEQ ID NO: 24;

(ii) comprises an amino acid sequence having at least 90% identity, for example, 95%, 96%, 97%, 99%, or higher identity to the amino acid sequence set forth in SEQ ID NO: 23; or

(iii) comprises an amino acid sequence having at least 90% identity, for example, 95%, 96%, 97%, 99%, or higher identity to the amino acid sequence set forth in SEQ ID NO: 24, and comprising the L234A/L235A and P329 deletion mutations.

In some embodiments, the present invention relates to an anti-CD40L antibody or an antigen-binding fragment thereof, which comprises a heavy chain constant region, wherein the heavy chain constant region is derived from the IgG1, IgG2, IgG3, or IgG4 constant region. Preferably, the heavy chain constant region

(i) comprises or consists of an amino acid sequence selected from SEQ ID NO: 5 or 26;

(ii) comprises an amino acid sequence having at least 85%, 90%, 91%, 92%, 93%, 94%, 95%, 96%, 97%, 98%, or 99% identity to the amino acid sequence set forth in SEQ ID NO: 26; or

(iii) comprises an amino acid sequence having at least 85%, 90%, 91%, 92%, 93%, 94%, 95%, 96%, 97%, 98%, or 99% identity to the amino acid sequence set forth in SEQ ID NO: 5, and comprises the L234A/L235A and P329 deletion mutations.

In some embodiments, the present invention relates to an anti-CD40L antibody or an antigen-binding fragment thereof, which comprises a light chain constant region, wherein the light chain constant region is a lambda or kappa light chain constant region. Preferably, the light chain constant region

(i) comprises or consists of an amino acid sequence having at least 85%, 90%, 91%, 92%, 93%, 94%, 95%, 96%, 97%, 98%, or 99% identity to the amino acid sequence set forth in SEQ ID NO: 11; or

(ii) comprises or consists of the amino acid sequence set forth in SEQ ID NO: 11.

In some embodiments, the present invention relates to an anti-CD40L antibody or an antigen-binding fragment thereof, which comprises a heavy chain, wherein the heavy chain

(i) comprises or consists of an amino acid sequence having at least 85%, 90%, 91%, 92%, 93%, 94%, 95%, 96%, 97%, 98%, or 99% identity to an amino acid sequence selected from SEQ ID NO: 6 or 17; or

(ii) comprises or consists of an amino acid sequence selected from SEQ ID NO: 6 or 17.

In some embodiments, the present invention relates to an anti-CD40L antibody or an antigen-binding fragment thereof, which comprises a light chain, wherein the light chain

(i) comprises or consists of an amino acid sequence having at least 85%, 90%, 91%, 92%, 93%, 94%, 95%, 96%, 97%, 98%, or 99% identity to an amino acid sequence selected from SEQ ID NO: 12 or 22; or

(ii) comprises or consists of an amino acid sequence selected from SEQ ID NO: 12 or 22.

In some embodiments, the present invention relates to an anti-CD40L antibody or an antigen-binding fragment thereof, which comprises a heavy chain and light chain, wherein

(i) the heavy chain comprises or consists of an amino acid sequence having at least 85%, 90%, 91%, 92%, 93%, 94%, 95%, 96%, 97%, 98%, or 99% identity to the amino acid sequence set forth in SEQ ID NO: 6; and the light chain comprises or consists of an amino acid sequence having at least 85%, 90%, 91%, 92%, 93%, 94%, 95%, 96%, 97%, 98%, or 99% identity to the amino acid sequence set forth in SEQ ID NO: 12;

(ii) the heavy chain comprises or consists of an amino acid sequence having at least 85%, 90%, 91%, 92%, 93%, 94%, 95%, 96%, 97%, 98%, or 99% identity to the amino acid sequence set forth in SEQ ID NO: 17; and the light chain comprises or consists of an amino acid sequence having at least 85%, 90%, 91%, 92%, 93%, 94%, 95%, 96%, 97%, 98%, or 99% identity to the amino acid sequence set forth in SEQ ID NO: 22.

In some embodiments, the present invention relates to an anti-CD40L antibody or an antigen-binding fragment thereof, which comprises a heavy chain and a light chain, wherein

(i) the heavy chain comprises or consists of the amino acid sequence set forth in SEQ ID NO: 6, and the light chain comprises or consists of the amino acid sequence set forth in SEQ ID NO: 12;

(ii) the heavy chain comprises or consists of the amino acid sequence set forth in SEQ ID NO: 17, and the light chain comprises or consists of the amino acid sequence set forth in SEQ ID NO: 22.

In some embodiments, the antibody is a monoclonal antibody. In some embodiments, the antibody is a humanized antibody or a chimeric antibody. In some embodiments, the antigen-binding fragment is an antibody fragment selected from the group consisting of: Fv, Fab, Fab', Fab'-SH, F(ab')$_2$, dAb (domain antibody), a linear antibody, a single-chain antibody (e.g., scFv), a single-domain antibody (sdAb) such as VHH, a bivalent antibody or fragment thereof, or a camelid antibody or diabody.

In some embodiments, the anti-CD40L antibody or an antigen-binding fragment thereof exhibits one or more the following properties:

a) binding to CD40L (e.g., human CD40L) with high affinity;

b) inhibiting the binding of CD40 to CD40L;

c) inhibiting the CD40/CD40L signaling pathway;

d) inhibiting activation or proliferation of B cells;

e) inhibiting proliferation of B cells;

f) inhibiting maturation and differentiation of B cells, for example, inhibiting differentiation of B cells into plasma cells;

g) inhibiting IL-12 secretion by dendritic cells;

h) not inducing platelet activation;

i) treating or preventing an immune disease, such as multiple sclerosis (MS); and/or

j) inhibiting an immune response, preferably a humoral immune response, for example, by inhibiting antibody production;

k) improving pharmacokinetics, preferably having an in vivo half-life of about 200 hours or more, more preferably about 210, 220, 230, 240, 250, 260, 270, 280, or 290 hours or more.

In some embodiments, the present invention relates to an isolated nucleic acid encoding the anti-CD40 antibody of the present invention or an antigen-binding fragment thereof. In some embodiments, the present invention relates to a vector comprising the nucleic acid, preferably an expression vector.

In some embodiments, the present invention relates to a host cell comprising the nucleic acid or vector of the present invention. Preferably, the host cell is prokaryotic or eukaryotic, more preferably selected from a yeast cell, a mammalian cell (e.g., a 293 cell or a CHO cell, such as a CHO-K cell or a HEK293 cell), or another cell suitable for producing an antibody or an antigen-binding fragment thereof.

In some embodiments, the present invention relates to a method for producing an anti-CD40L antibody or an antigen-binding fragment thereof, wherein the method comprises

1) culturing a host cell of the present invention under conditions suitable for expressing a nucleic acid encoding the anti-CD40L antibody or the antigen-binding fragment thereof of the present invention;

m) optionally isolating the antibody or antigen-binding fragment thereof;

n) optionally, the method further comprises recovering the anti-CD40L antibody or the antigen-binding fragment thereof from the host cell, wherein the antibody is optionally purified, for example, by Protein A.

In some embodiments, the present invention relates to an immunoconjugate comprising the anti-CD40L antibody or the antigen-binding fragment thereof of the present invention, and other substance, such as a therapeutic agent, for example an agent that can produce a biological effect, including but not limited to a cytokine, another antibody, a small-molecule drug, or an immunomodulator (e.g., an immunosuppressant).

In some embodiments, the present invention relates to a pharmaceutical composition comprising the anti-CD40L antibody or the antigen-binding fragment thereof of the present invention, or the immunoconjugate of the present invention, and optionally a pharmaceutically acceptable auxiliary material.

In some embodiments, the present invention relates to a pharmaceutical combination product comprising the anti-CD40L antibody or the antigen-binding fragment thereof of the present invention, or the immunoconjugate of the present invention, and one or more other therapeutic agents, for example agents that can produce a biological effect, including but not limited to cytokines, other antibodies, small-molecule drugs, or immunomodulators (e.g., immunosuppressants).

In some embodiments, the present invention relates to a method for preventing or treating a disease or disorder associated with an inappropriate activation of a CD40L/CD40-mediated pathway in a subject, wherein the method comprises administering to the subject an effective amount of the anti-CD40L antibody or an antigen-binding fragment thereof, or the immunoconjugate, or the pharmaceutical composition, or the pharmaceutical combination product of the present invention.

In some embodiments, the subject exhibits, compared to a healthy individual, an abnormal activation of a CD40L/CD40-mediated signaling pathway and/or excessive production and deposition of autoantibodies; and/or increased expression of CD40L or CD40 on the subject's cells (e.g., activated T cells or other types of immune cells or non-immune cells, such as epithelial cells, monocytes, dendritic cells, fibroblasts, smooth muscle cells, endothelial cells, and platelets), for example, compared to the corresponding cells of a healthy individual.

In some embodiments, the disease or disorder is selected from an autoimmune disease, lupus nephritis, immune thrombocytopenic purpura (ITP), transplant rejection, Crohn's disease, inflammatory bowel disease (IBD), colitis, asthma/allergy, atherosclerosis, myasthenia gravis, multiple sclerosis, psoriasis, rheumatoid arthritis, ankylosing spondylitis, coronary heart disease, type 1 diabetes, amyotrophic lateral sclerosis (ALS), and an immune response to recombinant drug products, such as Factor VII for hemophilia.

**Brief Description of the Drawings**

FIG. 1 shows that the antibody of the present invention inhibits the binding of CD40 to CD40L at the molecular level.

FIG. 2 shows that the antibody of the present invention inhibits activation of downstream signaling in Jurkat cells overexpressing CD40 at the cellular level.

FIG. 3 shows the inhibition for expression of CD86, the B cell activation marker, by the antibody of the present invention.

FIG. 4 shows the effect of the antibody of the present invention on the proliferation of B cells isolated in vitro.

FIG. 5 shows the effect of the antibody of the present invention on the differentiation of B cells into plasma cells.

FIG. 6 shows the effect of the antibody of the present invention on IL-12 secretion by moDCs.

FIGS. 7-9 show that the antibody of the present invention does not induce platelet activation in in vitro experiments.

FIGS. 10-12 show data for the antibody of the present invention in an experimental autoimmune encephalomyelitis (EAE) mouse model.

FIG. 13 shows that the antibody of the present invention inhibits the production of anti-keyhole limpet hemocyanin antibodies in mice.

FIG. 14 shows the pharmacokinetics of the antibody of the present invention in mice.

**Detailed Description of the Invention**

**I. Definitions**

Before the detailed description of the present invention, it should be understood that the present invention is not limited to the particular methodologies, embodiments, and reagents described herein, as these may vary. The terms used herein should be understood to describe the specific embodiments only and are not intended to limit the scope of the present invention; the scope of the present invention is defined only by the appended Claims. Unless otherwise defined, all technical and scientific terms used herein have the same meaning as those typically understood by those of ordinary skill in the art of the present invention.

For the purpose of explaining this Specification, the following definitions are provided, and, where appropriate, terms used in the singular may also include the plural, and vice versa. It should be understood that the terms used herein are solely for describing particular embodiments and are not intended to be limiting.

The term "approximately", when used in conjunction with a numerical value, is intended to encompass numerical values within a range having a lower limit that is 5% less than the specified numerical value and an upper limit that is 5% greater than the specified numerical value.

As used herein, the term "and/or" is intended to mean any one of the options, or any two or more of the options, or all of the options.

As used herein, the terms "including" or "comprising" are intended to mean including the recited elements, integers, or steps, but not excluding any other elements, integers, or steps. As used herein, the terms "including" or "comprising", unless otherwise specified, also encompass situations in which the recited elements, integers, or steps are combined. For example, when referring to an antibody variable region "comprising" a particular sequence, it is also intended to encompass an antibody variable region consisting of that particular sequence.

The term "CD40L" refers to the ligand expressed on activated T cells. Other names known in the art include CD154, CD40 ligand (CD40L), CD40 counter receptor (CD40CR), gp39, T-BAM, T-cell activation molecule, TRAF, TNF receptor-associated protein (TRAP), and tumor necrosis factor ligand superfamily member 5 (TNFSF5). These terms may be used interchangeably throughout the application. In some embodiments, the amino acid sequence of CD40L is as shown in UniProt P29965. In some embodiments, CD40L comprises the sequence set forth in SEQ ID NO: 27.

The terms "whole antibody" or "full-length antibody" may be used interchangeably herein and refer to an antibody molecule having the natural immunoglobulin molecular structure. In the case of a conventional four-chain IgG antibody, a full-length antibody comprises two heavy chains (H) and two light chains (L) interconnected by disulfide bonds. In the case of a heavy-chain antibody that only has heavy chains and lacks light chains, a full-length antibody comprises two heavy chains (H) interconnected by disulfide bonds. For a conventional four-chain IgG antibody, the heavy chain of a full-length antibody typically consists of a heavy chain variable region (abbreviated herein as VH) and a heavy chain constant region, wherein the heavy chain constant region comprises at least three domains: CH1, CH2, and CH3. The light chain of a full-length antibody consists of a light chain variable region (abbreviated herein as VL) and a light chain constant region, wherein the light chain constant region consists of the CL domain. Each heavy chain variable region VH and each light chain variable region VL consists of three CDRs and four FRs, arranged in the following order from the amino-terminus to the carboxyl-terminus: FR1, CDR1, FR2, CDR2, FR3, CDR3, FR4. The term "antibody fragment" encompasses a portion of an intact antibody. In a preferred embodiment, the antibody fragment is an antigen-binding fragment.

The term "antigen-binding fragment" of an antibody refers to a molecule distinct from the full-length antibody, which comprises a portion of the full-length antibody, but is capable of binding the antigen of the full-length antibody or competing with the full-length antibody (i.e., the full-length antibody from which the antigen-binding fragment is derived) for binding to the antigen. An antigen-binding fragment can be prepared by recombinant DNA techniques or by enzymatic or chemical cleavage of a full-length antibody. The antigen-binding fragment includes, but is not limited to, Fv, Fab, Fab', Fab'-SH, F(ab')$_2$, dAb (domain antibody), a linear antibody, a single-chain antibody (e.g., scFv); a single-domain antibody (sdAb) such as VHH, a bivalent antibody or fragment thereof, or a camelid antibody or diabody. For example, a Fab fragment may be obtained from papain digestion of the full-length antibody. In addition, full-length antibodies can be digested with pepsin below the disulfide bonds in the hinge region to produce F(ab')2, which is a dimer of Fab' and is a bivalent antibody fragment. F(ab')2 may be reduced under neutral conditions by breaking the disulfide bond in the hinge region, thereby transforming the F(ab')2 dimer into Fab' monomers. The Fv fragment consists of the VL and VH domains of one arm of an antibody. The two domains of an Fv fragment, VL and VH, can be encoded by separate genes, but they can also be linked using recombinant methods with a synthetic linker peptide to produce them as a single protein chain, in which the VL and VH regions pair to form a single-chain Fv (scFv).

"Fab fragment" or "Fab" may be used interchangeably herein to refer to an immunoglobulin fragment consisting of two polypeptide chains, which comprises an immunoglobulin heavy chain variable domain VH, a heavy chain constant domain CH1, a light chain variable domain VL, and a light chain constant domain CL, wherein one polypeptide chain comprises VH and one constant region selected from CH1 and CL from the N-terminus to the C-terminus, and the other polypeptide chain comprises VL and another constant region selected from CL and CH1 from the N-terminus to the C-terminus, wherein the VH domain and VL domain are paired to form an antigen-binding site. As used herein, the Fab polypeptide chain comprising the heavy chain constant region CH1 is also referred to as the "Fab heavy chain"; correspondingly, the Fab polypeptide chain comprising the light chain constant region CL is also referred to as the "Fab light chain".

A "diabody" is a small bivalent antibody constructed by gene fusion, for example, a dimer consisting of two polypeptide chains. In a diabody, the VL and VH domains of each polypeptide chain are linked by a connector, so that the VL and VH encoded in the same polypeptide chain form a dimer with distinct single-chain variable fragments. A diabody generally has two antigen-binding sites.

The "complementarity-determining region" or "CDR region" or "CDR" is a region within the variable domain of an antibody, which is highly variable in sequence and forms structurally defined loops ("hypervariable loops") and/or contains antigen-contacting residues ("antigen contact points"). The CDR is mainly responsible for binding with antigen epitopes. The CDRs of heavy chains and light chains are typically referred to as CDR1, CDR2, and CDR3, and numbered sequentially starting from the N-terminus. CDRs located in the heavy chain variable domain of an antibody are referred to as HCDR1, HCDR2, and HCDR3, while CDRs located in the light chain variable domain of an antibody are referred to as LCDR1, LCDR2, and LCDR3. In a given amino acid sequence of a light chain variable region or a heavy chain variable region, the precise amino acid sequence boundaries of each CDR may be determined using any one of various known antibody CDR assignment schemes or a combination thereof, which comprise, for example, Chothia (Chothia et al., (1989) Nature 342: 877-883, Al-Lazikani et al., "Standard conformations for the canonical structures of immunoglobulins", Journal of Molecular Biology, 273, 927-948, (1997)), based on three-dimensional structures of antibodies and the topology of CDR loops; Kabat (Kabat et al., Sequences of Proteins of Immunological Interest, 4th Edition, U.S. Department of Health and Human Services, National Institutes of Health (1987)), based on antibody sequence variability; AbM (University of Bath); Contact (University College London); the international ImMunoGeneTics database (IMGT) (available on the World Wide Web at http://imgt.cines.fr/); and North CDR definition, based on affinity propagation clustering using a large number of crystal structures.

Unless otherwise specified, as used in the present invention, the terms "CDR" or "CDR sequence" encompass CDR sequences determined by any one of the above methods or any combination thereof. CDRs may also be determined based on having the same Kabat numbering positions as a reference CDR sequence (e.g., any one of the exemplary CDRs of the present invention). Unless otherwise indicated, as used in the present invention, when referring to residue positions in an antibody variable region (including residues of a heavy chain variable region and a light chain variable region), such positions are based on the numbering according to the Kabat numbering system (Kabat et al., Sequences of Proteins of Immunological Interest, 5th Ed., Public Health Service, National Institutes of Health, Bethesda, Md., (1991)).

In some embodiments, the CDRs of the heavy chain variable region of the antibody of the present invention are determined according to Kabat or Chothia, or a combination of Kabat and Chothia. In some embodiments, the CDRs of the light chain variable region of the antibody of the present invention are determined according to Kabat.

In some embodiments, CDR1 of the heavy chain variable region of the antibody of the present invention is determined according to a combination of the Kabat and the Chothia schemes (hereinafter referred to as the "Kabat and Chothia combination", corresponding to H26-H32 in the Kabat numbering system), while CDR2 and CDR3 of the heavy chain variable region, as well as LCDR1, LCDR2, and LCDR3 of the light chain variable region, are determined according to the Kabat scheme.

| CDR | Kabat Scheme | Kabat and Chothia Combination |
|---|---|---|
| LCDR1 (Kabat and Chothia numbering systems) | L24-L34 | |
| LCDR2 (Kabat and Chothia numbering systems) | L50-L56 | |
| LCDR3 (Kabat and Chothia numbering systems) | L89-L97 | |
| HCDR1 (Kabat numbering system) | H31-H35 | H26-H32 |

(continued)

| CDR | Kabat Scheme | Kabat and Chothia Combination |
|---|---|---|
| HCDR2 (Kabat and Chothia numbering systems) | H50-H65 | |
| HCDR3 (Kabat and Chothia numbering systems) | H95-H102 | |

"An antibody that binds to the same or overlapping epitope" as a reference antibody refers to an antibody that, in a competitive assay, blocks 50%, 60%, 70%, 80%, 90%, or more than 95% of the binding of the reference antibody to its antigen; conversely, the reference antibody blocks 50%, 60%, 70%, 80%, 90%, or more than 95% of the binding of the antibody to its antigen in a competitive assay.

"An antibody that competes for binding to the antigen" with a reference antibody refers to an antibody that, in a competitive assay, blocks 50%, 60%, 70%, 80%, 90%, or more than 95% of the binding of the reference antibody to its antigen. Conversely, in a competitive assay, the reference antibody blocks 50%, 60%, 70%, 80%, 90%, or more than 95% of the binding of the antibody to its antigen. Various types of competitive binding assays can be used to determine whether one antibody competes with another. Such assays include, for example, solid-phase direct or indirect radioimmunoassays (RIA), solid-phase direct or indirect enzyme immunoassays (EIA), and sandwich and competitive assays.

"An antibody that inhibits (e.g., competitively inhibits) the binding of a reference antibody to its antigen" refers to an antibody that inhibits 50%, 60%, 70%, 80%, 90%, or more than 95% of the binding of the reference antibody to its antigen. Conversely, the reference antibody inhibits 50%, 60%, 70%, 80%, 90%, or more than 95% of the binding of the antibody to its antigen. The binding of an antibody to its antigen can be measured by affinity (e.g., the equilibrium dissociation constant). Methods for determining affinity are known in the art.

"An antibody that exhibits the same or similar binding affinity and/or specificity as a reference antibody" refers to an antibody that can possess at least 50%, 60%, 70%, 80%, 90%, or more than 95% of the binding affinity and/or specificity of the reference antibody. This can be determined using any method known in the art for assessing binding affinity and/or specificity.

The term "chimeric antibody" refers to an antibody molecule in which (a) the constant region, or a portion thereof, is changed, replaced, or exchanged, so that the antigen-binding site is linked to a constant region of a different or changed class, effector function, and/or species, or to a completely different molecule that imparts new properties to the chimeric antibody (e.g., an enzyme, toxin, hormone, growth factor, or drug); or (b) the variable region, or a portion thereof, is changed, replaced, or exchanged with a variable region having a different or changed antigen specificity. For example, a mouse antibody may be modified by replacing its constant region with a constant region derived from a human immunoglobulin. Due to the replacement with a human constant region, the chimeric antibody can retain its specificity for recognizing the antigen while exhibiting reduced immunogenicity in humans compared to the original mouse antibody.

A "humanized antibody" is an antibody that retains the antigen-specific reactivity of a non-human antibody (e.g., a mouse monoclonal antibody) while low immunogenicity when administered to humans as a therapeutic agent. This may be achieved, for example, by retaining the non-human antigen-binding sites and replacing the remaining parts of the antibody with their corresponding human counterparts (e.g., the constant region and the portions that are not involved in binding in the variable region are replaced with the corresponding parts of a human antibody).

The terms "Fc domain" or "Fc region," as used herein, refer to the C-terminal region of an immunoglobulin heavy chain that contains at least a portion of the constant region. The term encompasses both native-sequence Fc regions and variant Fc regions. The natural immunoglobulin "Fc domain" comprises two or three constant domains, that is, the CH2 domain, CH3 domain, and optionally the CH4 domain. For example, in a native antibody, the immunoglobulin Fc domain comprises the second and the third constant domains (CH2 domain and CH3 domain) of the two heavy chains derived from IgG, IgA, or IgD antibodies; or comprises the second, the third, and the fourth constant domains (CH2 domain, CH3 domain, and CH4 domain) of the two heavy chains derived from IgM or IgE antibodies. Unless otherwise indicated herein, amino acid residue numbering in the Fc region or the heavy chain constant region is according to the EU numbering system (also known as the EU index) as described in Kabat et al., Sequences of Proteins of Immunological Interest, 5th Edition, Public Health Service, National Institutes of Health, Bethesda, MD, 1991, NIH Publication 91-3242. However, the C-terminal lysine (Lys447) in the Fc region may or may not exist. Two Fc regions can dimerize to form a homodimeric Fc, and two different Fc regions can heterodimerize to form a heterodimeric Fc. As used herein, the terms "Fc region", "Fc portion", and "dimeric Fc" do not include the heavy chain variable region VH, or the light chain variable region VL, or the heavy chain constant region CH1, or the light chain constant region CL of an immunoglobulin, but may include the hinge region or a portion of the hinge region at the N-terminus of the heavy chain constant region in some cases, eg., the hinge region or a portion of the hinge region of IgG1, such as a sequence of D221 to P230 according to the EU numbering system. In an embodiment, the Fc region of a human IgG heavy chain extends from Asp221, or from Cys226, or from Asp231 to the carboxyl-terminus of the heavy chain.

In an embodiment, the Fc region polypeptide of a human IgG1 (including the hinge region) comprises the following amino acid sequence:

DKTHTCPPCPAPELLGGPSVFLFPPKPKDTLMISRTPEVTCVVVVDVSHEDPEVKFNWYVD
GVEVHNAKTKPREEQYNSTYRVVSVLTVLHQDWLNGKEYKCKVSNKALPAPIEKTISKAK
GQPREPQVYTLPPSRDELTKNQVSLTCLVKGFYPSDIAVEWESNGQPENNYKTTPPVLDSDG
SFFLYSKLTVDKSRWQGNVFSCSVMHEALHNHYTQKSLSLSPGK (SEQ ID NO: 23; the

underlined portion corresponds to the hinge region).

The term "effector function" refers to the functional ability of an antibody's Fc or constant region to bind to proteins and/or cells of the immune system.

The terms "amino acid substitution" and "amino acid replacement" are used interchangeably herein and refer to the replacement of at least one amino acid residue in a predetermined parent amino acid sequence with a different "substituted" amino acid residue. This substituted residue or more residues may be "naturally occurring amino acid residues" (i.e., encoded by the genetic code) and selected from the group consisting of alanine (Ala); arginine (Arg); asparagine (Asn); aspartate (Asp); cysteine (Cys); glutamine (Gln); glutamic acid (Glu); glycine (Gly); histidine (His); isoleucine (Ile); leucine (Leu); lysine (Lys); methionine (Met); phenylalanine (Phe); proline (Pro); serine (Ser); threonine (Thr); tryptophan (Trp); tyrosine (Tyr); and valine (Val). The definition of amino acid substitution herein also encompasses substitution with one or more non-naturally occurring amino acid residues. "Non-naturally occurring amino acid residues" refer to residues that, aside from those naturally occurring amino acid residues mentioned above, are capable of covalently binding to adjacent amino acid residues in a polypeptide chain. Examples of non-naturally occurring amino acid residues include norleucine, ornithine, norvaline, homoserine, Aib, and other amino acid residue analogs.

In some embodiments, amino acid substitutions are represented as (original amino acid, amino acid position, mutated amino acid). For example, when the substitution site is located in the C region, "L234A" means that the leucine (L) at EU position 234 is substituted with alanine (A). When referring to mutation combinations, the mutations in a combination are connected by a slash. "L234A/L235A" indicates that both mutations L234A and L235A are included.

In some embodiments, amino acid deletions are represented by △ + amino acid + mutation site. For example, △P329 indicates the deletion of proline at position 329. Amino acid deletions can also be represented as "amino acid + mutation site + deletion." For example, the deletion of proline at position 329 can also be written as "P329 deletion."

As used herein, "conservative changes" include replacements, deletions, or additions to the polypeptide sequence that do not substantially alter the desired functional activity of the polypeptide. In some embodiments, conservative changes are conservative replacements. A conservative replacement refers to the replacement of one amino acid with another one from the same class, such as replacing one acidic amino acid with another acidic amino acid, one basic amino acid with another basic amino acid, or one neutral amino acid with another neutral amino acid. For example, a conservative replacement often results in the replacement of one amino acid with a chemically similar amino acid. Conservative replacement tables that provide functionally similar amino acids are well known in the art. Eight sets of amino acids containing amino acids that are conservative replacements for each other are listed below: 1) alanine (A), glycine (G); 2) aspartate (D), glutamic acid (E); 3) asparagine (N), glutamine (Q); 4) arginine (R), lysine (K); 5) isoleucine (I), leucine (L), methionine (M), valine (V); 6) phenylalanine (F), tyrosine (Y), tryptophan (W); 7) serine (S), threonine (T); and 8) cysteine (C), methionine (M). In some embodiments, the term "conservative modification," when applied to the amino acid sequence of an antibody, refers to an amino acid modification that does not significantly affect or change the target antigen-binding characteristics of the antibody molecule of the present invention containing the amino acid sequence. For example, a conservatively changed variant maintains at least 80%, 85%, 90%, 95%, 98%, 99% or higher, such as 100-110% or higher binding affinity for a target antigen relative to the parent antibody.

As used herein, the term "vector" refers to a nucleic acid molecule capable of replicating another nucleic acid that is linked thereto. This term includes vectors as self-replicating nucleic acid structures and vectors integrated into the genome of a host cell into which it has been introduced. Some vectors are capable of guiding the expression of the nucleic acid they are operably linked to. Such vectors are referred to as "expression vectors" herein.

An "immunoconjugate" is an antibody conjugated to one or more other substances (including but not limited to a label).

As used herein, the term "therapeutic agent" encompasses any agents effective in preventing or treating diseases associated with inappropriate activation of the CD40L/CD40-mediated pathway, for example, an agent that produces a biological effect. Such agents that can produce a biological effect include, but are not limited to, cytokine, other antibodies, small-molecule drug, or immunomodulator (e.g., immunosuppressant).

The term "small molecule drug" refers to an organic compound with low molecular weight capable of modulating a biological process. A "small molecule" is defined as a molecule that has a molecular weight of less than 10 kD, typically less than 2 kD, and preferably less than 1 kD. Small molecules include, but are not limited to, inorganic molecules, organic molecules, organic molecules containing inorganic components, molecules containing radioactive atoms, synthetic molecules, peptide mimetics, and antibody mimetics. As a therapeutic agent, small molecules can penetrate cells more readily, be less susceptible to degradation, and be less likely to elicit an immune response compared to large molecules.

As used herein, the term "immunomodulator" refers to a natural or synthetic agent or a drug that suppresses or modulates an immune response. The immune response can be a humoral response or a cellular response. Immunomodulators include immunosuppressants.

As used herein, "immunosuppressant," "immunosuppressive drug," or "immunosuppressive substances" refers to a therapeutic agent used in immunosuppressive therapy to inhibit or prevent immune system activity.

The term "effective amount" refers to an amount or dose of the antibody of the present invention, or fragment or conjugate or composition or combination thereof that, when administered to a patient in a single or multiple doses, produces the desired effect in a patient in need of treatment or prevention.

A "therapeutically effective amount" refers to an amount effective to achieve a desired therapeutic result at a desired dose and for a desired period of time. A therapeutically effective amount is also an amount in which any toxic or harmful effects of the antibody or antibody fragment, or conjugate or composition or combination thereof do not outweigh the therapeutic benefits. Relative to an untreated subject, a "therapeutically effective amount" preferably inhibits a measurable parameter (e.g., tumor volume) by at least about 20%, more preferably by at least about 40%, and even more preferably by at least about 50%, 60%, or 70%. In some embodiments, as used herein, the term "therapeutically effective amount" is intended to specify an amount necessary in a treatment regimen to treat a condition (e.g., autoimmune disease, allergy, inflammation, GVHD, or transplantation) or to reduce or eliminate an immune response, particularly a T cell or B cell antibody response to an antigen (e.g., a self-antigen, allergen, inflammation-inducing agent, transplanted cell, tissue, or organ) or to an anti-drug (e.g., a biologic such as a therapeutic antibody, Ig fusion protein, hormone, growth factor, or other therapeutic protein or peptide).

A "prophylactically effective amount" refers to an amount effective to achieve a desired prophylactic result at a desired dose and for a desired period of time. Typically, because a prophylactic dose is administered to a subject before or at an early stage of a disease, a prophylactically effective amount is lower than a therapeutically effective amount. In some embodiments, as used herein, the term "prophylactically effective amount" is intended to specify an amount necessary in a treatment regimen to prevent the progression and symptoms of a condition or disease (e.g., autoimmune disease, allergy, inflammation, GVHD, or anti-transplant, drug, or rejection responses).

The terms "host cell," "host cell line," and "host cell culture" are used interchangeably and refer to a cell into which exogenous nucleic acid has been introduced, including the progenies of such a cell. Host cells include "transformants" and "transformed cells," which encompass both the initially transformed cells and their progenies, regardless of the number of generations. The progenies may not be identical to the parent cell in nucleic acid content and may contain mutations. Included herein are mutant progenies that were screened or selected from the initially transformed cells and that retain the same function or biological activity.

As used herein, the term "label" refers to a compound or composition that is directly or indirectly conjugated or fused to a reagent (such as a polynucleotide probe or antibody) and facilitates the detection of the reagent to which it is conjugated or fused. The label itself can be detectable (e.g., a radioactive isotope label or fluorescent label) or, in the case of an enzymatic label, can catalyze a chemical change in a detectable substrate compound or composition. The term is intended to encompass both direct labeling of a probe or antibody, in which a detectable substance is conjugated (i.e., physically linked) to the probe or antibody, and indirect labeling, in which the probe or antibody is labeled through reaction with another reagent that is directly labeled.

The terms "individual" or "subject" include mammals. Mammals include, but are not limited to, domesticated animals (e.g., cows, sheep, cats, dogs, and horses), primates (e.g., humans and non-human primates such as monkeys), rabbits, and rodents (e.g., mice and rats). In some embodiments, the individual or subject is a human.

"Subject/patient/individual sample" refers to a collection of cells or fluids obtained from a patient or subject. The source of a tissue or cell sample can be solid tissue, such as from fresh, frozen, and/or preserved organ or tissue samples, or biopsy samples, or aspirate samples; blood or any blood component; bodily fluids, such as cerebrospinal fluid, amniotic fluid, peritoneal fluid (ascites), or interstitial fluid; or cells from a subject at any stage of pregnancy or development. Tissue samples may include compounds not naturally present in the tissue in nature, such as preservatives, anticoagulants, buffers, fixatives, nutrients, antibiotics, and the like.

An "isolated" antibody is an antibody that has been separated from the components of its natural environment. In some embodiments, the antibody is purified to greater than 95% or 99% purity, as determined, for example, by electrophoresis (e.g., SDS-PAGE, isoelectric focusing (IEF), capillary electrophoresis) or chromatography (e.g., ion exchange or reverse-phase HPLC).

An "isolated nucleic acid encoding an anti-CD40L antibody or a fragment thereof" refers to one or more nucleic acid molecules that encode the heavy chain or light chain (or fragments thereof, such as the heavy chain variable region or light chain variable region) of an antibody, including such nucleic acid molecules on a single vector or on separate vectors, as well as such nucleic acid molecules present at one or more locations within a host cell.

The "percent identity (%)" of an amino acid sequence refers to the percentage of amino acid residues in a candidate sequence that are identical to the amino acid residues of the specific amino acid sequence disclosed in this Specification, after aligning the candidate sequence with the specific amino acid sequence disclosed herein and introducing gaps if necessary, to maximize the percent sequence identity, and without considering any conservative replacements as part of the sequence identity. In some embodiments, the present invention contemplates variants of the antibody molecule of the present invention, wherein the variant has a considerable degree of identity, e.g., identity of at least 80%, 85%, 90%, 95%, 97%, 98%, or 99% or higher, relative to the antibody molecules specifically disclosed herein and the sequences thereof. The variant may include conservative changes.

The term "pharmaceutically acceptable auxiliary materials" refers to a diluent, an adjuvant (e.g., Freund's adjuvant (complete or incomplete)), an excipient, a carrier, a stabilizer, or the like, administered together with an agent.

The term "pharmaceutical composition" refers to a composition that is in a form allowing the effectiveness of biological activity of the active components contained therein and that does not contain additional components that would impart unacceptable toxicity to a subject to which the composition would be administered.

The term "pharmaceutical combination" refers to a non-fixed or fixed combination product, including, but not limited to, a kit or a pharmaceutical composition. The term "non-fixed combination" means that the active components (e.g., (i) an anti-CD40L antibody or a fragment thereof, and (ii) other therapeutic agent) are administered as separate entities, either simultaneously, without a specific time limit, or sequentially at the same or different time intervals to a patient, wherein such administration provides prophylactically or therapeutically effective levels of two or more agents in the patient. In some embodiments, the anti-CD40L antibody or fragment thereof and the other therapeutic agent used in the pharmaceutical combination are administered at levels no greater than when they are used individually. The term "fixed combination" means that the two or more agents are administered to a patient simultaneously in the form of a single entity. Preferably, the doses and/or time intervals of two or more agents are selected such that the combined use of each part produces an effect in treating a disease or condition that is greater than what can be achieved by using any one component alone. Each component may be in the form of a separate formulation, which may be the same or different.

The term "combination therapy" refers to the administration of two or more therapeutic agents or treatment modalities (e.g., radiation therapy or surgery) to treat a disease described herein. Such administration includes co-administration of the therapeutic agents in a substantially simultaneous manner, for example, in a single capsule containing the active components in a fixed ratio. Alternatively, such administration includes co-administration of the individual active components in multiple or separate containers (e.g., tablets, capsules, powders, or liquids). Powders and/or liquids can be reconstituted or diluted to the desired dose prior to administration. In addition, such administration also includes using each type of therapeutic agent either at substantially the same time or sequentially at different times. In either case, the treatment regimen will provide the beneficial effects of the pharmaceutical combination in treating the disorders or conditions described herein.

As used herein, "treat", "treating" or "treatment" refers to slowing, interrupting, arresting, alleviating, stopping, reducing, or reversing the onset of symptoms, complications, or biochemical indicators of a disease, relieving symptoms, or preventing or inhibiting the further development of a disease, condition, or disorder.

As used herein, "prevent", "preventing" or "prevention" includes the inhibition on the onset or development of a disease or disorder, or the symptoms of a particular disease or disorder.

All publications, patent applications, patents, and other references mentioned herein are incorporated by reference in their entirety. Any or all of the features discussed above, as well as throughout the present application, may be combined in various embodiments of the present invention. In addition, the materials, methods, and examples described herein are illustrative only and are not intended to be limiting. Other features, objectives and advantages of the present invention will be apparent from this Specification and the drawings, as well as from the appended Claims.

## II. Antibodies

In some embodiments, the anti-CD40L antibody or the antigen-binding fragment thereof of the present invention binds to CD40L (e.g., human CD40L) with high affinity. In some embodiments, the antibody or the antigen-binding fragment thereof of the present invention exhibits a binding affinity to human CD40L that is higher than that of known anti-CD40L antibodies, such as the INX-021 antibody (WO2017011544A1).

In some embodiments, the antibody or the antigen-binding fragment thereof of the present invention binds to CD40L on a cell surface. In some embodiments, CD40L is expressed or overexpressed on the cell surface. In some embodiments, the cells are CHO cells that express or overexpress CD40L.

In some embodiments, the anti-CD40L antibody or the antigen-binding fragment thereof of the present invention exhibits one or more of the following properties:

a) binding to CD40L (e.g., human CD40L) with high affinity;

b) inhibiting the binding of CD40 to CD40L;

c) inhibiting the CD40/CD40L signaling pathway;

d) inhibiting activation of B cells;

e) inhibiting proliferation of B cells;

f) inhibiting maturation and differentiation of B cells, for example, inhibiting differentiation of B cells into plasma cells;

g) inhibiting IL-12 secretion by dendritic cells;

h) non-inducing platelet activation (e.g., in vitro or in vivo);

i) treating or preventing an immune disease, such as multiple sclerosis (MS);

j) inhibiting an immune response, preferably a humoral immune response, for example, by inhibiting antibody production; and/or

k) improving pharmacokinetics, preferably having an in vivo half-life of about 200 hours or more, more preferably about 210, 220, 230, 240, 250, 260, 270, 280, or 290 hours or more.

In some embodiments, the anti-CD40L antibody or the antigen-binding fragment thereof of the present invention comprises three complementarity-determining regions (HCDRs), i.e., HCDR1, HCDR2, and HCDR3 from a heavy chain variable region.

In some embodiments, the anti-CD40L antibody or the antigen-binding fragment thereof of the present invention comprises three complementarity-determining regions (LCDRs), i.e., LCDR1, LCDR2, and LCDR3 from a light chain variable region.

In some embodiments, the anti-CD40L antibody or the antigen-binding fragment thereof of the present invention comprises three complementarity-determining regions(HCDRs) from the heavy chain variable region and three complementarity-determining regions(LCDRs) from the light chain variable region.

In some aspects, the anti-CD40L antibody or the antigen-binding fragment thereof of the present invention comprises a heavy chain variable region (VH). In some aspects, the anti-CD40L antibody or the antigen-binding fragment thereof of the present invention comprises a light chain variable region (VL). In some aspects, the anti-CD40L antibody or the antigen-binding fragment thereof of the present invention comprises a heavy chain variable region (VH) and a light chain variable region (VL). In some embodiments, the heavy chain variable region comprises three complementarity-determining regions (CDRs), HCDR1, HCDR2, and HCDR3 from the heavy chain variable region,. In some embodiments, the light chain variable region comprises three complementarity-determining regions (CDRs), LCDR1, LCDR2, and LCDR3 from the light chain variable region,.

In some embodiments, the anti-CD40L antibody or the antigen-binding fragment thereof of the present invention further comprises an antibody heavy chain constant region HC. In some embodiments, the anti-CD40L antibody or the antigen-binding fragment thereof of the present invention further comprises an antibody light chain constant region LC. In some embodiments, the anti-CD40L antibody or the antigen-binding fragment thereof of the present invention further comprises a heavy chain constant region HC and a light chain constant region LC.

In some embodiments, the heavy chain variable region of the present invention:

(i) comprises or consists of an amino acid sequence having at least 90%, 91%, 92%, 93%, 94%, 95%, 96%, 97%, 98%, or 99% identity to an amino acid sequence selected from any one of SEQ ID NO: 4 and SEQ ID NO: 16; or

(ii) comprises or consists of an amino acid sequence selected from any one of SEQ ID NO: 4 and SEQ ID NO: 16; or

(iii) comprises or consists of an amino acid sequence that differs from an amino acid sequence selected from any one of SEQ ID NO: 4 and SEQ ID NO: 16 by one or more (preferably no more than 10, more preferably no more than 5, 4, 3, 2, 1) of amino acid modifications (preferably amino acid substitutions, more preferably conservative amino acid substitutions), preferably the amino acid modifications do not occurre in the CDR regions.

In some embodiments, the light chain variable region of the present invention

(i) comprises or consists of an amino acid sequence having at least 90%, 91%, 92%, 93%, 94%, 95%, 96%, 97%, 98%, or 99% identity to an amino acid sequence selected from any one of SEQ ID NO: 10 and SEQ ID NO: 21; or

(ii) comprises or consists of an amino acid sequence selected from any one of SEQ ID NO: 10 and SEQ ID NO: 21; or

(iii) comprises or consists of an amino acid sequence that differs from an amino acid sequence selected from any one of SEQ ID NO: 10 and SEQ ID NO: 21 by one or more (preferably no more than 10, more preferably no more than 5, 4, 3, 2, 1) of amino acid modifications (preferably amino acid substitutions, more preferably conservative amino acid substitutions), preferably the amino acid modifications do not occurre in the CDR regions.

In some embodiments, the three complementarity-determining regions (HCDRs), HCDR1, HCDR2, and HCDR3 from the heavy chain variable region of the present invention are selected from

(i) the three complementarity-determining regions HCDR1, HCDR2, and HCDR3 comprised in the VH set forth in SEQ ID NO: 4 or 16;

(ii) sequences that, compared to any one sequence of (i), contain at least 1 and no more than 5, 4, 3, 2, or 1 amino acid modification(s) (preferably amino acid substitutions, preferably conservative substitutions) in total in the three HCDRs,

for example, wherein the HCDRs may be determined respectively according to any scheme for defining CDRs, for example, according to the Kabat, AbM, Chothia, Contact, or IMGT scheme, or combination thereof;

for example, the HCDR1 may be defined according to a combination of the Kabat and Chothia schemes, and HCDR2 and HCDR3 may be defined according to the Kabat scheme.

In some embodiments, the three complementarity-determining regions (LCDRs), LCDR1, LCDR2, and LCDR3 from the light chain variable region of the present invention are selected from

(i) the three complementarity-determining regions LCDR1, LCDR2, and LCDR3 comprised in the VL set forth in SEQ ID NO: 10 or 21, or

(ii) sequences that, compared to any one sequence of (i), contain at least 1 and no more than 5, 4, 3, 2, or 1 amino acid modification(s) (preferably amino acid substitutions, preferably conservative substitutions) in total in the three LCDRs,

for example, wherein the LCDRs may be determined respectively according to any scheme for defining CDRs, for example, according to the Kabat, AbM, Chothia, Contact, or IMGT scheme, or combination thereof;

for example, the LCDR1-3 may be defined according to the Kabat scheme.

In some embodiments, the HCDR1 comprises or consists of the amino acid sequence set forth in SEQ ID NO: 1 or SEQ ID NO: 13, or the HCDR1 comprises an amino acid sequence that differs from SEQ ID NO: 1 or SEQ ID NO: 13 by one, two or three modifications (preferably amino acid substitutions, more preferably conservative substitutions).
In some embodiments, the HCDR2 comprises or consists of the amino acid sequence set forth in SEQ ID NO: 2 or SEQ ID NO: 14, or the HCDR2 comprises an amino acid sequence that differs from SEQ ID NO: 2 or SEQ ID NO: 14 by one, two or three modifications (preferably amino acid substitutions, more preferably conservative substitutions).
In some embodiments, the HCDR3 comprises or consists of the amino acid sequence set forth in SEQ ID NO: 3 or SEQ ID NO: 15, or the HCDR3 comprises an amino acid sequence that differs from SEQ ID NO: 3 or SEQ ID NO: 15 by one, two or three modifications (preferably amino acid substitutions, more preferably conservative substitutions).
In some embodiments, the LCDR1 comprises or consists of the amino acid sequence set forth in SEQ ID NO: 7 or SEQ ID NO: 18, or the LCDR1 comprises an amino acid sequence that differs from SEQ ID NO: 7 or SEQ ID NO: 18 by one, two or three modifications (preferably amino acid substitutions, more preferably conservative substitutions).
In some embodiments, the LCDR2 comprises or consists of the amino acid sequence set forth in SEQ ID NO: 8 or SEQ ID NO: 19, or the LCDR2 comprises an amino acid sequence that differs from SEQ ID NO: 8 or SEQ ID NO: 19 by one, two or three modifications (preferably amino acid substitutions, more preferably conservative substitutions).
In some embodiments, the LCDR3 comprises or consists of the amino acid sequence set forth in SEQ ID NO: 9 or SEQ ID NO: 20, or the LCDR3 comprises an amino acid sequence that differs from SEQ ID NO: 9 or SEQ ID NO: 20 by one, two or three modifications (preferably amino acid substitutions, more preferably conservative substitutions).
In some embodiments, the heavy chain constant region of the antibody of the present invention is a heavy chain constant region of IgG1, IgG2, IgG3, or IgG4, preferably the heavy chain constant region of IgG1. In some embodiments, the antibody light chain constant region of the present invention is a lambda or kappa light chain constant region, preferably the kappa light chain constant region.

In some embodiments, the heavy chain constant region of the antibody of the present invention comprises a mutated Fc region.

Accordingly, the present invention also relates to a mutated Fc region.

In some embodiments, the Fc region is derived from a human IgG Fc, for example, from a human IgG1 Fc, a human IgG2 Fc, a human IgG3 Fc, or a human IgG4 Fc. In an embodiment, the Fc region comprises or consists of the amino acid sequence set forth in SEQ ID NO: 23 or 24, or an amino acid sequence having at least 90%, for example, 95%, 96%, 97%, 99% or higher identity thereto.

In an embodiment, the Fc region is modified to alter effector function properties (for example, the complement activation function of the Fc region). In an embodiment, the effector function is reduced or eliminated compared to Fc regions of wild-type isotypes. In an embodiment, the effector function is reduced or eliminated by a method selected from the following: using an Fc isotype with a naturally reduced or eliminated effector function, and Fc region modification.

In one preferred embodiment, the Fc region has reduced Fc region-mediated effector functions, such as reduced or eliminated ADCC, ADCP, or CDC effector function, for example, comprises mutation(s) that achieve these effects.

The Fc region may comprise modification(s) that alter the binding affinity for one or more of Fc receptors. In an embodiment, the Fc receptor is a Fcγ receptor, particularly a human Fcγ receptor. In some embodiments, the Fc region comprises mutation(s) that reduce binding to Fcγ receptors. For example, in some embodiments, the Fc region for the present invention comprises L234A/L235A mutations that reduce binding to Fcγ receptors. In a further preferred embodiment, the Fc fragment may comprise mutation(s) that cause an increase in the serum half-life, for example, mutation(s) that improve a binding of the Fc fragment to FcRn.

In a preferred embodiment, the Fc region of the present invention has reduced binding to FcγRs (preferably with binding to all FcγRs completely eliminated) while still retaining an affinity for FcRn. Thus, the Fc region for the present invention comprises L234A/L235A and P329 deletion mutations.

Therefore, in a preferred embodiment, the Fc region of the present invention comprises the amino acid sequence set forth in SEQ ID NO: 24, or comprises an amino acid sequence having at least 85%, 90%, 95%, 96%, 97%, 98%, or 99% identity to the sequence set forth in SEQ ID NO: 24 and comprises L234A/L235A and P329 deletion mutations.

In some preferred embodiments, the heavy chain constant region of the antibody of the present invention

(i) comprises or consists of an amino acid sequence having at least 85%, 90%, 91%, 92%, 93%, 94%, 95%, 96%, 97%, 98%, or 99% identity to an amino acid sequence selected from SEQ ID NO: 5 or 26;

(ii) comprises or consists of an amino acid sequence selected from SEQ ID NO: 5 or 26; or

(iii) comprises or consists of an amino acid sequence that differs from an amino acid sequence selected from SEQ ID NO: 5 or 26 by one or more (preferably no more than 20 or 10, more preferably no more than 5, 4, 3, 2, 1) of amino acid modifications (preferably amino acid substitutions, more preferably conservative amino acid substitutions).

In some preferred embodiments, the heavy chain constant region of the antibody of the present invention comprises an amino acid sequence having at least 85%, 90%, 91%, 92%, 93%, 94%, 95%, 96%, 97%, 98%, or 99% identity to the amino acid sequence set forth in SEQ ID NO: 5, and comprises the L234A/L235A and P329 deletion mutations. In some preferred embodiments, the antibody heavy chain constant region of the present invention comprises or consists of an amino acid sequence set froth in SEQ ID NO: 5.

In some embodiments, the light chain constant region of the antibody of the present invention

(i) comprises or consists of an amino acid sequence having at least 85%, 90%, 91%, 92%, 93%, 94%, 95%, 96%, 97%, 98%, or 99% identity to an amino acid sequence set forth in SEQ ID NO: 11;

(ii) comprises or consists of an amino acid sequence set forth in SEQ ID NO: 11; or

(iii) comprises or consists of an amino acid sequence that differs from an amino acid sequence set forth in SEQ ID NO: 11 by one or more (preferably no more than 20 or 10, more preferably no more than 5, 4, 3, 2, 1) of amino acid modifications (preferably amino acid substitutions, more preferably conservative amino acid substitutions).

In some specific embodiments, the anti-CD40L antibody of the present invention comprises a first heavy chain complementarity-determining region (HCDR1), a second heavy chain complementarity-determining region (HCDR2), a third heavy chain complementarity-determining region (HCDR3), and a first light chain complementarity-determining region (LCDR1), a second light chain complementarity-determining region (LCDR2), and a third light chain complementarity-determining region (LCDR3), wherein:

(i) the HCDR1, HCDR2, HCDR3 and LCDR1, LCDR2, and LCDR3 comprise the amino acid sequences set forth in SEQ ID NO: 1, SEQ ID NO: 2, SEQ ID NO: 3, SEQ ID NO: 7, SEQ ID NO: 8 and SEQ ID NO: 9, respectively, or consist of the amino acid sequences set forth in SEQ ID NO: 1, SEQ ID NO: 2, SEQ ID NO: 3, SEQ ID NO: 7, SEQ ID NO: 8 and SEQ ID NO: 9, respectively; or

(ii) the HCDR1, HCDR2, HCDR3 and LCDR1, LCDR2, and LCDR3 comprise the amino acid sequences set forth in SEQ ID NO: 13, SEQ ID NO: 14, SEQ ID NO: 15, SEQ ID NO: 18, SEQ ID NO: 19 and SEQ ID NO: 20, respectively, or consist of the amino acid sequences set forth in SEQ ID NO: 13, SEQ ID NO: 14, SEQ ID NO: 15, SEQ ID NO: 18, SEQ ID NO: 19 and SEQ ID NO: 20, respectively.

In some embodiments, the VH of the present invention comprises HCDR1, HCDR2 and HCDR3, and the VL comprises LCDR1, LCDR2 and LCDR3, wherein the HCDR1, HCDR2, and HCDR3 comprise the amino acid sequences set forth in SEQ ID NO: 1, SEQ ID NO: 2 and SEQ ID NO: 3, respectively, or consist of the amino acid sequences set forth in SEQ ID NO: 1, SEQ ID NO: 2 and SEQ ID NO: 3, respectively, and the LCDR1, LCDR2 and LCDR3 comprise the amino acid sequences set forth in SEQ ID NO: 7, SEQ ID NO: 8 and SEQ ID NO: 9, respectively, or consist of the amino acid sequences set forth in SEQ ID NO: 7, SEQ ID NO: 8 and SEQ ID NO: 9.

In some embodiments, the VH of the present invention comprises HCDR1, HCDR2, HCDR3, and the VL comprises LCDR1, LCDR2 and LCDR3, wherein the HCDR1, HCDR2 and HCDR3 comprise the amino acid sequences set forth in SEQ ID NO: 13, SEQ ID NO: 14 and SEQ ID NO: 15, respectively, or consist of the amino acid sequences set forth in SEQ ID NO: 13, SEQ ID NO: 14 and SEQ ID NO: 15, respectively, and the LCDR1, LCDR2 and LCDR3 comprise the amino acid sequences set forth in SEQ ID NO: 18, SEQ ID NO: 19 and SEQ ID NO: 20, respectively, or consist of the amino acid sequences set forth in SEQ ID NO: 18, SEQ ID NO: 19 and SEQ ID NO: 20.

In some embodiments, the antibody or the antigen-binding fragment thereof of the present invention:

(i) comprises a VH comprising or consisting of the amino acid sequence set forth in SEQ ID NO: 4, or an amino acid sequence having at least 90%, 91%, 92%, 93%, 94%, 95%, 96%, 97%, 98%, or 99% identity thereto, and/or comprises a VL comprising or consisting of the amino acid sequence set forth in SEQ ID NO: 10, or an amino acid sequence having at least 90%, 91%, 92%, 93%, 94%, 95%, 96%, 97%, 98%, or 99% identity thereto;

(ii) comprises a VH comprising or consisting of the amino acid sequence set forth in SEQ ID NO: 16, or an amino acid sequence having at least 90%, 91%, 92%, 93%, 94%, 95%, 96%, 97%, 98%, or 99% identity thereto, and/or comprises a VL comprising or consisting of the amino acid sequence set forth in SEQ ID NO: 21, or an amino acid sequence having at least 90%, 91%, 92%, 93%, 94%, 95%, 96%, 97%, 98%, or 99% identity thereto.

In some embodiments, the antibody or the antigen-binding fragment thereof of the present invention comprises a heavy chain variable region and a light chain variable region, wherein

(i) the heavy chain variable region comprises or consists of the amino acid sequence set forth in SEQ ID NO: 4, and the light chain variable region comprises or consists of the amino acid sequence set forth in SEQ ID NO: 10; or

(ii) the heavy chain variable region comprises or consists of the amino acid sequence set forth in SEQ ID NO: 16, and the light chain variable region comprises or consists of the amino acid sequence set forth in SEQ ID NO: 21.

In some embodiments, the anti-CD40L antibody or the antigen-binding fragment thereof of the present invention further comprises an antibody heavy chain. In some embodiments, the anti-CD40L antibody or the antigen-binding fragment thereof of the present invention further comprises an antibody light chain. In some embodiments, the anti-CD40L antibody or the antigen-binding fragment thereof of the present invention further comprises a heavy chain and a light chain. In some embodiments, the antibody heavy chain of the present invention comprises the heavy chain variable region and the heavy chain constant region, or consists of the heavy chain variable region and the heavy chain constant region. In some embodiments, the antibody light chain of the present invention comprises the light chain variable region and the light chain constant region, or consists of the light chain variable region and the light chain constant region. In some embodiments, the antibody of the present invention comprises the two heavy chains and the two light chains, or consists of the two heavy chains and the two light chains.

In some embodiments, the antibody or the antigen-binding fragment thereof of the present invention comprises the heavy chain, wherein the heavy chain

(i) comprises or consists of an amino acid sequence having at least 85%, 90%, 91%, 92%, 93%, 94%, 95%, 96%, 97%, 98%, or 99% identity to an amino acid sequence selected from SEQ ID NO: 6 or 17;

(ii) comprises or consists of an amino acid sequence selected from SEQ ID NO: 6 or 17; or

(iii) comprises or consists of an amino acid sequence that differs from an amino acid sequence selected from SEQ ID NO: 6 or 17 by one or more (preferably no more than 20 or 10, more preferably no more than 5, 4, 3, 2, 1) of amino acid modifications (preferably amino acid substitutions, more preferably conservative amino acid substitutions).

In some embodiments, the antibody or the antigen-binding fragment thereof of the present invention comprises the light chain, wherein the light chain

(i) comprises or consists of an amino acid sequence having at least 85%, 90%, 91%, 92%, 93%, 94%, 95%, 96%, 97%, 98%, or 99% identity to an amino acid sequence selected from SEQ ID NO: 12 or 22;

(ii) comprises or consists of an amino acid sequence selected from SEQ ID NO: 12 or 22; or

(iii) comprises or consists of an amino acid sequence that differs from an amino acid sequence selected from SEQ ID NO: 12 or 22 by one or more (preferably no more than 20 or 10, more preferably no more than 5, 4, 3, 2, 1) of amino acid modifications (preferably amino acid substitutions, more preferably conservative amino acid substitutions).

In some embodiments, the antibody or the antigen-binding fragment thereof of the present invention comprises the heavy chain and the light chain, wherein

(i) the heavy chain comprises or consists of an amino acid sequence having at least 85%, 90%, 91%, 92%, 93%, 94%, 95%, 96%, 97%, 98% or 99% identity to the amino acid sequence set forth in SEQ ID NO: 6; and/or the light chain comprises or consists of an amino acid sequence having at least 85%, 90%, 91%, 92%, 93%, 94%, 95%, 96%, 97%, 98% or 99% identity to the amino acid sequence set forth in SEQ ID NO: 12;

(ii) the heavy chain comprises or consists of an amino acid sequence having at least 85%, 90%, 91%, 92%, 93%, 94%, 95%, 96%, 97%, 98% or 99% identity to the amino acid sequence set forth in SEQ ID NO: 17; and/or the light chain comprises or consists of an amino acid sequence having at least 85%, 90%, 91%, 92%, 93%, 94%, 95%, 96%, 97%, 98% or 99% identity to the amino acid sequence o set forth in SEQ ID NO: 22.

In some embodiments, the antibody or the antigen-binding fragment thereof of the present invention comprises the heavy chain and the light chain, wherein

(i) the heavy chain comprises or consists of the amino acid sequence set forth in SEQ ID NO: 6, and the light chain comprises or consists of the amino acid sequence set forth in SEQ ID NO: 12; or

(ii) the heavy chain comprises or consists of the amino acid sequence set forth in SEQ ID NO: 17, and the light chain comprises or consists of the amino acid sequence set forth in SEQ ID NO: 22.

In an embodiment of the present invention, the amino acid modifications described herein include amino acid substitutions, insertions or deletions. Preferably, the amino acid modifications described herein are amino acid substitutions, more preferably conservative substitutions.

In a preferred embodiment, the amino acid modifications of the present invention occur in regions outside the CDRs (e.g., in a FR). More preferably, the amino acid modifications of the present invention occur in regions outside the heavy chain variable region and/or outside the light chain variable region.

In certain embodiments, the antibody provided herein is modified to increase or decrease the extent of glycosylation of the antibody. The addition or deletion of glycosylation site(s) in the antibody can be readily achieved by altering the amino acid sequence to create or remove one or more of glycosylation sites. When the antibody comprises an Fc region, the glycans attached thereto can be modified. In certain applications, modifications that remove undesired glycosylation sites can be useful, for example, the removal of fucose motif to enhance antibody-dependent cellular cytotoxicity (ADCC) function. In other applications, galactosylation modifications can be performed to modulate complement-dependent cytotoxicity (CDC).

In certain embodiments, it may be desirable to produce antibodies engineered with cysteine, such as "thioMAbs", in which one or more of residues of the antibody are substituted with cysteine residues.

In certain embodiments, the antibody provided herein can be further modified to contain other non-proteinaceous moieties that are known in the art and readily obtainable. Suitable moieties for antibody derivatization include, but are not limited to, water-soluble polymers. Non-limiting examples of water-soluble polymers include, but are not limited to, polyethylene glycol (PEG), ethylene glycol/propylene glycol copolymer, carboxymethyl cellulose, dextran, polyvinyl alcohol, polyvinyl pyrrolidone, poly-1,3-dioxane, poly-1,3,6-trioxane, ethylene/maleic anhydride copolymer, polyamino acid (homopolymer or random copolymer), and dextran or poly(n-vinyl pyrrolidone) polyethylene glycol, propylene glycol homopolymer, polypropylene oxide/ethylene oxide copolymer, polyoxyethylated polyols (such as glycerol), polyvinyl alcohol, and mixtures thereof.

In some embodiments, the anti-CD40L antibody or the antigen-binding fragment thereof of the present invention have one or more of the following characteristics:

(i) exhibiting the same or similar binding affinity and/or specificity to CD40L as the antibody of the present invention;

(ii) inhibiting (e.g., competitively inhibiting) the binding of the antibody of the present invention to CD40L;

(iii) binding to the same or overlapping epitope as the antibody of the present invention;

(iv) competing with the antibody of the present invention for binding to CD40L;

(v) possessing one or more of biological characteristics of the antibody of the present invention.

In some embodiments, the anti-CD40L antibody of the present invention is in the form of an IgG1 antibody, IgG2 antibody, IgG3 antibody, or IgG4 antibody, preferably in the form of an IgG1 antibody.

In some embodiments, the anti-CD40L antibody is a monoclonal antibody.

In some embodiments, the anti-CD40L antibody is humanized.

In some embodiments, the anti-CD40L antibody is a chimeric antibody.

In an embodiment, the anti-CD40L antibody of the present invention also encompasses an antibody fragment thereof (e.g., an antigen-binding fragment), preferably antibody fragment selected from the following: Fv, Fab, Fab', Fab'-SH, F(ab')$_2$, dAb (domain antibody), a linear antibody, a single-chain antibody (e.g., scFv), a single-domain antibody (sdAb) such as VHH, a bivalent antibody or fragment thereof, or a camelid antibody or diabody.

In some embodiments, the anti-CD40L antibody of the present invention further encompasses a multi-specific antibody that specifically bind to CD40L, such as a bispecific antibody.

In some embodiments, the anti-CD40L antibody of the present invention is a full-length antibody.

## III. Nucleic Acids of the Present Invention and Host Cells Comprising the Same

In one aspect, the present invention provides a nucleic acid encoding any chain, monomer, or domain of the antibody or an antigen-binding fragment thereof of the present invention. Polynucleotide sequences encoding the various chains can be generated using methods well known in the art. For example, when expressed from a suitable expression vector, the polypeptide encoded by the nucleic acid is capable of exhibiting binding to human CD40L antigen. For example, in some embodiments, the nucleic acid encoding the variable region of the heavy chain and/or light chain is operably linked in a reading frame to the nucleic acid encoding the constant region of the heavy chain and/or light chain, such that, when expressed from a suitable expression vector, a nucleic acid encoding the heavy chain and/or light chain of the antibody is produced.

In one aspect, the present invention provides a nucleic acid encoding any anti-CD40L antibody or fragment thereof described herein. The nucleic acid may comprise a nucleic acid encoding the amino acid sequence of the light chain variable region and/or heavy chain variable region of the antibody, or comprise a nucleic acid encoding the amino acid sequence of the light chain and/or heavy chain of the antibody.

For example, the nucleic acid of the present invention comprises a nucleic acid encoding an amino acid sequence selected from any one set forth in SEQ ID NO: 4-6, 10, 12, 16, 17, 21, and 22, or a nucleic acid encoding an amino acid sequence having at least 85%, 90%, 91%, 92%, 93%, 94%, 95%, 96%, 97%, 98%, or 99% identity to an amino acid sequence selected from any one set forth in SEQ ID NO: 4-6, 10, 12, 16, 17, 21, and 22. As will be appreciated by those skilled in the art, each antibody or polypeptide amino acid sequence may be encoded by various nucleic acid sequences due to codon degeneracy. In some embodiments, the nucleic acid of the present invention comprises a nucleic acid selected from any one set forth in SEQ ID NO: 28-31, or comprises a nucleic acid having at least 85%, 90%, 91%, 92%, 93%, 94%, 95%, 96%, 97%, 98%, or 99% identity to a nucleic acid selected from any one set forth in SEQ ID NO: 28-31.

The nucleic acid sequence encoding the molecule of the present invention may be generated using methods well known in the art, for example, by de novo solid-phase DNA synthesis or by PCR amplification.

For ease of production and purification, a secretory signal peptide and/or a peptide tag that facilitates purification may be fused to the N-terminus of the heavy chain and/or light chain of an antibody.

The present invention also provides a vector comprising the nucleic acid of the present invention. In an embodiment, the vector is an expression vector, for example, a eukaryotic expression vector. The vector includes but is not limited to viruses, plasmids, cosmids, lambda phages and yeast artificial chromosomes (YACs). In a preferred embodiment, the expression vector of the present invention is a pcDNA vector, for example, a pcDNA3.1 expression vector.

In an embodiment, a host cell comprising the expression vector is provided. The present invention also provides a host cell comprising the nucleic acid or the vector. Host cells suitable for replication and supporting expression of the antibody of the present invention are well known in the art. Such cells can be transfected or transduced using a particular expression vector, and large numbers of vector-containing cells can be cultured for inoculation of large-scale fermentation vessels, thereby producing sufficient amounts of antibodies for clinical use. Appropriate host cells for cloning or expressing vectors encoding the antibody include prokaryotic cells or eukaryotic cells as described herein. For example, the antibody can be produced in bacteria, particularly when glycosylation and Fc effector functions are not required. Following expression, the antibody can be isolated from the bacterial cell paste in the soluble fraction and can be further purified.

In an embodiment, the host cell is eukaryotic. In another embodiment, the host cell is selected from a yeast cell, a mammalian cell (e.g., a CHO cell such as CHO-K1, CHO-S, CHO-K, or an ExpiCHO cell, or a 293 cell such as a 293F or HEK293 cell), or another cell suitable for producing the antibody or fragment thereof. In an embodiment, the host cell is prokaryotic, for example, a bacterium such as Escherichia coli.

For example, eukaryotic microorganisms such as filamentous fungi or yeast are suitable host cells for cloning or expressing vectors encoding the antibody. For example, fungal and yeast strains in which the glycosylation pathway has been "humanized" can produce antibodies with partially or fully human glycosylation patterns. Host cells suitable for expressing glycosylated antibodies can also be derived from multicellular organisms (invertebrates and vertebrates). Vertebrate cells can also be used as hosts. For example, mammalian cell lines engineered for growth in suspension can be used. Other examples of useful mammalian host cell lines include SV40-transformed monkey kidney CV1 lines (COS-7), human embryonic kidney lines (HEK293, 293F, or 293T cells), and the like. Other useful mammalian host cell lines include Chinese hamster ovary (CHO) cells, including CHO-K1, CHO-K, DHFR-CHO cells, CHO-S cells, ExpiCHO, and the like; as well as myeloma cell lines such as Y0, NS0, and Sp2/0. Mammalian host cell lines suitable for antibody production are known in the art.

## IV. Production and Purification of the Antibody Molecule of the Present Invention

In an embodiment, the present invention provides a method for preparing the antibody molecule of the present invention or a fragment thereof (preferably an antigen-binding fragment), wherein the method comprises culturing a host cell under conditions suitable for expressing a nucleic acid encoding the antibody molecule of the present invention or a fragment thereof (preferably an antigen-binding fragment), and optionally isolating the antibody or fragment thereof (e.g., an antigen-binding fragment). In an embodiment, the method further comprises recovering the antibody molecule of the present invention or a fragment thereof (e.g., an antigen-binding fragment) from the host cell.

A polynucleotide encoding a polypeptide chain of the antibody of the present invention may be inserted into one or more vectors for subsequent cloning and/or expression in a host cell. Methods well known to those skilled in the art may be used to construct an expression vector. Once the expression vector comprising one or more nucleic acid molecules of the present invention for expression has been prepared, the expression vector may be transfected into or introduced into a suitable host cell. Various techniques can be used to achieve this purpose, for example, protoplast fusion, calcium phosphate precipitation, electroporation, retroviral transduction, viral transfection, biolistics, liposome-based transfection, or other conventional techniques.

The antibody molecule prepared as described herein may be purified by known exiting techniques, such as high-performance liquid chromatography, ion exchange chromatography, gel electrophoresis, affinity chromatography, size-exclusion chromatography, and the like. The actual conditions used to purify a particular protein also depend on factors such as net charge, hydrophobicity, hydrophilicity, and the like, and these are apparent to those skilled in the art. The purity of the antibody molecule of the present invention may be determined by any one of various well-known analytical methods, and the well-known analytical methods include size-exclusion chromatography, gel electrophoresis, high-performance liquid chromatography, and the like.

## V. Assays

The physical/chemical properties and/or biological activity of the CD40L antibody provided herein may be identified, screened, or characterized by various assays known in the art.

The present invention also provides an assay for identifying an anti-CD40L antibody having biological activity. Biological activity may include, for example, binding to CD40L (e.g., binding to human CD40L), inhibiting the CD40/CD40L signaling pathway, blocking the binding of CD40 to CD40L, inhibiting activation of B cells, inhibiting proliferation of B cells, inhibiting differentiation of B cells, inhibiting cytokine secretion by dendritic cells, inhibiting antibody production in a subject, and therapeutic effects on immune system diseases. Antibodies having such biological activity in vivo and/or in vitro are also provided.

For the assay of the aforementioned biological activities, reference may be made to the exemplary assay methods provided in the Examples.

It may be understood that an immunoconjugate of the present invention may be used to replace or supplement an anti-CD40L antibody in any of the aforementioned assays.

It may be understood that a combination of an anti-CD40L antibody and other agents may be used in any of the aforementioned assays.

## VI. Immunoconjugates

In some embodiments, the present invention provides an immunoconjugate comprising any of the anti-CD40L antibodies provided herein and other substance, such as a therapeutic agent or a label. In some embodiments, the therapeutic agent may be a therapeutic agent suitable for forming an immunoconjugate with a CD40L antibody, for example, an agent or active component that can produce a biological effect. Such agents that can produce a biological effect include, but are not limited to, cytokines, other antibodies, small-molecule drugs, or immunomodulators (e.g., immunosuppressants).

In some embodiments, the immunoconjugate is an antibody drug conjugate (ADC).

## VII. Pharmaceutical Compositions and Pharmaceutical Formulations

In some embodiments, the present invention provides a composition comprising any of the anti-CD40L antibody as described herein or a fragment thereof (preferably an antigen-binding fragment) or an immunoconjugate thereof, wherein the composition is preferably a pharmaceutical composition. In an embodiment, the composition further comprises pharmaceutically acceptable auxiliary materials. In an embodiment, a composition, for example, a pharmaceutical composition, comprises a combination of the anti-CD40L antibody of the present invention or a fragment thereof or an immunoconjugate thereof, and one or more other therapeutic agents.

The present invention also encompasses a composition comprising the anti-CD40L antibody or immunoconjugate thereof (including a pharmaceutical composition or pharmaceutical formulation), or a composition comprising a polynucleotide encoding the anti-CD40L antibody (including a pharmaceutical composition or pharmaceutical formulation). In certain embodiments, the composition comprises one or more antibodies binding to CD40L or fragments thereof, or one or more polynucleotides encoding one or more anti-CD40L antibodies or fragments thereof. These compositions may also comprise a suitable pharmaceutically acceptable auxiliary materials, such as known pharmaceutically acceptable carriers and pharmaceutically acceptable auxiliary materials in the art, including buffers.

As used herein, "pharmaceutically acceptable carriers" comprises any or all physiologically compatible solvents, dispersing media, isotonic agents, absorption-delaying agents, and the like.

Refer to "Handbook of Pharmaceutical Excipients", Version 8, R.C.Rowe, P.J.Seskey and S.C. Owen, Pharmaceutical Press, London, Chicago for the usage and uses of pharmaceutically acceptable auxiliary materials.

The composition of the present invention may be in a plurality of forms. Such forms, for example, include liquid, semi-solid, and solid dosage forms, such as liquid solutions (for example, injectable solutions and infusible solutions), powders or suspensions, liposomal agents and suppositories. The preferred form depends on the expected mode of administration and therapeutic use.

A pharmaceutical formulation comprising the antibody of the present invention may be prepared by mixing the antibody of the present invention with the desired purity with one or more optional pharmaceutically acceptable auxiliary materials, preferably in the form of a lyophilized formulation or an aqueous solution.

The pharmaceutical composition or formulation of the present invention may further comprise more than one active component that is required for the treatment of a particular indication, preferably those active components having complementary activities that do not adversely affect each other. For example, it is desirable to further provide other therapeutic agents, such as agents that can produce a biological effect in the prevention or treatment of diseases associated with inappropriate activation of CD40L/CD40-mediated pathways, including, but not limited to, cytokines, small-molecule drugs, immunomodulators (e.g., immunosuppressants), or other antibodies. The active components are present in effective amounts, suitably combined to achieve the intended purpose. A sustained-release formulation may be prepared. Suitable examples of sustained-release formulations comprise a semipermeable matrix of a solid hydrophobic polymer containing the antibody, wherein the matrix is in the form of a shaped article, such as a film or a microsphere.

## VIII. Pharmaceutical Combinations and Kits

In some embodiments, the present invention further provides a pharmaceutical combination or pharmaceutical combination product comprising the anti-CD40L antibody of the present invention or a fragment thereof (preferably an antigen-binding fragment), or an immunoconjugate thereof, and one or more other therapeutic agents (such as cytokines, small-molecule drugs, immunomodulators (e.g., immunosuppressants or anti-inflammatory agents), or other antibodies).

Another objective of the present invention is to provide a kit of parts comprising the pharmaceutical combination of the present invention, preferably, the kit is in form of drug dosage units. As such, a dosage unit may be provided based on the dosing regimen or dosing interval of the drug administration.

In an embodiment, the kit of parts of the present invention comprises, in the same package:

- a first container containing the pharmaceutical composition comprising the anti-CD40L antibody or fragment thereof;

- a second container containing the pharmaceutical composition comprising other therapeutic agents.

In some embodiments, the other therapeutic agents are for example agents that can produce a biological effect in the prevention or treatment of diseases associated with inappropriate activation of CD40L/CD40-mediated pathways, including, but not limited to, cytokines, small-molecule drugs, immunomodulators (e.g., immunosuppressants), or other antibodies.

## IX. Uses and Methods

In some embodiments, a method for preventing or treating a disease or disorder associated with an abnormal activation of a CD40L/CD40-mediated signaling pathway in an individual comprises administering the antibody of the present invention as a monotherapy or in combination.

In some embodiments, the disease or disorder is that of the immune system. In some embodiments, a patient having the disease or disorder exhibits an overactive immune system mediated by CD40L/CD40 compared to a healthy individual. In some embodiments, a patient having the disease or disorder exhibits an abnormal activation of a CD40L/CD40-mediated signaling pathway compared to a healthy individual. In some embodiments, a patient having the disease or disorder produces or deposits excessive autoantibodies compared to a healthy individual. In some embodiments, a patient having the disease or disorder exhibits an abnormal activation of a CD40L/CD40-mediated signaling pathway and excessive production and deposition of autoantibodies compared to a healthy individual.

In some embodiments, cells in a patient having the disease or disorder (e.g., activated T cells or other types of immune cells or non-immune cells, such as epithelial cells, monocytes, dendritic cells, fibroblasts, smooth muscle cells, endothelial cells, and platelets) exhibit increased expression of CD40L or CD40, for example, compared to the corresponding cells of a healthy individual.

In some embodiments, the disease or disorder includes, but is not limited to, an autoimmune disease, lupus nephritis, immune thrombocytopenic purpura (ITP), transplant rejection, Crohn's disease, inflammatory bowel disease (IBD), colitis, asthma/allergy, atherosclerosis, myasthenia gravis, multiple sclerosis, psoriasis, rheumatoid arthritis, ankylosing spondylitis, coronary heart disease, type 1 diabetes, amyotrophic lateral sclerosis (ALS), and immune responses to recombinant drug products, such as Factor VII for hemophilia.

In some embodiments, the antibody or antibody fragment or immunoconjugate or composition or product of the present invention delays the onset of the disorder and/or symptoms associated with the disorder.

In some embodiments, the present invention provides the use of the anti-CD40L antibody of the present invention or a fragment thereof or the immunoconjugate or composition comprising the same in the manufacture or production of a medicament, which is for the uses described herein, for example, for preventing or treating the diseases or disorders mentioned herein.

In some embodiments, the prevention or treatment method described herein further comprises administering to the subject or individual the antibody molecule or pharmaceutical composition or immunoconjugate disclosed herein in combination, as well as one or more other therapies, such as treatment modalities and/or other therapeutic agents. In some embodiments, the anti-CD40L antibody or fragment thereof (as well as the immunoconjugate, composition, pharmaceutical composition, or formulation, or the like comprising the same) may be administered in combination with one or more other therapies, such as treatment modalities and/or other therapeutic agents, for the uses described herein, for example, for preventing and/or treating the diseases or disorders mentioned herein.

In some embodiments, the therapeutic agents are selected from cytokines, small-molecule drugs, immunomodulators (e.g., immunosuppressants or anti-inflammatory agents), or other antibodies.

In some embodiments, the antibody described herein may be combined with other antibodies for separate administration, for example, separately as separate antibodies, or administered when linked (e.g., as a bispecific or multispecific antibody molecule).

The combination therapy of the present disclosure encompasses administration in combination (e.g., two or more therapeutic agents contained in the same formulation or in separate formulations) and separate administration, in which case the administration of the antibody of the present invention or the fragment thereof may occur before, concurrently with, and/or after the administration of the other therapeutic agents and/or medicaments. In some embodiments, the other therapeutic agents are agents that can produce a biological effect in the prevention or treatment of diseases associated with inappropriate activation of CD40L/CD40-mediated pathways, including, but not limited to, cytokines, small-molecule drugs, immunomodulators (e.g., immunosuppressants), or other antibodies, or the like.

The antibody of the present invention or the fragment thereof (as well as the immunoconjugate, composition, pharmaceutical composition, formulation, combination product, or the like comprising the same) may be administered by any suitable method, including parenteral administration, and, if desired for local treatment, intralesional administration. Parenteral injection or infusion includes intramuscular, intravenous, intraarterial, intraperitoneal, or subcutaneous injection or infusion.

**X. Methods and Compositions for Diagnosis and Detection**

In one aspect, the present invention further relates to a method for diagnosis and detection using the antibody of the present invention or an antigen-binding fragment thereof, and a composition comprising the same for diagnosis and detection.

In certain embodiments, any anti-CD40L antibody or fragment thereof (preferably an antigen-binding fragment) provided herein can be used to detect the presence of CD40L in a biological sample.

As used herein, the term "detection" includes quantitative or qualitative detection. Exemplary detection methods can involve immunohistochemistry, immunocytochemistry, flow cytometry (e.g., FACS), antibody molecule-conjugated magnetic beads, ELISA, and PCR-based techniques (e.g., RT-PCR). In certain embodiments, the biological sample is blood, serum, or other liquid samples of biological origin. In certain embodiments, the biological sample includes cells or tissues.

In an embodiment, an anti-CD40L antibody or a fragment thereof is provided for use in a diagnostic or detection method.

In another aspect, a method is provided for detecting the presence of CD40L in a biological sample. In certain embodiments, the method comprises detecting the presence of CD40L protein in a biological sample. In certain embodiments, the CD40L is human CD40L. In certain embodiments, the method comprises contacting a biological sample with the anti-CD40L antibody or fragment thereof as described herein under conditions that allow the anti-CD40L antibody or fragment thereof to bind to CD40L, and detecting whether a complex is formed between the anti-CD40L antibody or fragment thereof and CD40L. Formation of the complex indicates the presence of CD40L. The method may be an in vitro or in vivo method. In an embodiment, an anti-CD40L antibody or a fragment thereof is used to select subjects suitable for treatment using the anti-CD40L antibody or fragment thereof, for example, wherein CD40L serves as a biomarker for selecting the subjects.

In some embodiments, a labeled anti-CD40L antibody or a fragment thereof is provided. The label includes, but is not limited to, directly detectable labels or moieties (such as fluorescent labels, chromophore labels, electron-dense labels, chemiluminescent labels, and radioactive labels), as well as indirectly detectable moieties, such as enzymes or ligands, for example, via enzymatic reactions or molecular interactions.

In some embodiments provided herein, the sample is obtained prior to treatment with an anti-CD40L antibody or a fragment thereof. In some embodiments, the sample is obtained prior to treatment with other therapies. In some embodiments, the sample is obtained during treatment with other therapies, or after treatment with other therapies.

In some embodiments, CD40L is detected prior to treatment, for example, before initial treatment or prior to a particular treatment following a treatment interval.

In some embodiments, a method of treating a disease of the present invention is provided, wherein the method comprises: detecting the presence of CD40L in a subject (e.g., a sample) (e.g., a subject sample), thereby determining a CD40L value, comparing the CD40L value to a control value, and, if the CD40L value is greater than the control value, administering to the subject a therapeutically effective amount of an anti-CD40L antibody or a fragment thereof (e.g., the anti-CD40L antibody or fragment thereof described herein), optionally in combination with one or more other therapies, thereby treating the disease.

These and other aspects and embodiments of the present invention are described in the drawings (brief description of the drawings follows) and the detailed description of the invention below, and are exemplified in the following examples. Any or all of the features discussed above, as well as throughout the present application, may be combined in various embodiments of the present invention. The following examples further illustrate the present invention; however, it should be understood that the examples are described for illustrative purposes only and not by way of limitation, and that various modifications may be made by those skilled in the art.

**Examples**

Example 1: Screening and humanization of anti-CD40L antibodies

Using hybridoma technology, mice were immunized with human CD40L (Human CD40 Ligand / TNFSF5 Protein, His, Flag Tag, active trimer (MALS verified), Acro) fusion protein. After immunization, the mice were sacrificed, the spleens were harvested and digested into single cell suspensions, and the splenocytes were hybridized with proliferative myeloma cells. Under the action of selective medium, hybridoma cells capable of expressing positive antibodies were obtained. Murine antibodies capable of blocking the CD40-CD40L interaction were further screened using in vitro ELISA and flow cytometry.

Preparation of electroporation plates

The electroporation plates were thoroughly soaked in 70% ethanol and allowed to air-dry in a laminar flow hood for later use.

Isolation of splenocytes: The mice were euthanized by cervical dislocation, and the body surface was disinfected with 75% alcohol for 5 minutes. The mice were then placed on a dissection board in a laminar flow hood in the left lateral position, with the limbs secured using a No. 7 needle. The abdominal cavity was aseptically opened to take the spleen, which was washed with 1640 medium (containing 10% fetal bovine serum and 1% GlutaMAX), and any surrounding attached connective tissue was carefully removed. The spleen was then transferred to another plate containing basal culture medium. The spleen was held down with a bent needle, punctured with a small needle, and gently squeezed with forceps to fully release the splenocytes, thereby preparing a splenocyte suspension. The cell suspension was filtered through a 70 $\mu$M cell strainer, washed once with 30 mL of 1640 medium (containing 10% fetal bovine serum and 1% GlutaMAX), and centrifuged at 1,200 rpm for 6 minutes.

Lysis of red blood cells

The supernatant was removed, and the cells were resuspended in 10 mL of RBC lysis buffer (GIBCO). Then, an additional 20 mL of RBC lysis buffer was added. The suspension was left to stand for 5 minutes and then centrifuged at 1,100 rpm for 6 minutes. After removing the supernatant, the cells were resuspended in 10 mL of basal culture medium, followed by the addition of 30 mL of basal culture medium, and centrifuged at 1,100 rpm for 6 minutes. After removing the supernatant, the cells were resuspended in 20 mL of RPMI 1640 medium (containing 10% fetal bovine serum and 1% GlutaMAX) and counted.

Electrofusion

The mouse myeloma SP2/0 cells (ATCC) were resuspended in 20 mL of RPMI 1640 medium (containing 10% fetal bovine serum and 1% GlutaMAX) and counted. The SP2/0 cells and splenocytes (after red blood cell lysis) were mixed at a ratio of 1:2-1:1 and centrifuged at 1,000 rpm for 6 minutes. After the supernatant was removed, the mixed cells were resuspended in 10 mL of fusion buffer (BTXpress). An additional 15 mL of fusion buffer was added, and the mixture was centrifuged at 1,000 rpm for 5 minutes, after which the supernatant was removed. After repeating the above steps once, the cells were resuspended in an appropriate volume of fusion buffer, and the density of the mixed cells was adjusted to $1 \times 10^7$ cells/mL. In each electroporation plate, 2 mL of the above cell suspension was added for electrofusion.

Plating after electrofusion

The cells were left to stand at room temperature for 5 minutes in the electroporation plate. The cells were then transferred into a centrifuge tube and diluted with screening medium (RPMI 1640 containing 10% fetal bovine serum, 1% GlutaMAX, and 1% HAT medium) to a density of $1\text{-}2 \times 10^4$ cells/mL. 100 $\mu$l of the cell suspension was added to each well of a 96-well plate. The screening medium was replaced on Day 7 after fusion. On Day 10 of culture (or longer, depending on cell growth), ELISA and flow cytometry were used to screen for hybridoma cells expressing specific anti-CD40L antibodies.

Subcloning of positive hybridoma cells

A 96-well plate was prepared, and 200 $\mu$L of the aforementioned RPMI 1640 medium (containing 10% fetal bovine serum and 1% GlutaMAX) was added to each well in rows 2 to 8. The cells from the positive wells identified in the above fusion screening were adjusted to a density of approximately $1 \times 10^5$ cells/mL, and 300 $\mu$L of the cell suspension was added to each well in the first row. Using a multichannel pipette, 100 $\mu$L of the cell suspension from the first row was transferred to the second row. After thorough mixing, 100 $\mu$L was taken and transferred to the next row. The above steps were repeated until the volume in the last column reached 300 μL. The 96-well plate was left to stand for 15 minutes, and the cells were observed and counted under a microscope. The volume corresponding to 100 cells was added to 20 mL of the aforementioned RPMI 1640 medium (containing 10% fetal bovine serum and 1% GlutaMAX), mixed, and plated at 200 μL per well. After one week, the wells were observed under a microscope, and the monoclonal wells were identified and marked. Positive wells were selected for further testing.

In vitro screening

Hybridoma cells expressing specific anti-CD40L antibodies were screened using ELISA and flow cytometry.

Hybridoma sequencing

RNA extraction: Approximately $5 \times 10^6$ freshly cultured cells were collected and centrifuged at 300 g for 5 minutes. The supernatant was removed, and 500 μL of LY buffer (Biomiga) was added to the pellet (with 20 μL of β-mercaptoethanol added per 1 mL of buffer before use), followed by mixing until the solution was clear. The mixture was added to a DNA removal column and centrifuged at 13,000 rpm for 2 minutes, and the flow-through was collected. An equal volume of 100% ethanol was added to the flow-through at a 1/2 ratio, and the mixture was inverted 5 times to mix until clear. The clarified solution was added to the RNA collection tube and centrifuged at 13,000 rpm for 1 minute to remove the liquid. Then, 500 μL of RB (Recovery Buffer, Takara) was added and centrifuged at 13,000 rpm for 30 seconds. Next, 500 μL of RNA wash buffer (Biomiga, pre-mixed with an appropriate volume of ethanol before use) was added and centrifuged for 30 seconds. The above step was repeated once. After centrifugation to completely evaporate and remove ethanol, 30 μL of DEPC water was added to the collection column (from the PrimeScript II 1st Strand cDNA Synthesis Kit) and centrifuged at 12,000 g for 2 minutes to collect the eluent. The RNA concentration was determined.

cDNA was obtained by reverse transcription using the PrimeScript II 1st Strand cDNA Synthesis Kit (Takara). The reaction was incubated at 65°C for 5 minutes and then immediately cooled on ice. Reaction system I was added to the reverse transcription system to a total volume of 20 μL and gently mixed, and reverse transcription was performed under the following conditions: 42°C for 60 minutes and 95°C for 5 minutes, and then cooled on ice to obtain cDNA. The cDNA was ligated into a T-vector using the Mighty TA-cloning Kit (Takara): the heavy chain and light chain variable regions were amplified by PCR separately.

Transformation of cells

TOP10 competent cells (Tiangen Biotech (Beijing) Co., Ltd.) were removed from -80°C and thawed on ice. The above-obtained ligation product in 5 μL was added to the thawed TOP10 competent cells, mixed, and incubated on ice for 30 minutes. The cells were heat-shocked at 42°C for 90 seconds and then immediately cooled on ice for 2 minutes. LB medium of 900 μL (Sangon Biotech (Shanghai) Co., Ltd.) was added to the EP tube, and the cells were incubated at 37°C on a 220-rpm shaker for 1 hour. The cells were centrifuged at 3,000 g for 2 minutes, 800 μL of the supernatant was removed, and the pellet was resuspended in the remaining medium and plated onto ampicillin-resistant agar plates. The plates were incubated at 37°C overnight, and clones were picked for sequencing.

Construction of chimeric antibodies

Using molecular biology techniques, the sequences of the anti-CD40L antibodies produced by the above hybridoma cells were obtained. The VH and VL region sequences of the candidate anti-CD40L antibodies obtained by sequencing were combined with the hIgG1 sequence (with mutations at L234A/L235A/ΔP329, SEQ ID NO: 5) to construct human-mouse chimeric antibodies.

Chimeric antibody screening

ELISA and Luciferase in vitro assays were used to screen and obtain anti-CD40L antibodies that both bind CD40L and block the CD40-CD40L interaction.

Humanization of chimeric antibodies

The obtained chimeric antibodies were humanized. Humanization was performed through the following steps:

    ① the CDR loop structures were identified;

(2) for each V/J region of the heavy and light chains, the closest homologous sequences were identified from human germline sequence databases;

③ human germlines that best match the heavy and light chains and require the minimal number of back-mutations were selected;

④ the CDR regions of the chimeric antibody were constructed onto the human framework regions;

⑤ using sequence and structural features, amino acid positions in the framework regions that are important for maintaining CDR function were identified;

⑥ back-mutations were performed at positions determined to be critical (restoring the original amino acid type);

⑦ amino acids at risk positions were optimized.

Finally, humanized antibodies binding human CD40L, Hz33B9C5 and Hz47E2, were obtained. The CDRs, light chain variable regions, heavy chain variable regions, light and heavy chain amino acid sequences, and the corresponding nucleotide sequences of the two humanized antibodies (Hz33B9C5 and Hz47E2) exemplified in the present invention are all listed in the sequence listing.

**Example 2: Binding kinetics of the antibodies of the present invention with antigen measured by ForteBio**

The equilibrium dissociation constant (KD) of the binding of the antibodies of the present invention to human CD40L was measured using bio-layer interferometry (ForteBio).

According to the number of samples, corresponding AMQ (Pall, 1506091) or AHQ (Pall, 1502051) sensors (for positive control detection) were prepared and soaked in SD buffer (PBS 1×, BSA 0.1 %, Tween-20 0.05 %).

SD buffer of 100 $\mu$L, and 200 $\mu$L each of antibody (Hz33B9C5 and Hz47E2, 100 nM) and antigen (human CD40L, purchased from ACROBiosystems, 100 nM) were separately added into a 96-well black polystyrene half-area microplate (Greiner, 675076). The sensor position was selected based on the sample position and layout. The instrument setting parameters are as follows: Run steps: Baseline, Loading ~1 nm, Baseline, Association and Dissociation; the run time of each step depended on the sample binding and dissociation rate, and the rotational speed was 400 rpm and temperature was 30°C. ForteBio analysis software was used to analysis the KD value.

The affinities of Hz33B9C5 and Hz47E2 in the experiments described in the above assay are shown in Table X:

Table 1. ForteBio Detection of Affinity Constant (Equilibrium Dissociation Constant) of Antigen-Antibody Binding

| Antibody | $K_D(M)$ |
|----------|----------|
| Hz33B9C5 | 2.97E-10 |
| Hz47E2 | 3.15E-10 |

**Example 3: Inhibition of the binding of CD40 to CD40L by the antibody of the present invention at the molecular level**

In the present application, the inventors of the present invention screened antibodies with higher affinity for CD40L using mouse hybridoma technology, and humanization was performed to ultimately obtain two humanized antibodies, Hz33B9C5 and Hz47E2. The specific sequences are provided in the sequence listing.

The expression and purification in ExpiCHO cells are as follows:
The sequences of Hz33B9C5 and Hz47E2 were respectively cloned into the pcDNA3.1 vector for subsequent use. ExpiCHO cells were taken, and when the detected cell density reached $8.0\text{-}10 \times 10^6$ viable cells/mL with viability between 95%-99%, the cell density was adjusted to $6.0 \times 10^6$ viable cells/mL. An appropriately sized container was taken, and Fectamine transfection buffer reagent (source: Gibco) was added at 8% of the volume of cells to be transfected; plasmid was added at a concentration of 0.8 $\mu$g/mL of the transfected cells, and the transfection buffer containing the DNA plasmid was filtered through a 0.22 $\mu$m filter for sterilization into another new clean container. FectPro transfection reagent (source: Polyplus) was added to the filtered mixture at a proportion of 3.2 $\mu$L/mL, and the mixture was left to stand for 10 min. The complex formed by the transfection reagent and the plasmid DNA was rapidly added to the cells while gently shaking the flask during addition. Culture was performed on a shaker at 36.5°C with 8% $CO_2$. After 18-22 h of incubation, Enhancer (source: Gibco) was added at 6 $\mu$L/mL cell, and Feed (source: Gibco) was added at 300 $\mu$L/mL cell. On Day 7, the cells were harvested to collect the supernatant. Protein purification was performed via affinity chromatography to obtain the final antibody molecule. The purified antibody solution was centrifuged at 4,000 rpm for 10 min using a 15 mL ultrafiltration centrifuge tube. The protein was diluted with PBS and centrifuged again at 4,000 rpm for 10 min. This process was repeated several times to replace the buffer. The antibody with the replaced buffer was pooled, and the antibody concentration was measured. The components of the monoclonal antibody were further qualitatively analyzed using liquid chromatography-mass spectrometry (LC-MS) and size exclusion chromatography (SEC), and quantitatively analyzed using an ultraviolet-visible spectrophotometer (UV spectrophotometer).

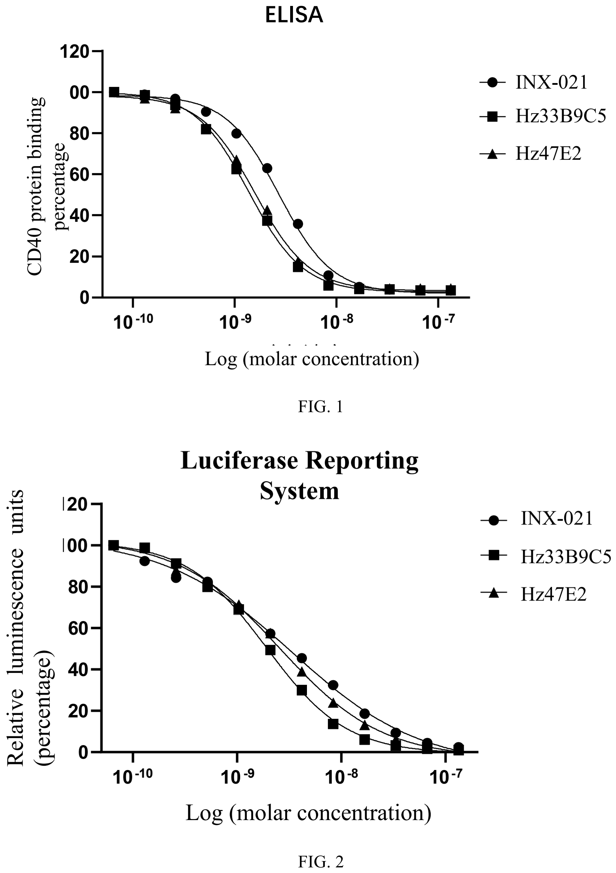

In the present example, its blocking effect on CD40L at the molecular level was tested. INX-021 was used as a control (see the sequences in FIG. 1A and FIG. 1B of WO2017011544A1). The antigen (human CD40 Ligand) source: commercial antigen: Acro Human CD40 Ligand/TNFSF5 Protein, His, Flag Tag (active trimer) (MALS verified).

Equipment: Thermo Fisher MULTISKAN-FC

Reagents and raw materials:

| Name | Manufacturer | Catalog No. |
|---|---|---|
| PBS (1X) | Gibco | 10010-023 |
| BSA | Sangon Biotech | A500023-0100 |
| Tween | Sangon Biotech | A600560-0500 |
| TMB single-component chromogenic solution | Solarbio | PR1200 |
| ELISA stop solution | Solarbio | C1058 |
| Human CD40 Ligand/TNFSF5 Protein, His, Flag Tag, active trimer (MALS verified) | Acro | CDL-H52Db |
| Biotinylated Human CD40/TNFRSF5 Protein, Fc, Avitag™ (MALS verified) | Acro | TN5-H82F9 |
| HRP Streptavidin | Biolegend | 405210 |
| ExpiFectamine CHO Enhancer | Gibco | 100033019 |
| ExpiCHO Feed | Gibco | A29101-01 |
| FectPro | Polyplus | 101000007 |
| Fectamine | Gibco | A38915 |

The details of the method are as follows:

a) Antigen coating: Human CD40 Ligand was diluted to 1 μg/mL with 1X PBS, and 100 μL/well was added to a 96-well immunoplate. The plate was sealed and incubated overnight at 4°C;

b) Blocking: The supernatant was discarded from the immunoplate, which was then washed 3 times with PBST wash buffer. 1% BSA was added at 300 μL/well for blocking for 2 h;

c) Preparation of primary antibody: The antibody to be tested was diluted from a starting concentration of 20 μg/mL using 1% BSA in a 2-fold serial dilution to prepare the working solutions; Biotinylated Human CD40 was diluted to 2 μg/mL with 1% BSA for later use;

d) Primary antibody incubation: The supernatant was discarded from the immunoplate, which was then washed six times with PBST wash buffer; antibody was added at 50 μL/well, followed by Biotinylated Human CD40 at 50 μL/well, and incubated for 2 h;

e) Preparation of enzyme-labeled antibody: HRP Streptavidin was diluted 1:3,000 with 1% BSA for later use;

f) Enzyme-labeled antibody incubation: The supernatant was discarded from the immunoplate, which was then washed three times with PBST wash buffer. Diluted HRP Streptavidin was added at 100 μL/well and incubated for 1 h;

g) Chromogenic reaction: The supernatant was discarded from the immunoplate, which was then washed three times with PBST wash buffer. TMB chromogenic solution was added at 100 $\mu$L/well and incubated for 5-10 min for the chromogenic reaction. After the chromogenic reaction was completed, the stop solution was added at 50 $\mu$L/well. The absorbance was read at 450 nm using a microplate reader.

The results are shown in FIG. 1. Compared with the positive control INX-021, the antibody molecules Hz33B9C5 and Hz47E2 of the present invention exhibited a lower percentage of CD40 protein binding, indicating that Hz33B9C5 and Hz47E2 more effectively inhibit the binding of CD40 protein to its ligand CD40L protein. In summary, compared with the positive control INX-021, the antibody molecules Hz33B9C5 and Hz47E2 of the present invention demonstrate superior target-blocking activity.

**Example 4: Inhibition of downstream signaling activation in Jurkat cells overexpressing CD40 by the antibody of the present invention at the cellular level**

The inhibitory activity of the anti-CD40L antibodies Hz33B9C5 and Hz47E2 on the activation of the CD40 downstream signaling pathway is detected using the Jurkat-NF$\kappa$B-Luc reporter system. In the absence of anti-CD40L antibodies, Jurkat-NF$\kappa$B-Luc cells overexpressing CD40 produce luciferase and catalyze substrate luminescence when stimulated by CHO-CD40L cells. When the anti-CD40L antibodies bind to the CD40L overexpressed on the surface of CHO cells, they inhibit the interaction between CD40L and the membrane protein of Jurkat-NF$\kappa$B-Luc overexpressing CD40, resulting in reduced secretion of luciferase. Therefore, the inhibition of cell activation by Hz33B9C5 and Hz47E2 can be evaluated using the Luciferase reporter system.

Construction of a CHO-CD40L cell line overexpressing CD40L:
The CD40L protein sequence (SEQ ID NO: 27) was cloned into the PXC17.4 vector. The prepared vector plasmid was co-incubated with Pvul-HF in diluted CutSmart Buffer to perform a restriction enzyme digestion reaction. GS-CHO cells (source: Lonza) were centrifuged, and the supernatant was removed. The cells were gently resuspended in CD CHO Medium containing 1% SP4. The digested plasmid was added to the cells, mixed well, and transferred into an electroporation cuvette. The electroporator program was set to 300 V, 900 $\mu$F, and resistance infinity, and electroporation was performed. After electroporation, the cells were immediately transferred into prewarmed CD CHO medium containing 1% SP4 and cultured in a shaker at 120 rpm, 37°C, with 6% $CO_2$. After 1-2 days of culture, the culture medium was replaced with culture medium containing MSX for screening. Cell viability initially decreased and then recovered. Once viability recovered to above 95%, the cells were cryopreserved for later use.

Construction of the Jurkat-Luc-CD40 cell line overexpressing CD40:
The CD40 protein sequence (SEQ ID NO: 32) was cloned into the pLVX-IRES-EGFP vector. The prepared vector plasmid was mixed with pSPAX, pMD2.G, PEIpro, and Opti-MEM, and transfected into 293T cells for viral packaging. After 48 h, the virus was collected, concentrated, and used to infect the NF$\kappa$B Reporter Jurkat cell line (source: Genomeditech (Shanghai) Co., Ltd.). Cells with fluorescence signals were selected by flow cytometry, expanded in culture, and cryopreserved for later use.

In the co-culture system of the constructed CHO-CD40L overexpressing cells and Jurkat-Luc-CD40 overexpressing cells, the addition of the antibody of the present invention blocked the binding between CD40L on the surface of CHO cells and CD40 on the surface of Jurkat-NF$\kappa$B-Luc cells, thereby inhibiting the activation of downstream NF$\kappa$B in Jurkat cells and the production of luciferase, decreasing the fluorescence signal in the system. The experimental results are shown in FIG. 2.

The details of the method are as follows:

a) The cells were cultured to the logarithmic growth phase, and the cell numbers were adjusted using 1640 complete medium, with Jurkat-Luc-CD40 adjusted to $1 \times 10^5$ cells/45 $\mu$L and CHO-CD40L adjusted to 5,000 cells/45 $\mu$L.

b) Antibody preparation: The antibody concentration was adjusted to 200 $\mu$g/mL using 1640 complete medium and then diluted in a 2-fold serial dilution for later use.

c) Sample addition: CHO-CD40L cells were first gently pipetted 5-7 times, then added to a 96-well immunoplate at 45 $\mu$L/well. Subsequently, 10 $\mu$L/well of the prepared antibody dilution was added and mixed by pipetting 5 times with a multichannel pipette. Finally, Jurkat-Luc-CD40 cells were gently pipetted 5-7 times and then added at 45 $\mu$L to the 96-well immunoplate, followed by mixing by pipetting 5 times with a multichannel pipette. The 96-well immunoplate was placed in a cell culture incubator at 37°C, with 5% $CO_2$, and cultured for 6 h.

d) After reacting for 6 h, the 96-well immunoplate was removed and equilibrated to room temperature. The Bio-Glo™ Luciferase Assay System was added at a 1:1 volume ratio, and after reacting at room temperature for 10 min, the readings were taken.

Equipment: Fluorescence quantitative microplate reader SPARK

Reagents and raw materials:

| Name | Manufacturer | Catalog No. |
|---|---|---|
| RPMI medium 1640 (1x) | Gibco | 22400-089 |
| FBS | Hyclone | SH30406.05 |
| Bio-Glo™ Luciferase Assay System | Promega | G7940 |
| PXC17.4 vector | GENEWIZ from Azenta Life Sciences | NA |
| PvuI-HF | NEB | R3150SVIAL |
| CutSmart Buffer | NEB | B6004SVIAL |
| CD CHO Medium | Gibco | 10743-029 |
| MSX | SIGMA | M5379-500MG |
| pLVX-IRES-EGFP vector | GENEWIZ from Azenta Life Sciences | NA |
| pSPAX | GENEWIZ from Azenta Life Sciences | 80-735332355 |
| pMD2.G | GENEWIZ from Azenta Life Sciences | 80-735332355 |
| PEIpro | Polyplus | 115-100 |
| Opti-MEM | Gibco | 11058021 |

The results are shown in FIG. 2. Compared with the positive control INX-021, the antibody molecules Hz33B9C5 and Hz47E2 of the present invention more strongly inhibited downstream NF$\kappa$B signal activation of CD40-overexpressing cells, indicating that the antibody molecules of the present invention can significantly inhibit CD40 downstream signal activation mediated by CD40L.

**Example 5: Inhibition of B cell activation marker CD86 expression by the antibody of the present invention**

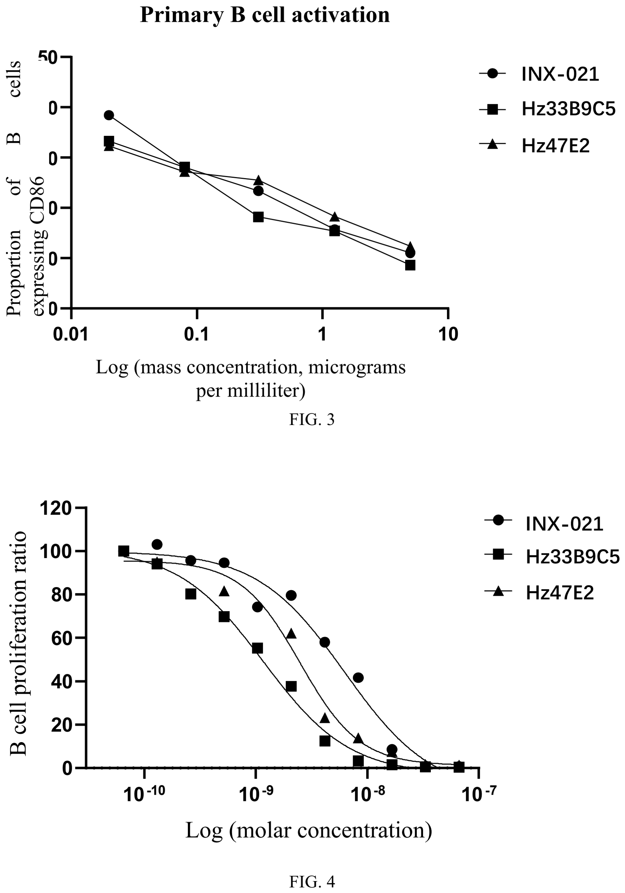

The inhibition of B cell activation by the anti-CD40L antibodies Hz33B9C5 and Hz47E2 in an in vitro culture system of peripheral blood mononuclear cells (PBMCs) was detected by flow cytometry (FACs). When B cells receive stimulation signals, the expression of the activation marker CD86 on their surface increases rapidly, but in the presence of anti-CD40L antibodies, B cell activation is inhibited. Therefore, the inhibitory effect of the antibody can be reflected by detecting the proportion of CD86 expression on the surface of B cells. The experimental results are shown in FIG. 3.
The details of the method are as follows:

a) PBMCs were taken, rapidly thawed in a 37°C water bath, added to 9 mL of complete medium, and centrifuged at 300 g for 5 min. The supernatant was discarded, and the cells were resuspended in complete medium (RPMI supplemented with 10% fetal bovine serum). The cells were then added into a 96-well immunoplate (U-shaped) at $5 \times 10^3$ cells per well;

b) CHO-CD40L cells were taken and added into the above immunoplate at $5 \times 10^3$ cells per well, followed by mixing evenly by pipetting with a multichannel pipette;

c) Different concentrations of the antibody to be tested were diluted and added to the above immunoplate, then mixed evenly by pipetting with a multichannel pipette, and cultured for 18 h;

d) Staining was performed, and detection was carried out by flow cytometry (PC5.5 CD19, PE CD86, LIVE/DEAD™ Fixable Near-IR Dead Cell Stain Kit).

Equipment: Beckman Cytoflex

Reagents and raw materials:

| Name | Manufacturer | Catalog No. |
|---|---|---|
| Human PBMCs | Shanghai Saily Biotechnology Co., Ltd. | XFB-HP100B |
| RPMI | HYCLONE | SH30809.01 |
| Fetal bovine serum | HYCLONE | SH30084.03 |
| PC5.5 CD19 | Beckman Coulter | A66328 |
| PE CD86 | Biolegend | 325406 |
| LIVE/DEAD™ Fixable Near-IR Dead Cell Stain Kit | Invitrogen | L34975 |

As shown in FIG. 3, the antibody molecules Hz33B9C5 and Hz47E2 of the present invention inhibited CD86 expression in B cells in a dose-dependent manner, indicating that they significantly inhibit early activation of B cells, and the inhibitory effect is similar to that of the positive control INX-021.

**Example 6: Effect of the antibody of the present invention on proliferation of isolated B cells in vitro**

B cells proliferate extensively during the process of class switching and differentiation into plasma cells. By isolating B cells from PBMCs and culturing them in vitro under certain conditions, B cells rapidly proliferate in a system containing both the first and second signals as well as cytokines. The addition of Hz33B9C5 or Hz47E2 blocks the interaction between CD40L, as the second signal, and CD40 on the surface of B cells, thereby inhibiting B cell proliferation. The experimental results are shown in FIG. 4.

The details of the method are as follows:

a) PBMCs were resuscitated, rapidly thawed in a 37°C water bath, added to 9 mL of complete medium, and centrifuged at 300 g for 5 min. The supernatant was discarded, and the cells were resuspended in MACs buffer.

b) B cells were isolated from PBMCs according to the B cell isolation kit II protocol, counted, and then incubated in 10 mL of complete medium containing 0.5 $\mu$g/mL Anti-IgM (AffiniPure F(ab')$_2$ Fragment Goat Anti-Human IgM) at 4°C for 2 h.

c) The treated cells were washed and counted, and B cells were diluted at 5e+5/mL in culture medium containing 1 $\mu$g/mL Anti-IgM, 160 ng/mL Recombinant Human IL-21 Protein, and 2 $\mu$g/mL CD40L.

d) Each antibody was serially diluted twofold in PBS starting from 20 $\mu$g/mL in the first well across 12 dilutions, and 100 $\mu$L of antibody + 100 $\mu$L of B cells were added into a 96-well white-bottom immunoplate.

e) On Day 4 of culture, fresh culture solution with the same antibody concentrations as in step d) above was prepared, and half of the culture solution was replaced.

f) On Day 7, the immunoplate was centrifuged, 60 $\mu$L of supernatant was carefully aspirated from each well, and 100 $\mu$L of CellTiter-Glo® Luminescent Cell Viability Assay was added. The mixture was pipetted 3-5 times, left to stand at room temperature for 10 min, and then placed in a microplate reader to take readings.

Equipment: Fluorescence quantitative microplate reader SPARK

Reagents and raw materials:

| Name | Manufacturer | Catalog No. |
|---|---|---|
| RPMI | HYCLONE | SH30809.01 |
| Australian fetal bovine serum (FBS) | HYCLONE | SH30084.03 |
| Penicillin-Streptomycin | GIBCO | 15070-063 |
| $\beta$-mercaptoethanol | SIGMA | 07604-100ML |

(continued)

| Name | Manufacturer | Catalog No. |
|---|---|---|
| HEPES (1 M) | GIBCO | 15630080 |
| AffiniPure F(ab')$_2$ Fragment Goat Anti-Human IgM, Fc5$\mu$ fragment specific | Jackson | 109-006-129 |
| Recombinant Human IL-21 Protein | R&D | 8879-IL-050 |
| CD40L | Acro | CDL-H52Db |
| Cell Titer-Glo® Luminescent Cell Viability Assay | Promega | G7573 |
| B cell isolation kit II | Miltenyi Biotec | 130-091-151 |

The results are shown in FIG. 4. By detecting cell numbers using the CellTiter-Glo® kit, Hz33B9C5 and Hz47E2 were found to inhibit B cell proliferation induced by CD40L signaling in the in vitro B cell culture system in a dose-dependent manner, and the inhibitory effect was comparable to that of the positive control INX-021.

**Example 7: Effect of the antibody of the present invention on B cell differentiation into plasma cells**

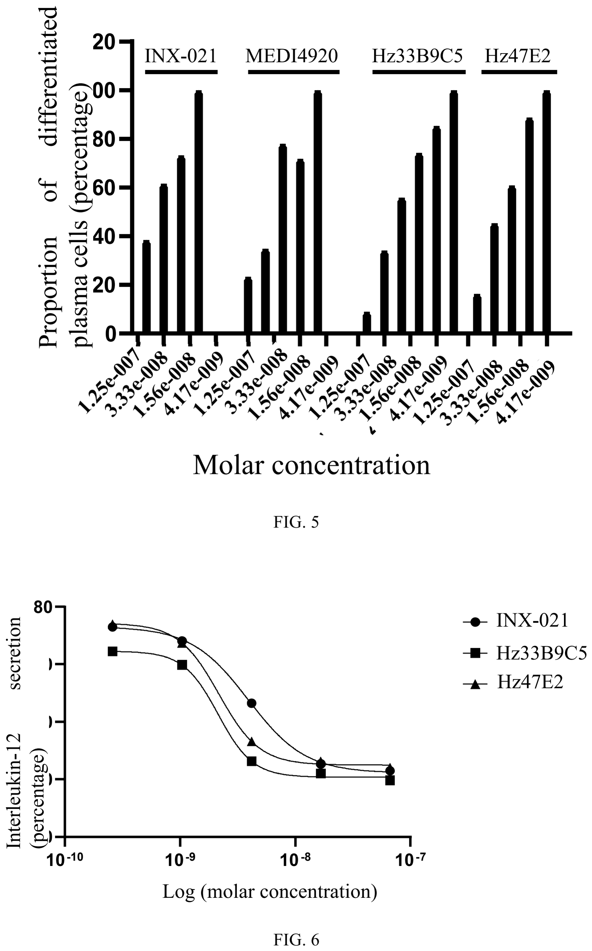

Under appropriate conditions, B cells differentiate into plasma cells that ultimately produce antibodies. The antibodies secreted by plasma cells are the primary mode of action of humoral immunity in adaptive immunity. B cells isolated from PBMCs differentiate into plasma cells when stimulated with 3T3-CD40L cells, Anti-IgM (AffiniPure F(ab')$_2$ Fragment Goat Anti-Human IgM, Fc5$\mu$ fragment specific), and IL-21. At the same time, the addition of anti-CD40L antibody Hz33B9C5 or Hz47E2 inhibits the interaction between 3T3-CD40L and B cells, thereby reducing the differentiation of B cells into plasma cells. The experimental results are shown in FIG. 5.

Construction of the 3T3-CD40L cell line overexpressing CD40L:

a) Lentiviral (Lenti) packaging

1) One T175 flask of 293T cells was plated;

2) When the cell density reached over 80%, transfection was performed. Before transfection, the culture medium in the T175 flask was discarded, and 20 mL of basal DMEM medium (without serum and antibiotics) was added. The flask was then placed back into a 37°C, 5% CO$_2$ incubator;

3) The transfection mixture was prepared, wherein the ratio of plasmid:pSPAX2:pMD2.G:PEIpro was 2:2:1:15. The total volume of the transfection mixture was 10% of the total culture volume;

4) The prepared transfection mixture was gently added dropwise into the culture flask. The flask was gently shaken to distribute the mixture evenly and then placed in a 37°C, 5% CO$_2$ incubator for culture;

5) After 6 h of transfection, the culture medium in the culture flask was aspirated, 20 mL of fresh DMEM medium containing 4% FBS was added, and left in a 37°C, 5% CO$_2$ incubator;

b) Virus collection and infection of 3T3 cells (source: Cell Bank, Shanghai Institutes for Biological Sciences, Chinese Academy of Sciences)

1) One day before infection, 3T3 cells were prepared by seeding them into a 6-well immunoplate, with 4 wells designated for the experimental group and 2 wells for the negative control group;

2) On the day of infection, the virus-containing supernatant obtained in step a) was collected and centrifuged (500 g, 10 min) to remove dead cells;

3) The centrifuged virus-containing supernatant was added to the 3T3 cells, with 2 mL of virus-containing supernatant added to each well of the experimental group, and 2 mL of culture medium added to each well of the negative control group. The cells were then transferred to the incubator;

4) One day after viral infection, the medium was replaced with complete medium (10% FBS, containing antibiotics);

c) Cell sorting

1) After the cells had stably expanded, they were collected by centrifugation and stained for CD40L on the cell surface using flow cytometry antibodies;

2) Sorting was performed using a flow cytometer to screen out cells with fluorescence signals;

3) The sorted cells were expanded in culture to obtain 3T3-CD40L cells, which were cryopreserved for later use.

The details of the method are as follows:

a) 3T3-CD40L cells were treated with 10 μg/mL mitomycin C at 37°C for 30 min and seeded into a 96-well immunoplate at 6,250 cells per well.

b) B cell basal medium was prepared on the following day: 50 mL RPMI1640 + 0.192 μL 2-mercaptoethanol + 100 μL HEPES. All subsequent reagents were prepared using this medium.

c) B cells were separated from PBMCs using the B cell isolation kit II. The separated cells were treated in 10 mL of B cell basal medium containing 0.5 μg/mL anti-IgM at 4°C for 2 h, washed, counted, and diluted to 1e+6/mL in B cell basal medium containing 160 ng/mL IL-21.

d) Each antibody was prepared at an initial concentration of 40 μg/mL for the first well and then diluted in a 2-fold serial dilution across 12 gradients. The culture medium in the 96-well immunoplate seeded with 3T3-CD40L cells was aspirated, and 100 μL of antibody + 100 μL of B cells were added to each well. The addition was performed slowly, and the mixture must not be mixed by pipetting.

e) On Day 4 of culture, half of the B cell basal medium was replaced.

f) On Day 7 of differentiation, the cells were stained with IgD (BB515 Mouse Anti-Human IgD, 1:100), CD38 (Brilliant Violet 785™ anti-human CD38 Antibody, 1.5:100), CD19 (CD19-PE-CF594, 1:100), and DCM (DCM APC-Cy7, 1:1000), and analyzed by flow cytometry.

Equipment: BD Symphony

Reagents and raw materials:

| Name | Manufacturer | Catalog No. |
|---|---|---|
| RPMI1640 | HYCLONE | SH30809.01B |
| IL21 (100 μg/mL) | R&D | 8879-IL-050 |
| AffiniPure F(ab')₂ Fragment Goat Anti-Human IgM, Fc5μ fragment specific (1.3 mg/mL) | jakson | 109-006-129 |
| B cell isolation kit II, human | Miltenyi Biotec | 130-091-151 |
| FBS | HYCLONE | SH30406.05 |
| DMEM | HYCLONE | SH30022.01 |
| 2-mercaptoethanol | Thermo Fisher/Gibco | 21985023 |
| Brilliant Violet 785™ anti-human CD38 Antibody | Biolegend | 303530 |
| CD19-PE-CF594 | BD Biosciences | 562294 |
| BB515 Mouse Anti-Human IgD | BD Pharmingen | 565243 |
| DCM APC-Cy7 | Invitrogen | L34963 |
| pSPAX2:pMD2.G: | GENEWIZ from Azenta Life Sciences | NA |

(continued)

| Name | Manufacturer | Catalog No. |
|------|--------------|-------------|
| PEIpro | Polyplus | 115-100 |
| HEPES | GIBCO | 15630080 |

As shown in FIG. 5, similar to the positive controls INX-021 and MEDI4920 (as set forth in SEQ ID NO: 145 of CN103874501B), the antibody molecules Hz33B9C5 and Hz47E2 of the present invention inhibited the proportion of plasma cell differentiation in a concentration-dependent manner. It is evident that, by inhibiting the interaction between 3T3-CD40L and B cells, the antibodies of the present invention can reduce the differentiation of B cells into plasma cells.

## Example 8: Effect of the antibody of the present invention on IL-12 secretion by moDCs

Although CD40 is mainly expressed on B cells, CD40 expressed on DCs, which also serve as antigen-presenting cells, interacts with CD40L in many cases to participate in immune system responses. IL-12 secreted by DCs acts on receptors on T cells and NK cells, inducing T cell differentiation and NK cell secretion of IFN-$\gamma$ and TNF-$\alpha$, thereby promoting inflammatory responses. In this experiment, monocytes were separated from PBMCs and differentiated into moDCs under the induction of IL-4 and GM-CSF. The cells were co-cultured in the presence of CD40L protein and the antibodies of the present invention. The concentration of IL-12 secreted by DCs was detected using the ELISA method. The experimental results are shown in FIG. 6.

The details of the method are as follows:

a) PBMCs (50 million/vial) were resuscitated, and monocytes were negatively selected using the Pan monocyte isolation kit;

b) The cells were resuspended in X-VIVO culture medium and seeded into a 6-well immunoplate. GM-CSF at 100 ng/mL and IL-4 at 10 ng/mL were added to induce differentiation for 7 days. On Day 2 and Day 4, 1 mL of culture medium containing 100 ng/mL GM-CSF and 10 ng/mL IL-4 was supplemented;

c) The induced imDCs obtained in step b) were collected, resuspended in X-VIVO culture medium, and the cell density was adjusted to $2.5 * 10^5$/mL;

d) The DC suspension obtained in step c) was added to a 96-well flat-bottom immunoplate at 100 $\mu$L/well;

e) CD40L was prepared at 4 $\mu$g/mL using RPMI1640 culture medium containing 10% FBS and added at 50 $\mu$L/well, resulting in a final system concentration of 1 $\mu$g/mL;

f) The antibody to be tested was diluted to 20 $\mu$g/mL with X-VIVO culture medium. 50 $\mu$L of the diluted antibody was added to the cells, mixed well, and incubated at 37°C;

g) After 4 days of culture, the cell culture supernatant was collected, and the content of IL-12 p40 was detected using a commercial ELISA kit (Human IL-12/IL-23 p40 ELISA kit).

Equipment: Thermo Fisher MULTISKAN-FC

Reagents and raw materials:

| Name | Manufacturer | Catalog No. |
|------|--------------|-------------|
| Pan monocyte isolation kit | Miltenyi Biotec | 130-096-537 |
| X-VIVO | LONZA | 04-418Q |
| GM-CSF | R&D | 215-GM/CF |
| IL-4 | R&D | 204-IL |
| Human IL-12/IL-23 p40 ELISA kit | Multi Sciences | EK1183-96 |

As shown in FIG. 6, compared with the positive control INX-021, the antibody molecules Hz33B9C5 and Hz47E2 of the present invention more strongly inhibited IL-12 secretion by DCs, indicating that the antibodies of the present invention more significantly inhibit IL-12, thereby suppressing the series of inflammatory responses mediated by IL-12.

**Example 9: Non-induction of platelet activation in in vitro experiments by the antibody of the present invention**

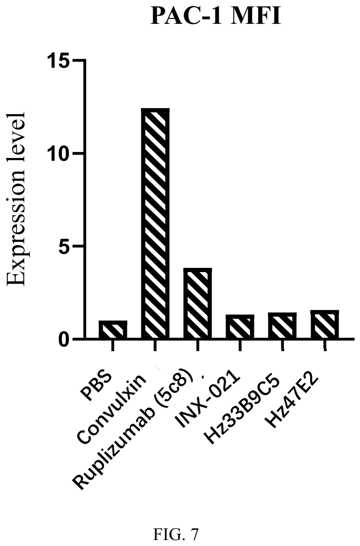

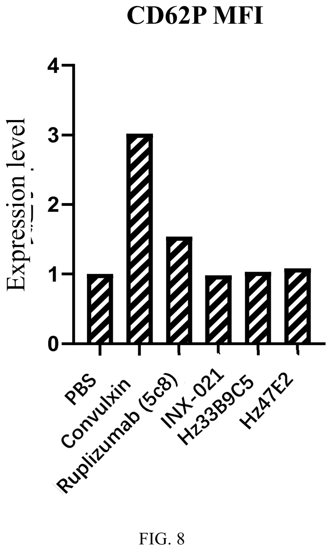

To determine whether the antibodies of the present invention induce platelet activation, two detection methods were employed, including the expression of marker molecules for platelet activation and a whole blood platelet aggregation analysis system to assess aggregation. Platelets were treated with a positive control drug and a negative control drug, followed by detection and quantitative analysis of the expression of the activation marker molecules CD62P and PAC1 on platelets using flow cytometry technology. At the same time, real-time monitoring and quantitative analysis were conducted using a whole blood platelet aggregation analysis system. The experimental results are shown in FIGS. 7-9.

The details of the method are as follows:

a) Human platelets were resuspended in Tyrode's buffer (sodium chloride (140 mM), potassium chloride (5 mM), HEPES (25 mM), calcium chloride dihydrate (2 mM), magnesium chloride hexahydrate (2 mM), dextrose/glucose (10 mM), water (rest)) and temporarily stored at room temperature. The platelet suspension was centrifuged at 3,000 rpm for 2 min, and the pellet was gently resuspended in PBS. Platelet counting was performed using a three-part hematology analyzer, typically yielding $3 * 10^{11}$/L.

b) The CD40L antigen and the antibody to be tested were mixed evenly at a molar ratio of 1:1, with the final concentrations of both CD40L and the antibody drug adjusted to 3 μM. The mixture was left to stand at room temperature for 15 min to activate the antibody drug and form an immune complex.

c) Platelet aggregation analysis system analysis: The Chrono-Log 560Ca aggregometer was preheated to 37°C. 200 μL of platelets and 50 μL of the antigen-antibody complex were gently mixed in a cuvette. The stirring speed was set to 1,000 rpm. $CaCl_2$ was added to a final concentration of 1 mM. A cuvette containing PBS was used as the control to reset the baseline, and the change curve of cuvette light transmittance over time was recorded.

d) Flow cytometry analysis: The antigen-antibody complex was prepared at the above ratio in a 10 μL system, and $CaCl_2$ was added to a final concentration of 1 mM. 50 μL of platelets was mixed with the aforementioned 10 μL system and incubated in a 37°C water bath for 10 min. Then, 2.5 μL of P-selectin and PAC-1 flow cytometry antibodies were added, and the mixture was incubated away from light at room temperature for 15 min. 300 μL of PBS was added for dilution and mixed evenly, and the sample was loaded into a flow tube for analysis.

Equipment: Sysmex Corporation three-part hematology analyzer (model: KX-21N), Chrono-Log Corporation whole blood platelet aggregation analysis system (model: Chrono-Log 560Ca), BD Biosciences flow cytometry analyzer (model: FACS Calibur)

Reagents and raw materials:

| Name | Manufacturer | Catalog No. |
|---|---|---|
| FITC anti-human CD41/CD61 | BioLegend | 362804 |
| Hu CD62P PE AK-4 | BD Pharmingen | 555524 |
| Convulxin | Enzo Life Sciences | ALX-350-100-C050 |
| Ruplizumab | Shanghai Biointron Co., Ltd. | 20210919A062 |
| PBS | Solarbio | P1020-500ml |

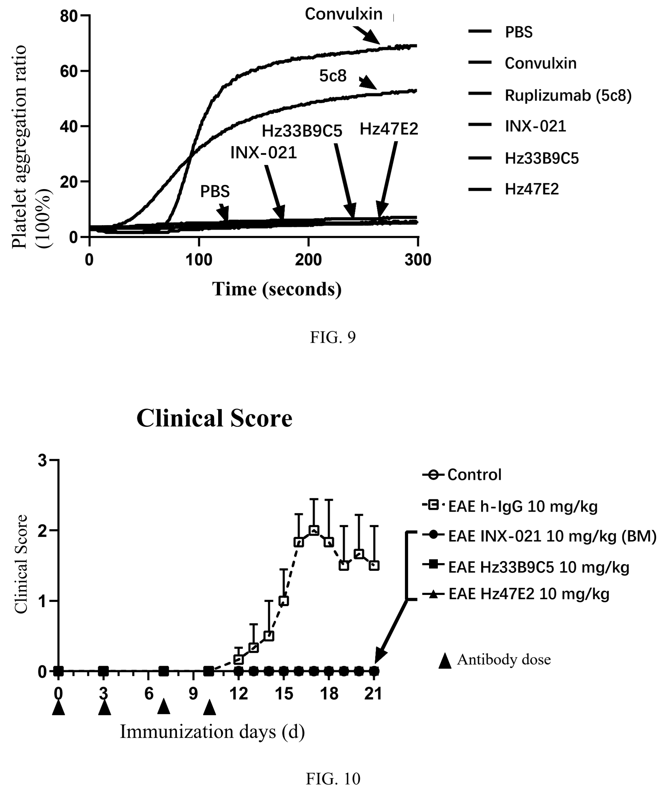

As shown in the FACS results in FIG. 7-8, compared with the positive controls Convulxin and Ruplizumab, the antibody molecules Hz33B9C5 and Hz47E2 of the present invention had almost no effect on the expression of the platelet activation markers PAC-1 and CD62P. At the same time, the antibodies of the present invention exhibited performance similar to that of the Fc-mutated antibody INX-021. Meanwhile, as shown in FIG. 9, the platelet aggregation ratio of the antibodies of the present invention was essentially the same as that of the negative control PBS, comparable to that of INX-021, and significantly lower than that of Convulxin and Ruplizumab. This indicates that the immune complexes formed between the antibodies of the present invention and CD40L do not cause platelet aggregation or activation.

**Example 10: Data for the antibody of the present invention in an experimental autoimmune encephalomyelitis (EAE) mouse model**

Multiple sclerosis (MS) is a chronic inflammatory disease of the central nervous system that can lead to encephalitis and demyelination. MS is considered an autoimmune disease caused by autoreactive T cells, and is characterized by recurrent relapses, with symptoms including muscle stiffness and paralysis, visual disturbances and blindness, loss of sensation, and ataxia. At present, there are several distinct animal models for MS, among which the EAE model is widely used in the studies of MS due to its pathological features of inflammation and demyelination similar to those of MS. Myelin proteins (in this experiment, myelin oligodendrocyte glycoprotein [MOG] was used) or peptides of these proteins are used to immunize and induce the EAE model. This may be due to the activation of myelin-specific T cells in the periphery, which cross the blood-brain barrier into the central nervous system and are reactivated, triggering a series of inflammatory responses that lead to demyelination and axonal apoptosis, ultimately causing nerve damage and loss of function. A standardized scoring system is used to assess clinical symptoms and quantify the extent of disease induction.
The scoring criteria for the EAE model mice used in this experiment are as follows:

0 points: The mouse is healthy

1 point: The mouse has a limp tail

2 points: The mouse has paralysis in one hind limb

3 points: The mouse has paralysis in both hind limbs

4 points: The mouse has lost 20% or more of body weight

5 points: The mouse is dead or moribund

In addition, the health status of mice can also be indirectly assessed by measuring their body weight, and the inhibitory effect of the antibody of the present invention on the antibody-producing function in mice can be reflected by detecting the titer of anti-MOG antibodies in mouse peripheral blood serum. The experimental results are shown in FIGS. 10-12.
The details of the method are as follows:

a) Reagent preparation:

1. Inactivated Mycobacterium tuberculosis stock solution (H37Ra stock solution) (100 mg/mL): 10 mL complete Freund's adjuvant + 100 mg H37Ra, dissolved by ultrasound;

2. MOG35-55 stock solution (10 mg/mL): 5 mL complete Freund's adjuvant + 50 mg MOG35-55;

3. Pertussis toxin stock solution (PTX stock solution) (100 $\mu$g/mL): 50 $\mu$g PTX + 500 $\mu$L DPBS (aliquoted and stored in a -40°C freezer);

4. Inactivated Mycobacterium tuberculosis working solution (H37Ra): 500 $\mu$L complete Freund's adjuvant + 500 $\mu$L inactivated Mycobacterium tuberculosis stock solution;

5. MOG35-55 emulsifier: 2 mL of MOG35-55 emulsifier = 200 $\mu$L of MOG35-55 stock solution + 800 $\mu$L of DPBS + 1 mL of H37Ra working solution, used after thorough emulsification;

6. Pertussis toxin working solution (PTX working solution): 30 $\mu$L of PTX stock solution + 1970 $\mu$L of DPBS (prepared fresh for immediate use)

b) Modeling method:

1. Each group consisted of 5 mice aged 8-10 weeks, all female, administered with control (PBS), hIgG, INX-021, Hz33B9C5, and Hz47E2, with a dosage of 10 mg/kg for all groups;

2. Day 0: 100 μL of MOG35-55 emulsifier was injected at two points each on the back; 200 μL of PTX working solution was administered via tail vein injection; antibody or control was administered via IP injection;

3. Day 3: PTX working solution was administered via tail vein injection, and antibody or control was administered via IP injection;

4. On Day 7 and Day 10, the antibody or control was administered via IP injection;

5. On Days 0, 3, 7, 10, and 12-21, disease severity was evaluated based on a 0-5 scoring system, and mouse body weight was recorded;

6. On Days 7, 14, and 21, blood was collected from the orbital sinus of the mice to obtain serum, and the titer of anti-MOG antibody in the serum was detected by ELISA.

Equipment: Thermo Fisher MULTISKAN-FC

Reagents and raw materials:

| Name | Manufacturer | Catalog No. |
| --- | --- | --- |
| MOG35-55 | MedChemExpress | HY-P1240A |
| PTX | EMD | 516560-50UG |
| Humanized CD40/CD40L mice | Shanghai Model Organisms | 20170010014874 |
| Complete Freund's adjuvant | SIGMA | F5881-10X10ML |
| HRP Goat anti-mouse IgG (minimal x-reactivity) Antibody | Biolegend | 405306 |
| H37Ra | BD | 231141 |
| DPBS | Gibco | 70011-044 |
| IgG | Innovent Biologics Co., Ltd. | 20210330 |

As shown in FIGS. 10-11, administration of the antibodies Hz33B9C5 and Hz47E2 of the present invention at 10 mg/kg significantly reduced the clinical score of experimental EAE without affecting the body weight of the mice. These effects were close to those of the control group not induced with MOG and were superior to those of the positive control INX-021. These results indicate that Hz33B9C5 and Hz47E2 are capable of improving experimental EAE and autoimmune diseases such as MS.

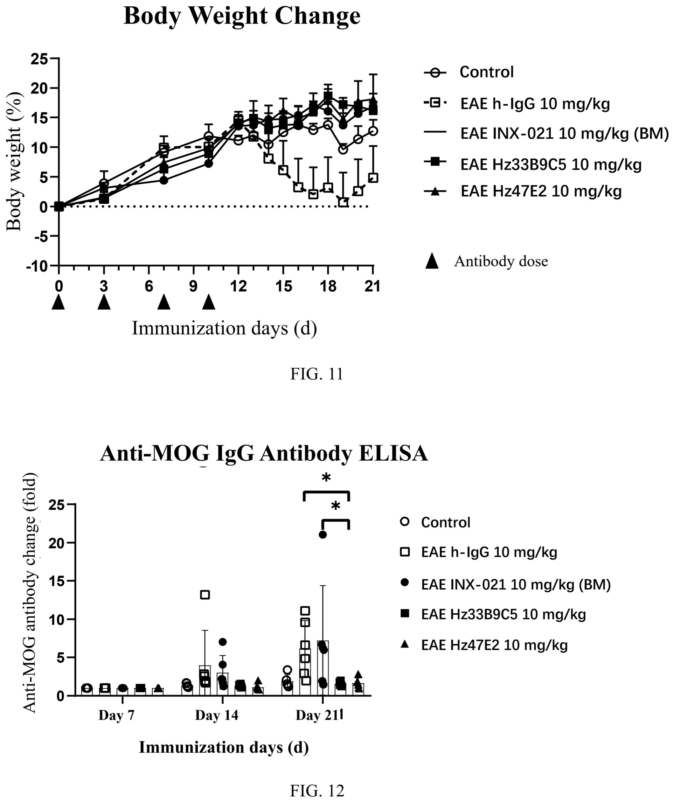

As shown in FIG. 12, following administration of the antibodies Hz33B9C5 and Hz47E2 of the present invention at 10 mg/kg, the anti-MOG antibody titer was lower than that following administration of INX-021, particularly at 14 days and especially at 21 days after immunization. This indicates that the antibodies of the present invention exhibit a stronger inhibitory effect on humoral immunity compared to INX-021, and preferably possess a stronger ability to inhibit antibody production.

**Example 11: Inhibition of anti-keyhole limpet hemocyanin antibody production in mice by the antibody of the present invention**

Since peptides, small proteins, and certain drug molecules are haptens and generally lack immunogenicity, they require the help of carrier proteins such as hemocyanin (KLH) to stimulate an immune system response in the form of antibody production. Therefore, KLH is widely used in studies, biotechnology, and therapeutic applications. At the same time, since KLH is derived from limpets (a type of gastropod), which differ significantly from mammals in terms of development and growth, the likelihood of producing false-positive results in mammal-based immunological studies is relatively low.

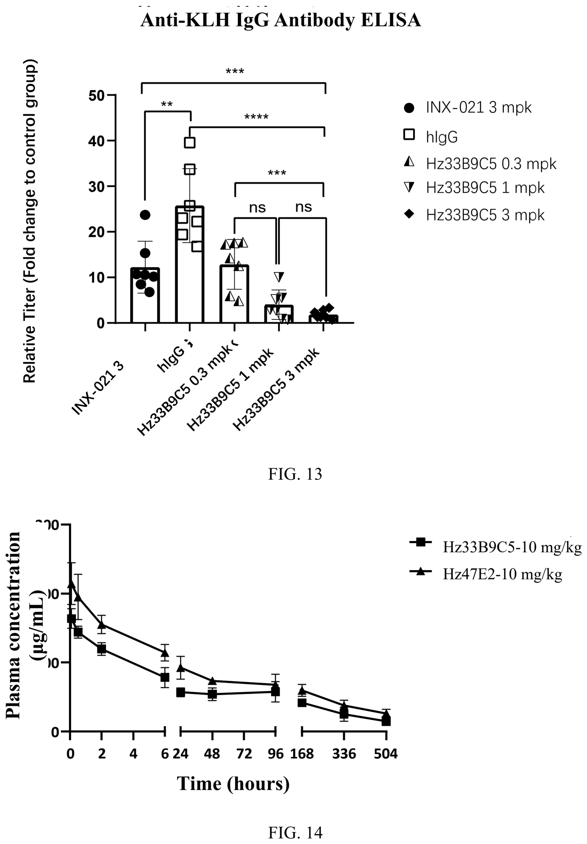

Following injection of the antibody of the present invention into humanized CD40/CD40L mice and subsequent immunization with KLH, the antibody of the present invention inhibits the interaction between CD40L and CD40, thereby suppressing B cell maturation, differentiation, and the production of anti-KLH antibodies. The experimental results are shown in FIG. 13.

The details of the method are as follows:

a) Reagent preparation: KLH was prepared at 250 $\mu$g per mouse; it was correspondingly dissolved in 100 $\mu$L of PBS for later use;

b) Modeling method:

1. Each group consisted of 5 mice, approximately 8-10 weeks of age, separately administered with control hIgG, INX-021 (3 mpk), and Hz33B9C5 (0.3 mpk, 1 mpk, and 3 mpk);

2. Day 0: The KLH stock solution was mixed with alum adjuvant at a volume ratio of 1:1 and administered via IP injection, while the antibody drug was also administered via IP injection to the mice;

3. Day 7: The antibody drug was administered to the mice via IP injection;

4. On Days 7, 14, and 21, blood was collected from the orbital sinus of the mice to obtain serum, and the titer of anti-MOG antibody in the serum was detected using the ELISA method.

Equipment: Thermo Fisher MULTISKAN-FC

Reagents and raw materials:

| Name | Manufacturer | Catalog No. |
|---|---|---|
| KLH derived from the giant keyhole limpet (Megathura crenulata) | SIGMA | H7017-20MG |
| Imject™ Alum Adjuvant | Thermo Scientific | 77161 |
| Humanized CD40/CD40L mice | Biocytogen | 320726210100343216 |
| HRP Goat anti-mouse IgG (minimal x-reactivity) Antibody | Biolegend | 405306 |

As shown in FIG. 13, in humanized CD40/CD40L mice, compared to INX-021, the concentration of anti-KLH IgG in the serum of mice in the Hz33B9C5 group of the present invention was lower. This indicates that the antibody of the present invention more strongly inhibits the interaction between CD40L and CD40, thereby suppressing T cell-dependent B cell maturation and differentiation, and ultimately reducing the production of anti-KLH antibodies. Moreover, in different Hz33B9C5 dose groups, the serum titer of anti-KLH IgG in mice exhibited a dose-response trend, indicating that the pharmacological effect of Hz33B9C5 in this animal model is dose-dependent.

**Example 12: The pharmacokinetics of the antibody of the present invention in mice**

**Experimental Materials**

**Information of Reagents and Consumables**

| Name | Brand | Catalog No. | Batch No. |
|---|---|---|---|
| 96-well immunoplate | USA, Thermo | 442404 | 149868 |
| 10× PBS buffer | Shanghai, Sangon | E607016-0500 | F910FA0002 |
| TMB single-component chromogenic solution | Beijing, Solarbio | PR1200 | 20190404 |
| BSA | Shanghai, Sangon | A500023-0100 | F722BA0005 |
| Tween 20 | Shanghai, Sangon | A600560-0500 | FB11BA0001 |
| Carbonate powder | USA, Thermo | 28382 | QH224052 |
| Skim milk powder | Shanghai, Sangon | A600669-0250 | D4424BA0028 |

(continued)

| Name | Brand | Catalog No. | Batch No. |
|---|---|---|---|
| ELISA stop solution | Beijing, Solarbio | C1058 | 20190620 |
| Human CD40 ligand/TNFSF5 protein | ACROBiosystems | CDL-H52Db | N/A |
| Goat anti-Human IgG Fc-HRP | BETHYL | A80-104P | A80-104P-91 |

## Information of Instrument and Equipment

| Name | Place of Origin and Brand | Model No. | No. |
|---|---|---|---|
| Microplate reader | USA, Thermo | Multiskan FC | AS-A1-008 |
| Microplate constant-temperature oscillator | Hangzhou, Allsheng | MB100-4A | AS-A1-015 |
| Plate washer | USA, BioTek | 405TSUS | AS-A1-076 |

## Experimental Preparation

2.1 1× PBS buffer

100 mL of 10× PBS buffer was brought to a constant volume of 1 L and mixed thoroughly.

2.2 Coating buffer

One packet of carbonate powder was taken, dissolved in 400 mL of ultrapure water, brought to a constant volume of 500 mL, and mixed thoroughly.

2.3 Wash buffer

1 mL of Tween-20 was added to 1,999 mL of 1× PBS buffer and mixed thoroughly.

2.4 Blocking buffer

5 g of skim milk powder was weighed and dissolved in 100 mL of wash buffer and mixed thoroughly.

2.5 Sample dilution (antibody dilution)

1 g of BSA was weighed, dissolved in 100 mL of wash buffer, and mixed thoroughly.

## Experimental Process

3.1.1 Pre-coating: A 96-well immunoplate was coated one day in advance. CD40L protein was diluted to 1 µg/mL using coating buffer. 100 µL was added to each well and the immunoplate was sealed with a plate sealing film and incubated overnight at 4°C.

3.1.2 Plate washing: The pre-coating solution in the 96-well immunoplate was discarded, and the plate was blotted dry on absorbent paper towel. Then, 300 µL of wash buffer was added to each well and mixed by shaking for 10 seconds. After blotting off the wash buffer, the washing step was repeated 3 more times.

3.1.3 Blocking: Blocking solution was added using a multichannel pipette at 200 µL per well. The plate was sealed with a plate sealing film and incubated overnight at 4°C or for 2 h at room temperature.

3.1.4 Plate washing: The step described in 3.1.2 was repeated.

3.1.5 Sample loading: The diluted reference standards (Hz33B9C5 and Hz47E2, with concentrations ranging from 1 to 512 ng/mL, 10 concentrations in 2-fold dilution), quality control samples (Hz33B9C5 and Hz47E2, 5.12 to 200 ng/mL, 5 concentrations in 2-fold dilution), and samples to be tested (serum collected from mouse orbital sinus at specified time points) were added at 100 μL per well and incubated for 2 h at room temperature, protected from light.

3.1.6 Plate washing: The solution in the 96-well immunoplate was discarded, and the plate was blotted dry on absorbent paper towel. Then, 300 μL of wash buffer was added to each well and mixed by shaking for 10 seconds. After blotting off the wash buffer, the washing step was repeated 6 more times.

3.1.7 Enzyme-labeled antibody incubation: The diluted Goat anti-Human IgG Fc-HRP (25 ng/mL) was added at 100 μL per well and incubated for 1 h at room temperature, protected from light.

3.1.8 Plate washing: The step described in 3.1.6 was repeated.

3.1.9 Chromogenic reaction: TMB substrate was added to the 96-well immunoplate at 100 μL per well and incubated away from light at room temperature for 5-10 min for the chromogenic reaction.

3.1.10 Termination: 50 μL of ELISA stop solution was added to each well, and the plate was shaken at medium speed for 10 seconds. The OD450 nm/620 nm values were read within 30 min.

**Result Analysis**

**4.1** Measured concentrations were retained to 4 decimal places, and the average concentration was retained to 2 decimal places, denoted as "mean".

**4.2** Absorbance values at 450 nm and 620 nm were provided by the microplate reader and cannot be altered. The OD of each sample was calculated as (A450-A620) for the sample. Absorbance values were retained to 4 decimal places. Each sample was loaded in duplicate wells. Accuracy (used to evaluate the relative error compared to the theoretical value, hereinafter abbreviated as %RE) and precision (used to evaluate the relative standard deviation, hereinafter abbreviated as %CV) were retained to 1 decimal place. The calculation formula is as follows:

$$\text{Precision (\%CV)} = \text{Standard deviation (SD)/Mean measured concentration} \times 100\%$$

Accuracy (%RE) = (Mean measured concentration - Theoretical concentration)/Theoretical concentration $\times$ 100%

**4.3** Experimental data: Measured concentrations were obtained by fitting a four-parameter logistic curve using SkanIt 3.1 analysis software (Thermo). The calculations of mean concentration, accuracy, and precision were all performed using the built-in functions of Excel.

The calibration curve equation is:

$$y = d + (a - d)/(1 + (x / c)\text{^}b)$$

See FIG. 14 for the results. As shown in Table 2 and FIG. 14, Hz33B9C5 and Hz47E2 exhibited a relatively long in vivo half-life, indicating that the antibodies of the present invention improve the pharmacokinetics of anti-CD40L antibodies and possess excellent pharmacokinetic properties.

Table 2. Pharmacokinetics of the anti-CD40L antibody of the present invention

| Group | $C_{max}$ (μg/mL) | $AUC_{0-t}$ (μg*h/mL) | $AUC_{0-\infty}$ (μg*h/mL) | $MRT_{0-\infty}$ (h) | $T_{1/2}$ (h) | CL (mL/h) | Vss (mL/kg) |
|---|---|---|---|---|---|---|---|
| Hz33B9C5 | 164 | 18463 | 23512 | 323 | 231 | 0.43 | 142 |
| Hz47E2 | 214 | 26304 | 37260 | 406 | 290 | 0.27 | 112 |

Sequence information:

| | | Antibody Hz33B9C5 | |
|---|---|---|---|
| SEQ ID NO | Sequence Name | | Specific Sequence |
| 1 | HCDR1 | | GFTFSDYYMY |
| 2 | HCDR2 | | TISGGGTYTYSPDSVKG |
| 3 | HCDR3 | | GEPGLAY |
| 4 | Heavy chain variable region (VH) | | QVQLVESGGGLVKPGGSLRLSCAASGFTFSDYYMYWIRQAPGKGLEWVSTISGGGT YTYSPDSVKGRFTISRDNAKNSLYLQMNSLRAEDTAVYYCARGEPGLAYWGQGTLV TVSS |
| 5 | Heavy chain constant region | | ASTKGPSVFPLAPSSKSTSGGTAALGCLVKDYFPEPVTVSWNSGALTSGVHTFPAVLQ SSGLYSLSSVVTVPSSSLGTQTYICNVNHKPSNTKVDKKVEPKSCDKTHTCPPCPAPE AAGGPSVFLFPPKPKDTLMISRTPEVTCVVVDVSHEDPEVKFNWYVDGVEVHNAKT KPREEQYNSTYRVVSVLTVLHQDWLNGKEYKCKVSNKALAPIEKTISKAKGQPREP QVYTLPPSRDELTKNQVSLTCLVKGFYPSDIAVEWESNGQPENNYKTTPPVLDSDGSF FLYSKLTVDKSRWQQGNVFSCSVMHEALHNHYTQKSLSLSPGK |
| 6 | Full length of heavy chain (HC) | | QVQLVESGGGLVKPGGSLRLSCAASGFTFSDYYMYWIRQAPGKGLEWVSTISGGGT YTYSPDSVKGRFTISRDNAKNSLYLQMNSLRAEDTAVYYCARGEPGLAYWGQGTLV TVSSASTKGPSVFPLAPSSKSTSGGTAALGCLVKDYFPEPVTVSWNSGALTSGVHTFP AVLQSSGLYSLSSVVTVPSSSLGTQTYICNVNHKPSNTKVDKKVEPKSCDKTHTCPPC PAPEAAGGPSVFLFPPKPKDTLMISRTPEVTCVVVDVSHEDPEVKFNWYVDGVEVHN AKTKPREEQYNSTYRVVSVLTVLHQDWLNGKEYKCKVSNKALAPIEKTISKAKGQP REPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSPGK |
| 7 | LCDR1 | | KASQSVDYYGDSYMH |
| 8 | LCDR2 | | ASSNLES |
| 9 | LCDR3 | | QQSHEDPWT |
| 10 | Light chain variable region (VL) | | DIVMTQSPDSLAVSLGERATINCKASQSVDYYGDSYMHWYQQKPGQPPKLLIYASSN LESGVPDRFSGSGSGTDFTLTISSLQAEDVAVYYCQQSHEDPWTFGGGTKVEIK |
| 11 | Light chain constant region | | RTVAAPSVFIFPPSDEQLKSGTASVVCLLNNFYPREAKVQWKVDNALQSGNSQESVT EQDSKDSTYSLSSTLTLSKADYEKHKVYACEVTHQGLSSPVTKSFNRGEC |
| 12 | Full length of light chain (LC) | | DIVMTQSPDSLAVSLGERATINCKASQSVDYYGDSYMHWYQQKPGQPPKLLIYASSN LESGVPDRFSGSGSGTDFTLTISSLQAEDVAVYYCQQSHEDPWTFGGGTKVEIKRTVA APSVFIFPPSDEQLKSGTASVVCLLNNFYPREAKVQWKVDNALQSGNSQESVTEQDS KDSTYSLSSTLTLSKADYEKHKVYACEVTHQGLSSPVTKSFNRGEC |

(continued)

| | SEQ ID NO | Sequence Name | Specific Sequence |
|---|---|---|---|
| | | | **Antibody Hz33B9C5** |
| | 28 | VHDNA | AAGCTTGCCGCCACCATGAAGTGGGTGACTTTTATCAGTCTACTATTCCTGTTCTC CTCCAGCTCTCGGGCTCAAGTGCAGCTGGTCGAGTCTGGCGGAGGCCTGGTGAA GCCTGGAGGCTCTCTCCGGCTGTCCTGTGCAGCCTCTGGCTTCACCTTCTCTGACT ACTACATGTACTGGATCAGACAGGCCCCTGGCAAGGGACTGGAATGGGTGTCAAC AATCAGCGGCGGCGGAACCTACACCTATTCTCCTGATTCCGTCAAGGGCAGATTC ACCATCAGCCGGGACAACGCCAAGAACTCCCTGTACCTGCAGATGAACTCCCTGA GAGCCGAGGATACCGCCGTGTACTACTGCGCCAGAGGCGAGCCTGGCCTGGCCTA CTGGGGACAAGGGACCCTCGTGACCGTGTCGTCCGCCTCCACCAAGGGACCTTC TGTGTTTCCTCTGGCTCCTAGCTCCAAGTCCACCTCCGGCGGCACCGCTGCTCTGG GCTGTCTGGTGAAGGACTACTTCCCTGAGCCCGTGACAGTGTCCTGGAACTCTGG CGCCCTGACCAGCGGCGTGCATACCTTTCCCGCTGTGTTGCAGTCCTCTGGCCTGT ACTCTCTGTCTTCCGTGGTCACCGTGCCTTCATCCTCCCTGGGCACCCAGACCTAC ATCTGCAACGTGAACCACAAGCCCTCCAACACCAAGGTGGACAAGAAAGTGGAA CCCAAATCTTGCGACAAGACCCACACCTGTCCTCCTTGCCCTGCTCCCGAGGCCG CTGGCGGCCCTTCCGTCTTTCTGTTCCCACCTAAGCCTAAGGATACACTGATGATC TCTAGAACACCTGAAGTGACCTGCGTGGTGGTCGACGTGTCTCACGAGGATCCAG AGGTGAAGTTCAATTGGTACGTGGATGGCGTGGAAGTGCACAACGCCAAAACCA AACCTAGAGAGGAACAGTACAACTCCACCTACAGAGTGGTTTCTGTCCTGACCGT GCTGCACCAGGACTGGCTGAACGGCAAAGAGTACAAGTGCAAGGTGAGCAACA |
| | | | AGGCCCTGGCCCCTATCGAGAAGACCATCTCCAAGGCTAAGGGCCAGCCCCGGG AACCTCAGGTTTATACACTGCCCCCATCTCGGGACGAGCTGACCAAGAACCAGGT GTCTCTGACCTGCCTGGTGAAAGGCTTCTACCCCTCTGACATCGCCGTGGAATGG GAGTCCAATGGCCAGCCTGAGAACAACTACAAGACAACCCCTCCTGTGCTGGAC TCCGACGGCTCCTTCTTCCTGTACTCCAAGCTGACAGTGGACAAGTCCCGCTGGC AGCAGGGCAACGTGTTCTCCTGCTCCGTGATGCACGAGGCTCTGCATAATCACTA CACCCAGAAGAGCCTGTCACTGTCTCCAGGCAAGTGATGAGAATTC |
| | 29 | VLDNA | AAGCTTGCCGCCACCATGAAGTGGGTGACTTTTATCAGTCTACTATTTCTGTTCTC CTCCAGCTCTAGAGCCGACATCGTGATGACCCAGAGTCCCGATAGCCTGGCTGTG TCCCTGGGAGAGCGCGCTACCATCAACTGCAAAGCCTCTCAGTCCGTGGATTACT ACGGCGACTCCTACATGCACTGGTACCAGCAGAAGCCTGGCCAGCCTCCTAAGCT GCTGATCTACGCCTCTTCCAACCTGGAATCCGGCGTGCCCGACCGGTTTAGCGGC TCCGGCTCTGGTACCGACTTCACCCTCACAATCAGCTCTCTGCAGGCCGAGGACG TGGCCGTGTACTACTGCCAGCAGTCTCACGAGGATCCTTGGACCTTCGGCGGCGG AACAAAAGTGGAAATCAAGCGGACCGTGGCTGCTCCTTCCGTGTTCATCTTCCCC CCATCTGATGAACAGCTGAAGAGCGGCACAGCTTCTGTGGTGTGCCTGCTGAACA ACTTCTACCCTAGAGAGGCCAAGGTGCAGTGGAAGGTGGACAACGCCCTGCAAT CTGGCAACTCCCAAGAGTCCGTCACCGAGCAGGACTCCAAGGACTCTACCTACTC CCTGTCCTCCACCCTGACCCTGTCCAAAGCTGACTACGAGAAGCACAAGGTCTAT GCCTGCGAGGTGACCCATCAGGGCCTGTCTTCTCCTGTGACCAAGTCCTTCAATA GAGGCGAATGTTGATGAGAATTC |
| | | | **Antibody Hz47E2** |
| | SEQ ID NO | Sequence Name | Specific Sequence |
| | 13 | HCDR1 | GFTFSDYAMS |
| | 14 | HCDR2 | EISTVHTYTYYSDAVAG |
| | 15 | HCDR3 | DRVLDY |
| | 16 | Heavy chain variable region (VH) | EVQLVESGGGLVKPGGSLRLSCAASGFTFSDYAMSWVRQAPGKGLEWVSEISTVHT YTYYSDAVAGRFTISRDNAKNSLYLQMNSLRAEDTAVYYCARDRVLDYWGQGTLVT VSS |

| | SEQ ID NO | Sequence Name | Specific Sequence |
|---|---|---|---|
| | | | **Antibody Hz47E2** |
| | 5 | Heavy chain constant region | ASTKGPSVFPLAPSSKSTSGGTAALGCLVKDYFPEPVTVSWNSGALTSGVHTFPAVLQ SSGLYSLSSVVTVPSSSLGTQTYICNVNHKPSNTKVDKKVEPKSCDKTHTCPPCPAPE AAGGPSVFLFPPKPKDTLMISRTPEVTCVVVDVSHEDPEVKFNWYVDGVEVHNAKT KPREEQYNSTYRVVSVLTVLHQDWLNGKEYKCKVSNKALAPIEKTISKAKGQPREP QVYTLPPSRDELTKNQVSLTCLVKGFYPSDIAVEWESNGQPENNYKTTPPVLDSDGSF FLYSKLTVDKSRWQQGNVFSCSVMHEALHNHYTQKSLSLSPGK |
| | 17 | Full length of heavy chain (HC) | EVQLVESGGGLVKPGGSLRLSCAASGFTFSDYAMSWVRQAPGKGLEWVSEISTVHT YTYYSDAVAGRFTISRDNAKNSLYLQMNSLRAEDTAVYYCARDRVLDYWGQGTLVT VSSASTKGPSVFPLAPSSKSTSGGTAALGCLVKDYFPEPVTVSWNSGALTSGVHTFPA VLQSSGLYSLSSVVTVPSSSLGTQTYICNVNHKPSNTKVDKKVEPKSCDKTHTCPPCP APEAAGGPSVFLFPPKPKDTLMISRTPEVTCVVVDVSHEDPEVKFNWYVDGVEVHN AKTKPREEQYNSTYRVVSVLTVLHQDWLNGKEYKCKVSNKALAPIEKTISKAKGQP REPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSPGK |
| | 18 | LCDR1 | RASESVEYYDTSLMQ |
| | 19 | LCDR2 | AASNLES |
| | 20 | LCDR3 | QQTKKLPWT |
| | 21 | Light chain variable region (VL) | DIVMTQSPDSLAVSLGERATINCRASESVEYYDTSLMQWYQQKPGQPPKLLIYAASN LESGVPDRFSGSGSGTDFTLTISSLQAEDVAVYYCQQTKKLPWTFGQGTKVEIK |
| | 11 | Light chain constant region | RTVAAPSVFIFPPSDEQLKSGTASVVCLLNNFYPREAKVQWKVDNALQSGNSQESVT EQDSKDSTYSLSSTLTLSKADYEKHKVYACEVTHQGLSSPVTKSFNRGEC |
| | 22 | Full length of light chain (LC) | DIVMTQSPDSLAVSLGERATINCRASESVEYYDTSLMQWYQQKPGQPPKLLIYAASN LESGVPDRFSGSGSGTDFTLTISSLQAEDVAVYYCQQTKKLPWTFGQGTKVEIKRTVA APSVFIFPPSDEQLKSGTASVVCLLNNFYPREAKVQWKVDNALQSGNSQESVTEQDS |
| | | | KDSTYSLSSTLTLSKADYEKHKVYACEVTHQGLSSPVTKSFNRGEC |

(continued)

| | | Antibody Hz47E2 | |
|---|---|---|---|
| SEQ ID NO | Sequence Name | Specific Sequence | |
| 30 | VHDNA | AAGCTTGCCGCCACCATGAAGTGGGTGACTTTTATCAGTCTACTATTTCTGTTCTC CTCTTCCTCTAGAGCCGAGGTGCAGCTGGTGGAATCCGGCGGAGGCCTGGTCAAG CCTGGAGGCTCTCTGAGACTGTCCTGTGCCGCTTCCGGCTTCACCTTCAGCGACT ACGCCATGTCTTGGGTGCGGCAGGCCCCTGGCAAGGGCCTGGAATGGGTCTCTGA GATCAGCACCGTGCACACCTATACCTATTACAGCGACGCCGTGGCCGGCAGATTC ACCATCTCTCGGGACAACGCTAAGAACAGCCTGTACCTGCAGATGAACTCCCTCC GGGCTGAGGACACCGCCGTGTACTACTGCGCCAGAGATCGGGTGCTGGATTACTG GGGCCAAGGCACCCTGGTTACAGTCTCCTCCGCCTCTACCAAGGGACCAAGCGTG TTTCCTCTGGCCCCGAGTTCTAAGTCCACCTCTGGCGGCACCGCTGCCCTGGGCT GCCTGGTGAAGGACTACTTCCCTGAGCCCGTGACCGTGTCCTGGAACTCTGGCGC TCTGACCTCTGGTGTGCATACTTTCCCCGCTGTGCTGCAGTCCTCTGGACTGTACT CCCTGTCTTCGGTCGTGACAGTGCCCTCTAGCTCTCTGGGCACACAGACCTACATC TGCAACGTGAATCACAAGCCCTCCAACACCAAGGTGGACAAGAAAGTGGAACCT AAGTCTTGCGACAAGACACACACCTGTCCTCCTTGCCCTGCTCCTGAGGCTGCTG GCGGCCCTTCCGTGTTCCTGTTTCCACCCAAGCCTAAAGATACCCTGATGATCTCC CGGACCCCTGAAGTGACCTGCGTGGTGGTGGACGTGTCTCACGAAGATCCTGAA GTGAAGTTCAACTGGTACGTGGACGGCGTGGAAGTGCACAACGCCAAAACCAAA CCTCGCGAAGAGCAGTACAACTCCACCTACAGAGTGGTCTCCGTGCTGACCGTGC TGCACCAGGACTGGCTGAACGGCAAAGAGTACAAGTGCAAGGTTTCCAACAAGG CTCTGGCTCCCATCGAGAAGACCATCTCCAAGGCCAAAGGCCAGCCTAGAGAGC CTCAGGTGTACACACTGCCTCCTAGCAGAGACGAGCTGACAAAGAACCAGGTGT CTTTGACCTGTCTGGTGAAGGGCTTCTACCCTTCTGACATCGCCGTGGAGTGGGA GTCCAATGGCCAACCCGAGAACAACTACAAGACCACCCCACCTGTGCTGGACTC CGATGGCTCCTTCTTCCTGTACTCCAAGCTGACCGTGGATAAGTCCAGATGGCAGC AGGGCAACGTGTTCTCCTGCTCTGTGATGCACGAGGCCCTGCATAATCACTACAC CCAGAAGTCCCTGTCCCTAAGCCCTGGTAAGTGATGAGAATTC | |
| 31 | VLDNA | AAGCTTGCCGCCACCATGAAGTGGGTGACTTTTATCAGTCTACTATTTCTGTTCTC CAGCTCTTCTCGGGCCGATATCGTGATGACCCAGTCTCCAGATTCTCTGGCTGTGT CCCTGGGCGAAAGAGCTACCATCAACTGCAGAGCCTCCGAGTCCGTGGAATACTA CGACACCAGCCTGATGCAGTGGTACCAGCAGAAGCCTGGCCAGCCTCCTAAGCT GCTGATCTACGCCGCTTCCAACCTGGAATCCGGCGTGCCCGACCGGTTTAGCGGA TCTGGCTCTGGCACCGACTTCACCCTGACCATCTCCTCTCTCCAGGCCGAGGACG TGGCCGTGTACTACTGCCAGCAGACCAAGAAGCTGCCTTGGACCTTCGGCCAAG GCACCAAAGTCGAGATCAAGCGGACCGTGGCTGCTCCTTCCGTGTTCATCTTCCC CCCCTCCGATGAACAGCTGAAGTCTGGCACAGCTTCTGTGGTGTGCCTGCTGAAC AACTTCTACCCTAGAGAGGCCAAAGTGCAGTGGAAGGTGGACAATGCCCTGCAG TCCGGCAACTCCCAAGAGTCTGTTACTGAGCAGGACTCCAAGGACTCTACCTACT CCCTGAGCTCCACCCTGACACTGTCTAAGGCCGACTACGAGAAGCACAAAGTCTA TGCCTGCGAGGTGACACACCAGGGACTGTCCAGCCCTGTGACCAAGTCCTTCAA CAGAGGCGAGTGTTGATGAGAATTC | |

| | | | |
|---|---|---|---|
| SEQ ID NO | Sequence Name | Specific Sequence | |
| 23 | IgG1 Fc region (wild type) | DKTHTCPPCPAPELLGGPSVFLFPPKPKDTLMISRTPEVTCVVVDVSHEDPEVKFNWY VDGVEVHNAKTKPREEQYNSTYRVVSVLTVLHQDWLNGKEYKCKVSNKALPAPIE KTISKAKGQPREPQVYTLPPSRDELTKNQVSLTCLVKGFYPSDIAVEWESNGQPENNY KTTPPVLDSDGSFFLYSKLTVDKSRWQQGNVFSCSVMHEALHNHYTQKSLSLSPGK | |

(continued)

| SEQ ID NO | Sequence Name | Specific Sequence |
|---|---|---|
| 24 | Mutated IgG1 Fc region (comprising L234A/L235A and P329 deletion mutations (△P329)) | DKTHTCPPCPAPEAAGGPSVFLFPPKPKDTLMISRTPEVTCVVVDVSHEDPEVKFNW YVDGVEVHNAKTKPREEQYNSTYRVVSVLTVLHQDWLNGKEYKCKVSNKALAPIE KTISKAKGQPREPQVYTLPPSRDELTKNQVSLTCLVKGFYPSDIAVEWESNGQPENNY KTTPPVLDSDGSFFLYSKLTVDKSRWQQGNVFSCSVMHEALHNHYTQKSLSLSPGK |
| 25 | CH1 | ASTKGPSVFPLAPSSKSTSGGTAALGCLVKDYFPEPVTVSWNSGALTSGVHTFPAVLQ SSGLYSLSSVVTVPSSSLGTQTYICNVNHKPSNTKVDKKVEPKSC |
| 26 | Wild-type heavy chain constant region | ASTKGPSVFPLAPSSKSTSGGTAALGCLVKDYFPEPVTVSWNSGALTSGVHTFPAVLQ SSGLYSLSSVVTVPSSSLGTQTYICNVNHKPSNTKVDKKVEPKSCDKTHTCPPCPAPE LLGGPSVFLFPPKPKDTLMISRTPEVTCVVVDVSHEDPEVKFNWYVDGVEVHNAKT |
| | | KPREEQYNSTYRVVSVLTVLHQDWLNGKEYKCKVSNKALPAPIEKTISKAKGQPREP QVYTLPPSRDELTKNQVSLTCLVKGFYPSDIAVEWESNGQPENNYKTTPPVLDSDGSF FLYSKLTVDKSRWQQGNVFSCSVMHEALHNHYTQKSLSLSPGK |
| 27 | CD40L | MIETYNQTSPRSAATGLPISMKIFMYLLTVFLITQMIGSALFAVYLHRRLDKIEDERNL HEDFVFMKTIQRCNTGERSLSLLNCEEIKSQFEGFVKDIMLNKEETKKENSFEMQKG DQNPQIAAHVISEASSKTTSVLQWAEKGYYTMSNNLVTLENGKQLTVKRQGLYYIYA QVTFCSNREASSQAPFIASLCLKSPGRFERILLRAANTHSSAKPCGQQSIHLGGVFELQ PGASVFVNVTDPSQVSHGTGFTSFGLLKL |
| 32 | CD40 protein | EPPTACREKQYLINSQCCSLCQPGQKLVSDCTEFTETECLPCGESEFLDTWNRETHCH QHKYCDPNLGLRVQQKGTSETDTICTCEEGWHCTSEACESCVLHRSCSPGFGVKQIA TGVSDTICEPCPVGFFSNVSSAFEKCHPWTSCETKDLVVQQAGTNKTDVVCGPQDRL RALVVIPIIFGILFAILLVLVFIKKVAKKPTNKAPHPKQEPQEINFPDDLPGSNTAAPVQE TLHGCQPVTQEDGKESRISVQERQ |

**Claims**

1. An anti-CD40L antibody or an antigen-binding fragment thereof, wherein the antibody comprises:

    (i) the three complementarity-determining regions HCDR1, HCDR2, and HCDR3 comprised in the VH set forth in SEQ ID NO: 4, and the three complementarity-determining regions LCDR1, LCDR2, and LCDR3 comprised in the VL set forth in SEQ ID NO: 10; or
    (ii) the three complementarity-determining regions HCDR1, HCDR2, and HCDR3 comprised in the VH set forth in SEQ ID NO: 16, and the three complementarity-determining regions LCDR1, LCDR2, and LCDR3 comprised in the VL set forth in SEQ ID NO: 21.

2. An anti-CD40L antibody or an antigen-binding fragment thereof, comprising a first heavy chain complementarity-determining region (HCDR1), a second heavy chain complementarity-determining region (HCDR2), a third heavy chain complementarity-determining region (HCDR3), and a first light chain complementarity-determining region (LCDR1), a second light chain complementarity-determining region (LCDR2), and a third light chain complementarity-determining region (LCDR3), wherein:

    (i) the HCDR1, HCDR2, HCDR3, and LCDR1, LCDR2, and LCDR3 comprise the amino acid sequences set forth in SEQ ID NO: 1, SEQ ID NO: 2, SEQ ID NO: 3, SEQ ID NO: 7, SEQ ID NO: 8, and SEQ ID NO: 9, respectively; or consist of the amino acid sequences set forth in SEQ ID NO: 1, SEQ ID NO: 2, SEQ ID NO: 3, SEQ ID NO: 7, SEQ ID NO: 8, and SEQ ID NO: 9, respectively; or
    (ii) the HCDR1, HCDR2, HCDR3, and LCDR1, LCDR2, and LCDR3 comprise the amino acid sequences set forth in SEQ ID NO: 13, SEQ ID NO: 14, SEQ ID NO: 15, SEQ ID NO: 18, SEQ ID NO: 19, and SEQ ID NO: 20, respectively, or consist of the amino acid sequences set forth in SEQ ID NO: 13, SEQ ID NO: 14, SEQ ID NO: 15, SEQ ID NO: 18, SEQ ID NO: 19, and SEQ ID NO: 20, respectively.

3. The anti-CD40L antibody or the antigen-binding fragment thereof according to claim 1 or 2, comprising a heavy chain variable region (VH), wherein the heavy chain variable region comprises or consists of an amino acid sequence having at least 90% identity to an amino acid sequence selected from SEQ ID NO: 4 or 16, or comprises or consists of an amino acid sequence selected from SEQ ID NO: 4 or 16; or comprising a light chain variable region (VL), wherein the light chain variable region comprises or consists of an amino acid sequence having at least 90% identity to an amino acid sequence selected from SEQ ID NO: 10 or 21, or comprises or consists of an amino acid sequence selected from SEQ ID NO: 10 or 21.

4. An anti-CD40L antibody or an antigen-binding fragment thereof, comprising a heavy chain variable region and a light chain variable region, wherein

    (i) the heavy chain variable region comprises or consists of the amino acid sequence set forth in SEQ ID NO: 4, or an amino acid sequence having at least 90%, 91%, 92%, 93%, 94%, 95%, 96%, 97%, 98%, or 99% identity thereto, and the light chain variable region comprises or consists of the amino acid sequence set forth in SEQ ID NO: 10, or an amino acid sequence having at least 90%, 91%, 92%, 93%, 94%, 95%, 96%, 97%, 98%, or 99% identity thereto; or
    (ii) the heavy chain variable region comprises or consists of the amino acid sequence set forth in SEQ ID NO: 16, or an amino acid sequence having at least 90%, 91%, 92%, 93%, 94%, 95%, 96%, 97%, 98%, or 99% identity thereto, and the light chain variable region comprises or consists of the amino acid sequence set forth in SEQ ID NO: 21, or an amino acid sequence having at least 90%, 91%, 92%, 93%, 94%, 95%, 96%, 97%, 98%, or 99% identity thereto.

5. The anti-CD40L antibody or the antigen-binding fragment thereof according to claim 1 or 2, comprising a heavy chain variable region and a light chain variable region, wherein

    (i) the heavy chain variable region comprises or consists of the amino acid sequence set forth in SEQ ID NO: 4, and the light chain variable region comprises or consists of the amino acid sequence set forth in SEQ ID NO: 10; or
    (ii) the heavy chain variable region comprises or consists of the amino acid sequence set forth in SEQ ID NO: 16, and the light chain variable region comprises or consists of the amino acid sequence set forth in SEQ ID NO: 21.

6. The anti-CD40L antibody or the antigen-binding fragment thereof according to any one of claims 1-5, comprising an Fc region.

7. The anti-CD40L antibody or the antigen-binding fragment thereof according to claim 6, wherein the Fc region is derived from a human IgG Fc, for example, from a human IgG 1 Fc, a human IgG2 Fc, a human IgG3 Fc, or a human IgG4 Fc.

8. The anti-CD40L antibody or the antigen-binding fragment thereof according to claim 7, wherein the Fc region comprises mutation(s) that reduce or eliminate effector function and/or mutation(s) that eliminate a binding to Fcγ receptors while retaining an affinity for FcRn.

9. The anti-CD40L antibody or the antigen-binding fragment thereof according to claim 8, wherein the mutations are L234A/L235A and P329 deletion mutations.

10. The anti-CD40L antibody or the antigen-binding fragment thereof according to any one of claims 6-9, wherein the Fc region

    (i) comprises or consists of the amino acid sequence set forth in SEQ ID NO: 23 or 24;
    (ii) comprises an amino acid sequence having at least 90%, for example, 95%, 96%, 97%, 99%, or higher identity to the amino acid sequence set forth in SEQ ID NO: 23; or
    (iii) comprises an amino acid sequence having at least 90%, for example, 95%, 96%, 97%, 99%, or higher identity to the amino acid sequence set forth in SEQ ID NO: 24, and comprising the L234A/L235A and P329 deletion mutations.

11. The anti-CD40L antibody or the antigen-binding fragment thereof according to any one of claims 1-10, comprising a heavy chain constant region, wherein the heavy chain constant region is derived from the IgG1, IgG2, IgG3, or IgG4 constant region, preferably, the heavy chain constant region

(i) comprises or consists of an amino acid sequence selected from SEQ ID NO: 5 or 26;
(ii) comprises an amino acid sequence having at least 85%, 90%, 91%, 92%, 93%, 94%, 95%, 96%, 97%, 98%, or 99% identity to the amino acid sequence set forth in SEQ ID NO: 26; or
(iii) comprises an amino acid sequence having at least 85%, 90%, 91%, 92%, 93%, 94%, 95%, 96%, 97%, 98%, or 99% identity to the amino acid sequence set forth in SEQ ID NO: 5, and comprises the L234A/L235A and P329 deletion mutations.

12. The anti-CD40L antibody or the antigen-binding fragment thereof according to any one of claims 1-11, comprising a light chain constant region, wherein the light chain constant region is a lambda or kappa light chain constant region, preferably, the light chain constant region

(i) comprises or consists of an amino acid sequence having at least 85%, 90%, 91%, 92%, 93%, 94%, 95%, 96%, 97%, 98%, or 99% identity to the amino acid sequence set forth in SEQ ID NO: 11; or
(ii) comprises or consists of the amino acid sequence set forth in SEQ ID NO: 11.

13. The anti-CD40L antibody or the antigen-binding fragment thereof according to any one of claims 1-12, comprising a heavy chain, wherein the heavy chain

(i) comprises or consists of an amino acid sequence having at least 85%, 90%, 91%, 92%, 93%, 94%, 95%, 96%, 97%, 98%, or 99% identity to an amino acid sequence selected from SEQ ID NO: 6 or 17; or
(ii) comprises or consists of an amino acid sequence selected from SEQ ID NO: 6 or 17; or
comprising a light chain, wherein the light chain

(i) comprises or consists of an amino acid sequence having at least 85%, 90%, 91%, 92%, 93%, 94%, 95%, 96%, 97%, 98%, or 99% identity to an amino acid sequence selected from SEQ ID NO: 12 or 22; or
(ii) comprises or consists of an amino acid sequence selected from SEQ ID NO: 12 or 22.

14. An anti-CD40L antibody or an antigen-binding fragment thereof, comprising a heavy chain and a light chain, wherein

(i) the heavy chain comprises or consists of an amino acid sequence having at least 85%, 90%, 91%, 92%, 93%, 94%, 95%, 96%, 97%, 98%, or 99% identity to the amino acid sequence set forth in SEQ ID NO: 6; and the light chain comprises or consists of an amino acid sequence having at least 85%, 90%, 91%, 92%, 93%, 94%, 95%, 96%, 97%, 98%, or 99% identity to the amino acid sequence set forth in SEQ ID NO: 12; or
(ii) the heavy chain comprises or consists of an amino acid sequence having at least 85%, 90%, 91%, 92%, 93%, 94%, 95%, 96%, 97%, 98%, or 99% identity to the amino acid sequence set forth in SEQ ID NO: 17; and the light chain comprises or consists of an amino acid sequence having at least 85%, 90%, 91%, 92%, 93%, 94%, 95%, 96%, 97%, 98%, or 99% identity to the amino acid sequence set forth in SEQ ID NO: 22.

15. The anti-CD40L antibody or the antigen-binding fragment thereof according to any one of claims 1-14, comprising a heavy chain and a light chain, wherein

(i) the heavy chain comprises or consists of the amino acid sequence set forth in SEQ ID NO: 6, and the light chain comprises or consists of the amino acid sequence set forth in SEQ ID NO: 12;
(ii) the heavy chain comprises or consists of the amino acid sequence set forth in SEQ ID NO: 17, and the light chain comprises or consists of the amino acid sequence set forth in SEQ ID NO: 22.

16. The anti-CD40L antibody or the antigen-binding fragment thereof according to any one of claims 1-15, wherein the antibody is a monoclonal antibody.

17. The anti-CD40L antibody or the antigen-binding fragment thereof according to any one of claims 1-16, wherein the antibody is a humanized antibody or a chimeric antibody.

18. The anti-CD40L antibody or the antigen-binding fragment thereof according to any one of claims 1-17, wherein the antigen-binding fragment is an antibody fragment selecting from Fv, Fab, Fab', Fab'-SH, F(ab')$_2$, dAb (domain antibody), a linear antibody, a single-chain antibody (e.g., scFv), a single-domain antibody (sdAb) such as VHH, a bivalent antibody or fragment thereof, or a camelid antibody or diabody.

19. The anti-CD40L antibody or the antigen-binding fragment thereof according to any one of claims 1-18, wherein the antibody or antigen-binding fragment thereof has one or more of the following properties:

   a) binding to CD40L (e.g., human CD40L) with high affinity;
   b) inhibiting the binding of CD40 to CD40L;
   c) inhibiting the CD40/CD40L signaling pathway;
   d) inhibiting activation or proliferation of B cells;
   e) inhibiting proliferation of B cells;
   f) inhibiting maturation and differentiation of B cells, for example, inhibiting differentiation of B cells into plasma cells;
   g) inhibiting IL-12 secretion by dendritic cells;
   h) not inducing platelet activation;
   i) treating or preventing an immune disease, such as multiple sclerosis (MS); and/or
   j) inhibiting an immune response, preferably a humoral immune response, for example, inhibiting antibody production;
   k) improving pharmacokinetics, preferably having an in vivo half-life of about 200 hours or more, more preferably about 210, 220, 230, 240, 250, 260, 270, 280, or 290 hours or more.

20. An isolated nucleic acid encoding the anti-CD40L antibody or the antigen-binding fragment thereof according to any one of claims 1-19.

21. A vector comprising the nucleic acid of claim 20, wherein the vector is preferably an expression vector.

22. A host cell comprising the nucleic acid of claim 20 or the vector of claim 21, wherein the host cell is preferably prokaryotic or eukaryotic, more preferably selected from a yeast cell, a mammalian cell(e.g., a 293 cell or a CHO cells, such as a CHO-K cell or a HEK293 cell), or another cell suitable for producing an antibody or an antigen-binding fragment thereof.

23. A method for preparing an antibody or an antigen-binding fragment thereof that binds to CD40L, wherein the method comprises

   a) culturing the host cell of claim 22 under conditions suitable for expressing the nucleic acid encoding the anti-CD40L antibody or the antigen-binding fragment thereof according to any one of claims 1-19;
   b) optionally isolating the antibody or antigen-binding fragment thereof;
   c) optionally, the method further comprises recovering the anti-CD40L antibody or the antigen-binding fragment thereof from the host cell, wherein the antibody is optionally purified, for example, by Protein A.

24. An immunoconjugate comprising the anti-CD40L antibody or the antigen-binding fragment thereof according to any one of claims 1-19 and other substance, such as a therapeutic agent, for example an agent that can produce a biological effect, including but not limited to a cytokine, another antibody, a small-molecule drug, or an immunomodulator (e.g., an immunosuppressant).

25. A pharmaceutical composition, comprising the anti-CD40L antibody or the antigen-binding fragment thereof according to any one of claims 1-19 or the immunoconjugate of claim 24, and optionally a pharmaceutically acceptable auxiliary material.

26. A pharmaceutical combination product, comprising the anti-CD40L antibody or the antigen-binding fragment thereof according to any one of claims 1-19, or the immunoconjugate of claim 24, and one or more other therapeutic agents, for example agents that can produce a biological effect, including but not limited to cytokines, other antibodies, small-molecule drugs, or immunomodulators (e.g., immunosuppressants).

27. A method for preventing or treating a disease or disorder associated with an inappropriate activation of a CD40L/CD40-mediated pathway in a subject, wherein the method comprises administering to the subject an effective amount of the anti-CD40L antibody or the antigen-binding fragment thereof according to any one of claims 1-19, or the immunoconjugate of claim 24, or the pharmaceutical composition of claim 25, or the pharmaceutical combination product of claim 26.

28. The method according to claim 27, wherein the subject exhibits, compared to a healthy individual, an abnormal activation of a CD40L/CD40-mediated signaling pathway and/or excessive production and deposition of autoantibodies; and/or increased expression of CD40L or CD40 on the subject's cells (e.g., activated T cells or other types of immune cells or non-immune cells, such as epithelial cells, monocytes, dendritic cells, fibroblasts, smooth muscle cells, endothelial cells, and platelets), for example, compared to the corresponding cells of a healthy individual.

29. The method according to claim 27 or 28, wherein the disease or disorder is selected from an autoimmune disease, lupus nephritis, immune thrombocytopenic purpura (ITP), transplant rejection, Crohn's disease, inflammatory bowel disease (IBD), colitis, asthma/allergy, atherosclerosis, myasthenia gravis, multiple sclerosis, psoriasis, rheumatoid arthritis, ankylosing spondylitis, coronary heart disease, type 1 diabetes, amyotrophic lateral sclerosis (ALS), and an immune response to recombinant drug products, such as Factor VII for hemophilia.

**ELISA**

FIG. 1

**Luciferase Reporting System**

FIG. 2

## Primary B cell activation

FIG. 3

FIG. 4

FIG. 5

FIG. 6

## PAC-1 MFI

FIG. 7

**CD62P MFI**

FIG. 8

FIG. 9

FIG. 10

## Body Weight Change

FIG. 11

## Anti-MOG IgG Antibody ELISA

FIG. 12

## Anti-KLH IgG Antibody ELISA

FIG. 13

FIG. 14

## INTERNATIONAL SEARCH REPORT

| | | |
|---|---|---|
| International application No. | | |
| **PCT/CN2024/090122** | | |

**A. CLASSIFICATION OF SUBJECT MATTER**

C07K16/28(2006.01)i; C07K16/46(2006.01)i; A61K39/395(2006.01)i

According to International Patent Classification (IPC) or to both national classification and IPC

**B. FIELDS SEARCHED**

Minimum documentation searched (classification system followed by classification symbols)

IPC: C07K; A61K

Documentation searched other than minimum documentation to the extent that such documents are included in the fields searched

Electronic data base consulted during the international search (name of data base and, where practicable, search terms used)

CNKI, CNTXT, DWPI, ENTXT, ELSEVIER, ISI Web of Knowledge, 中国专利生物序列检索系统, China Patent Biological Sequence Search System, Genbank, EMBL, STN, PubMed, 抗体, 抗原, 抗原结合片段, 抗原结合蛋白, CDR, complementarity determining region, 互补决定区, 相关序列, related sequences, antibodies, antigens, antigen binding fragments, antigen binding proteins, CD40L, CD154, CD40 ligand, gp39

**C. DOCUMENTS CONSIDERED TO BE RELEVANT**

| Category* | Citation of document, with indication, where appropriate, of the relevant passages | Relevant to claim No. |
|---|---|---|
| A | CN 107949572 A (SNU R&DB FOUNDATION) 20 April 2018 (2018-04-20) specific embodiments, and sequence listing | 1-29 |
| A | US 2022380478 A1 (TONIX PHARMA HOLDINGS LTD.) 01 December 2022 (2022-12-01) entire document | 1-29 |
| A | CN 108135969 A (IMMUNEXT, INC.) 08 June 2018 (2018-06-08) entire document | 1-29 |
| A | US 2004038293 A1 (NOVARTIS AG) 26 February 2004 (2004-02-26) entire document | 1-29 |
| A | CN 107921128 A (JANSSEN BIOTECHNOLOGY, INC.) 17 April 2018 (2018-04-17) entire document | 1-29 |
| A | CN 103214577 A (BIOGEN IDEC MA INC.) 24 July 2013 (2013-07-24) entire document | 1-29 |

☑ Further documents are listed in the continuation of Box C.    ☑ See patent family annex.

| | |
|---|---|
| \* Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
|---|---|
| **02 July 2024** | **11 July 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2024/090122** |

**C.    DOCUMENTS CONSIDERED TO BE RELEVANT**

| Category* | Citation of document, with indication, where appropriate, of the relevant passages | Relevant to claim No. |
| --- | --- | --- |
| A | CN 107206075 A (UCB BIOPHARMA SPRL et al.) 26 September 2017 (2017-09-26) entire document | 1-29 |

Form PCT/ISA/210 (second sheet) (July 2022)

**INTERNATIONAL SEARCH REPORT**

International application No.

**PCT/CN2024/090122**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

| International application No. |
| --- |
| **PCT/CN2024/090122** |

**Box No. II    Observations where certain claims were found unsearchable (Continuation of item 2 of first sheet)**

This international search report has not been established in respect of certain claims under Article 17(2)(a) for the following reasons:

1. ☑ Claims Nos.: **27-29**
   because they relate to subject matter not required to be searched by this Authority, namely:

   Claims 27-29 relate to a method for treating a disease, which falls within subject matter for which a search is not performed as defined in PCT Rule 39.1(iv). In the present report, a search is performed on the basis of "a pharmaceutical use of the related substance".

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

## INTERNATIONAL SEARCH REPORT
### Information on patent family members

| International application No. |
| --- |
| **PCT/CN2024/090122** |

| Patent document cited in search report | | | Publication date (day/month/year) | Patent family member(s) | | | Publication date (day/month/year) |
| --- | --- | --- | --- | --- | --- | --- | --- |
| CN | 107949572 | A | 20 April 2018 | KR | 20170000346 | A | 02 January 2017 |
| | | | | KR | 101810778 | B1 | 20 December 2017 |
| | | | | EP | 3315513 | A1 | 02 May 2018 |
| | | | | EP | 3315513 | A4 | 26 December 2018 |
| | | | | US | 2018194847 | A1 | 12 July 2018 |
| | | | | US | 10882911 | B2 | 05 January 2021 |
| US | 2022380478 | A1 | 01 December 2022 | EP | 3993876 | A1 | 11 May 2022 |
| | | | | BR | 112021026410 | A2 | 08 February 2022 |
| | | | | AU | 2020300002 | A1 | 24 February 2022 |
| | | | | MX | 2022000133 | A | 27 April 2022 |
| | | | | WO | 2021001458 | A1 | 07 January 2021 |
| | | | | IL | 289354 | A | 01 February 2022 |
| | | | | JP | 2022538674 | A | 05 September 2022 |
| | | | | CA | 3145453 | A1 | 07 January 2021 |
| CN | 108135969 | A | 08 June 2018 | DOP | 2018000018 | A | 15 October 2019 |
| | | | | JP | 2021063129 | A | 22 April 2021 |
| | | | | EA | 201890297 | A1 | 29 June 2018 |
| | | | | CO | 2018001363 | A2 | 10 May 2018 |
| | | | | MX | 2018000548 | A | 26 September 2018 |
| | | | | EP | 3331548 | A1 | 13 June 2018 |
| | | | | EP | 3331548 | A4 | 13 February 2019 |
| | | | | HK | 1249735 | A1 | 09 November 2018 |
| | | | | CL | 2018000103 | A1 | 29 June 2018 |
| | | | | AU | 2016294417 | A1 | 08 March 2018 |
| | | | | AU | 2016294417 | B2 | 04 August 2022 |
| | | | | CR | 20180097 | A | 25 May 2018 |
| | | | | CA | 2992116 | A1 | 19 January 2017 |
| | | | | US | 2021145966 | A1 | 20 May 2021 |
| | | | | US | 11596689 | B2 | 07 March 2023 |
| | | | | AU | 2022263520 | A1 | 08 December 2022 |
| | | | | BR | 112018000653 | A2 | 18 September 2018 |
| | | | | GT | 201800018 | A | 08 August 2019 |
| | | | | IL | 310460 | A | 01 March 2024 |
| | | | | US | 2024000929 | A1 | 04 January 2024 |
| | | | | ECSP | 18009949 | A | 31 October 2018 |
| | | | | IL | 297907 | A | 01 January 2023 |
| | | | | IL | 297907 | B1 | 01 March 2024 |
| | | | | PH | 12018500098 | A1 | 09 July 2018 |
| | | | | PE | 20180741 | A1 | 27 April 2018 |
| | | | | JP | 2023123812 | A | 05 September 2023 |
| | | | | WO | 2017011544 | A1 | 19 January 2017 |
| | | | | KR | 20180037984 | A | 13 April 2018 |
| | | | | TN | 2018000021 | A1 | 08 July 2019 |
| | | | | IL | 256800 | A | 29 March 2018 |
| | | | | IL | 256800 | B | 01 December 2022 |
| | | | | IL | 256800 | B2 | 01 April 2023 |
| | | | | JP | 2018528258 | A | 27 September 2018 |
| | | | | JP | 6826113 | B2 | 03 February 2021 |
| | | | | MY | 196991 | A | 17 May 2023 |
| | | | | SG | 10201913431 | QA | 30 March 2020 |

Form PCT/ISA/210 (patent family annex) (July 2022)

## INTERNATIONAL SEARCH REPORT
### Information on patent family members

| International application No. |
| --- |
| **PCT/CN2024/090122** |

| Patent document cited in search report | | | Publication date (day/month/year) | Patent family member(s) | | | Publication date (day/month/year) |
| --- | --- | --- | --- | --- | --- | --- | --- |
| | | | | US | 2018193454 | A1 | 12 July 2018 |
| | | | | US | 10874738 | B2 | 29 December 2020 |
| US | 2004038293 | A1 | 26 February 2004 | AU | 4835001 | A | 24 September 2001 |
| | | | | GB | 0006398 | D0 | 03 May 2000 |
| | | | | EP | 1263959 | A1 | 11 December 2002 |
| | | | | CO | 5280086 | A1 | 30 May 2003 |
| | | | | WO | 0168860 | A1 | 20 September 2001 |
| | | | | PE | 20030104 | A1 | 10 March 2003 |
| | | | | AU | 2001248350 | B2 | 14 October 2004 |
| | | | | CA | 2400106 | A1 | 20 September 2001 |
| | | | | AR | 027976 | A1 | 23 April 2003 |
| | | | | JP | 2003526371 | A | 09 September 2003 |
| | | | | US | 7169389 | B2 | 30 January 2007 |
| CN | 107921128 | A | 17 April 2018 | AU | 2016301361 | A1 | 08 February 2018 |
| | | | | AU | 2016301361 | B2 | 16 June 2022 |
| | | | | JP | 2022058414 | A | 12 April 2022 |
| | | | | JP | 2018522562 | A | 16 August 2018 |
| | | | | JP | 7049988 | B2 | 07 April 2022 |
| | | | | HK | 1256804 | A1 | 04 October 2019 |
| | | | | PE | 20180802 | A1 | 09 May 2018 |
| | | | | WO | 2017024146 | A1 | 09 February 2017 |
| | | | | CO | 2018001256 | A2 | 10 May 2018 |
| | | | | SV | 2018005624 | A | 25 September 2018 |
| | | | | GT | 201800034 | A | 26 September 2019 |
| | | | | BR | 112018002319 | A2 | 11 December 2018 |
| | | | | US | 2020317794 | A1 | 08 October 2020 |
| | | | | US | 11421037 | B2 | 23 August 2022 |
| | | | | EA | 201890434 | A1 | 31 October 2018 |
| | | | | US | 2017037136 | A1 | 09 February 2017 |
| | | | | US | 10669343 | B2 | 02 June 2020 |
| | | | | CL | 2018000298 | A1 | 11 May 2018 |
| | | | | MA | 45573 | A | 15 May 2019 |
| | | | | CA | 2994825 | A1 | 09 February 2017 |
| | | | | KR | 20180030917 | A | 26 March 2018 |
| | | | | MX | 2018001522 | A | 15 March 2018 |
| | | | | MY | 188405 | A | 08 December 2021 |
| | | | | ECSP | 18008922 | A | 30 April 2018 |
| | | | | UA | 123398 | C2 | 31 March 2021 |
| | | | | ES | 2944982 | T3 | 27 June 2023 |
| | | | | NZ | 739597 | A | 26 March 2021 |
| | | | | PH | 12018500203 | A1 | 30 July 2018 |
| | | | | SG | 10201913275 | QA | 27 February 2020 |
| | | | | EP | 3331563 | A1 | 13 June 2018 |
| | | | | EP | 3331563 | A4 | 04 September 2019 |
| | | | | EP | 3331563 | B1 | 19 April 2023 |
| | | | | IL | 256997 | A | 29 March 2018 |
| | | | | IL | 256997 | B | 01 June 2022 |
| | | | | NI | 201800017 | A | 24 August 2018 |
| | | | | IL | 293065 | A | 01 July 2022 |
| CN | 103214577 | A | 24 July 2013 | GEP | 20146112 | B | 25 June 2014 |

Form PCT/ISA/210 (patent family annex) (July 2022)

## INTERNATIONAL SEARCH REPORT
### Information on patent family members

| International application No. |
| --- |
| **PCT/CN2024/090122** |

| Patent document cited in search report | Publication date (day/month/year) | Patent family member(s) | | Publication date (day/month/year) |
| --- | --- | --- | --- | --- |
| | | EP | 3524626 A1 | 14 August 2019 |
| | | CR | 11030 A | 04 December 2009 |
| | | KR | 20100015804 A | 12 February 2010 |
| | | KR | 101605908 B1 | 23 March 2016 |
| | | HK | 1187630 A1 | 11 April 2014 |
| | | CO | 6231038 A2 | 20 December 2010 |
| | | NZ | 580245 A | 12 January 2012 |
| | | RS | 58518 B1 | 30 April 2019 |
| | | DK | 2125894 T3 | 18 March 2019 |
| | | US | 2016200823 A1 | 14 July 2016 |
| | | DOP | 2009000221 A | 31 October 2009 |
| | | PL | 2125894 T3 | 30 August 2019 |
| | | EA | 200970879 A1 | 30 April 2010 |
| | | EA | 019636 B1 | 30 May 2014 |
| | | ECSP | 099683 A | 28 December 2009 |
| | | ZA | 200907385 B | 28 April 2011 |
| | | JP | 2017036319 A | 16 February 2017 |
| | | CY | 1121471 T1 | 29 May 2020 |
| | | SG | 196697 A1 | 13 February 2014 |
| | | MX | 2009010120 A | 19 October 2009 |
| | | IL | 201070 A0 | 17 May 2010 |
| | | IL | 201070 A | 31 July 2014 |
| | | US | 2014302016 A1 | 09 October 2014 |
| | | US | 9321840 B2 | 26 April 2016 |
| | | AU | 2008232409 A1 | 02 October 2008 |
| | | AU | 2008232409 B2 | 20 June 2013 |
| | | AU | 2008232409 C1 | 05 December 2013 |
| | | PH | 12013500858 A1 | 25 January 2016 |
| | | SI | 2125894 T1 | 31 May 2019 |
| | | PT | 2125894 T | 27 February 2019 |
| | | BR | 122020022640 B1 | 29 March 2022 |
| | | HRP | 20190312 T1 | 05 April 2019 |
| | | LT | 2125894 T | 25 March 2019 |
| | | JP | 2015062424 A | 09 April 2015 |
| | | JP | 6053744 B2 | 27 December 2016 |
| | | CA | 2681530 A1 | 02 October 2008 |
| | | CA | 2681530 C | 28 March 2017 |
| | | ME | 00832 B | 20 March 2012 |
| | | US | 2019233530 A1 | 01 August 2019 |
| | | MY | 166021 A | 21 May 2018 |
| | | BRPI | 0809042 A2 | 16 September 2014 |
| | | BRPI | 0809042 A8 | 19 December 2017 |
| | | BRPI | 0809042 B1 | 31 August 2021 |
| | | HUE | 041818 T2 | 28 May 2019 |
| | | IL | 219653 A0 | 28 June 2012 |
| | | TW | 200911825 A | 16 March 2009 |
| | | TWI | 417299 B | 01 December 2013 |
| | | HK | 1139691 A1 | 24 September 2010 |
| | | JP | 2010521967 A | 01 July 2010 |
| | | JP | 5721951 B2 | 20 May 2015 |

Form PCT/ISA/210 (patent family annex) (July 2022)

## INTERNATIONAL SEARCH REPORT
### Information on patent family members

| | International application No. |
|---|---|
| | **PCT/CN2024/090122** |

| Patent document cited in search report | Publication date (day/month/year) | Patent family member(s) | | | Publication date (day/month/year) |
|---|---|---|---|---|---|
| | | US | 2023135399 | A1 | 04 May 2023 |
| | | US | 2013045219 | A1 | 21 February 2013 |
| | | US | 8784823 | B2 | 22 July 2014 |
| | | ES | 2713859 | T3 | 24 May 2019 |
| | | EP | 2125894 | A2 | 02 December 2009 |
| | | EP | 2125894 | B1 | 19 December 2018 |
| | | GT | 200900248 | A | 06 September 2011 |
| | | WO | 2008118356 | A2 | 02 October 2008 |
| | | WO | 2008118356 | A3 | 20 November 2008 |
| | | US | 2010104573 | A1 | 29 April 2010 |
| | | US | 8293237 | B2 | 23 October 2012 |
| CN 107206075 A | 26 September 2017 | CA | 2975336 | A1 | 04 August 2016 |
| | | CA | 2975336 | C | 18 October 2022 |
| | | RU | 2017130464 | A | 28 February 2019 |
| | | RU | 2017130464 | A3 | 23 July 2019 |
| | | US | 2021324095 | A1 | 21 October 2021 |
| | | US | 11884737 | B2 | 30 January 2024 |
| | | PT | 3250600 | T | 17 June 2020 |
| | | ES | 2802982 | T3 | 22 January 2021 |
| | | HRP | 20201013 | T1 | 16 October 2020 |
| | | KR | 20170106476 | A | 20 September 2017 |
| | | KR | 102543877 | B1 | 14 June 2023 |
| | | US | 2018022812 | A1 | 25 January 2018 |
| | | DK | 3250600 | T3 | 08 June 2020 |
| | | PL | 3250600 | T3 | 21 September 2020 |
| | | SI | 3250600 | T1 | 30 September 2020 |
| | | EP | 3744735 | A1 | 02 December 2020 |
| | | AU | 2015379642 | A1 | 27 July 2017 |
| | | AU | 2015379642 | B2 | 04 November 2021 |
| | | WO | 2016119909 | A1 | 04 August 2016 |
| | | GB | 201501613 | D0 | 18 March 2015 |
| | | CY | 1123116 | T1 | 29 October 2021 |
| | | RS | 60575 | B1 | 31 August 2020 |
| | | EP | 3250600 | A1 | 06 December 2017 |
| | | EP | 3250600 | B1 | 06 May 2020 |
| | | NZ | 733454 | A | 22 December 2023 |
| | | LT | 3250600 | T | 27 July 2020 |
| | | HUE | 050024 | T2 | 30 November 2020 |
| | | AU | 2022200347 | A1 | 17 February 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 101072587 B
- AU 2016294417 B2
- US 8293237 B2
- CN 101679521 B
- WO 2017011544 A1

### Non-patent literature cited in the description

- *Nat. Med.*, 2000, vol. 6 (2), 114
- **CHOTHIA et al.** *Nature*, 1989, vol. 342, 877-883
- **AL-LAZIKANI et al.** Standard conformations for the canonical structures of immunoglobulins. *Journal of Molecular Biology*, 1997, vol. 273, 927-948
- Sequences of Proteins of Immunological Interest. **KABAT et al.** U.S. Department of Health and Human Services. National Institutes of Health, 1987
- Sequences of Proteins of Immunological Interest. **KABAT et al.** Public Health Service. National Institutes of Health, 1991
- **KABAT et al.** Sequences of Proteins of Immunological Interest. Public Health Service, National Institutes of Health, 1991
- **R.C.ROWE** ; **P.J.SESKEY** ; **S.C. OWEN**. Handbook of Pharmaceutical Excipients. Pharmaceutical Press